# EUROPEAN PATENT APPLICATION

(11) **EP 4 173 485 A1**
(43) Date of publication of application: **03.05.2023**
(21) Application number: 21205130.4
(22) Date of filing: 27.10.2021
(51) Int. Cl.: A01N 43/50, A01N 43/56, A01N 43/653, A01N 43/76, A01N 43/80, A01N 43/82, A01P 1/00, A01P 3/00, C07D 231/12, C07D 233/58, C07D 249/08, C07D 261/08, C07D 263/32, C07D 271/06

(54) **PROTEIN AGGREGATION INHIBITING COMPOUNDS FOR PLANT DISEASE CONTROL**

(71) Applicant: Max-Planck-Gesellschaft zur Förderung der Wissenschaften e.V., 80539 München (DE)
(72) Inventor: Griesinger, Christian, 72076 Tübingen (DE); Ruhe, Jonas, 48155 Münster (DE); Ryazanov, Sergey, 37081 Göttingen (DE); Kemen, Eric, 72076 Tübingen (DE); Leonov, Andrei, 37083 Göttingen (DE)
(74) Representative: Arth, Hans-Lothar

(57) **Abstract**

The present invention relates to the use of the compound of the formula (I) and the composition thereof as control agent for plant diseases caused by fungi, oomycetes and bacteria. Plant pathogens produce self-aggregating proteins, like beta-amyloid proteins, that can be important parts of extracellular structures, for example cell walls, adhesion structures to biological surfaces and other pathogenicity related infection structures. This invention discloses that the compound of the formula (I) interferes with the aggregation of such proteins and thus reduce plant pathogen growth significantly.

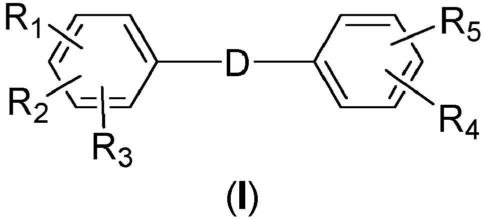

## Description

The present invention relates to the use of the compound of the formula (I) and the composition thereof as control agent for plant diseases caused by fungi, oomycetes and bacteria. Plant pathogens produce self-aggregating proteins, like beta-amyloid proteins, that can be important parts of extracellular structures, for example cell walls, adhesion structures to biological surfaces and other pathogenicity related infection structures. This invention discloses that the compound of the formula (I) interferes with the aggregation of such proteins and thus reduce plant pathogen growth significantly.

### Background of the invention

Plant pathogenic fungi, oomycetes and bacteria have highly diverse lifestyles, infection strategies and morphologies. Therefore, pesticides frequently target basic cellular processes, as they are often very similar in several plant pathogens and well-studied. Many pesticides inhibit enzymes that take part in e.g. nucleic acid synthesis, respiration, cell division and other essential cellular processes. Nevertheless, the molecular targets remain mostly unknown and resistances are constantly evolving (Gisi and Sierotzki, Fungicide modes of action and resistance in downy mildews. Eur. J. Plant Pathol., 2008, 122, 157-167). As microbial plant pests still cause huge losses of crops worldwide, new crop protectants are needed.

The present invention displays a conceptual novelty, as the compounds are not only specific for inhibition of protein functions, but also for protein structures. By inhibiting protein aggregation of amyloid-like proteins, the compounds have significant effects on the growth of plant pathogens. Amyloid proteins harbor structural and functional plasticity. They can change their folding status from monomers, oligomers and protofibrils and form eventually stable fibrils, which changes the protein function (Kumar and Udgaonkar, Mechanisms of amyloid fibril formation by proteins, Curr. Sci. 2010, 98, 639-656). Amyloid proteins can be important components of cell membranes and cell walls, functioning in cell adhesion and biofilm formation, scaffolding, substrate adhesion, modulation of host responses or be cytotoxic and antibacterial (Garcia-Sherman et al., Peptide Detection of Fungal Functional Amyloids in Infected Tissue. PLoS ONE, 2014, 9, e86067; Garcia et al., A Role for Amyloid in Cell Aggregation and Biofilm Formation. PLoS ONE, 2011, 6, e17632; Marcoleta et al., Microcin E492 Amyloid Formation Is Retarded by Posttranslational Modification. J. Bacteriol., 2013, 195, 3995-4004). Regarding this broad functional plasticity, amyloid proteins can be important effectors in plant-pathogen interactions, which are barely described so far. In addition, the mechanism by which these aggregating proteins exert their toxicity is not known and therefore little conclusions can be drawn from amyloid forming proteins known in neurodegenerative diseases.

In the prior art, the medical use of a library of related diphenyl isoxazole/imidazole/oxadiazole/pyrazole compounds have been described. For example, in an international patent application WO2010/000372, such compounds are used as oligomer modulators for treatment or prevention of neurodegenerative diseases, and type II diabetes. A European patent application (EP17170855) discloses the use of such compound in treatment of melanoma occurring in humans. In European patent EP2069318B1 some diphenyl-1,2,4-oxadiazole derivatives are used as agonists for the G protein-coupled receptor S1P1/EDG1 for immunomodulation effects to treat uncontrolled inflammatory disease and to improve vascular functionality. Further, in US patent US 6277872 B1 certain 3,5-diphenyl-1,2,4-oxadiazole compounds are used for the treatment of cerebral ischaemia and neurodegenerative disorders. However, it is still unknown that such diphenyl-1,2,4-oxadiazoles have antimicrobial activity in plants.
Surprisingly, it was found in the present invention that the compound of the formula (I) prevents proteins from amyloid-like aggregation and thus is useful as control agent for plant diseases caused by fungi, oomycetes and bacteria.

### Description of the invention

Accordingly, the present invention relates to the use of a compound of the formula (I) as an active ingredient for treatment or protection of plant diseases caused by fungi, oomycetes or bacteria 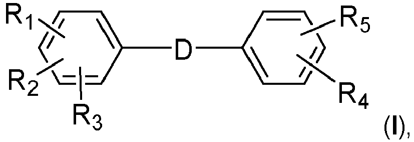 wherein
**D** represents 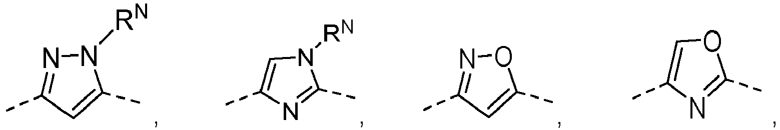 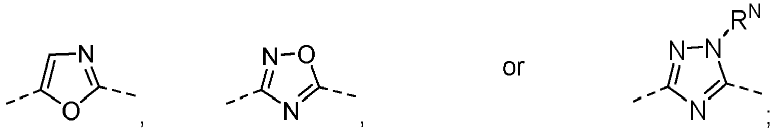
**R₁, R₂, R₃, R₄, R₅** are independently selected from hydrogen, halogen, hydroxy, C₁₋₄ alkoxy, C₁₋₄ alkyl, C₁₋₄ alkylene-OH, C₁₋₄ alkylene-OCH₃, -N**R^{E1}R^{E2}**, -OCF₃, -OCF₂CF₃, -NO₂, -CF₃, -CF₂CF₃, C₁₋₄ alkylthio, -C(=O)CH₃, -C(=O)CF₃, -COO**R^{E3}**, -C(=O)N**R ^{E4}R^{E5}**, -NHC(=O)**R^{E6}**, -NHS(=O)₂**R^{E7}**, wherein at least one of **R₁-R₅** is different from -H; or
**R₁**, and **R₂**, **R₂** and **R₃**, **R₄** and **R₅** together can non-directionally form a structure -T-(C**R^{E8}R^{E9}**)ₙ-V- as well as corresponding structures in which one or two double bond(s) is/are present, if they are attached to adjacent carbon atoms; and **T** is independently selected from C**R^{E10}R^{E11}**, N**R^{E1}** and O; and **V** is independently selected from C**R^{E8}R^{E9}**, N**R^{E1}** and O;
**R^{N}** is independently selected from hydrogen, C₁₋₄ alkyl, C₂₋₄ alkylene-OH, C₂₋₄ alkenyl, -CH₂-O**R^{E1}**; -CH₂CH₂-O**R^{E1}**;
**R^{E1}, R^{E2}** are independetly of each other selected from -H, C₁₋₄ alkyl; or -N**R^{E1}R^{E2}** forms a cyclic amine;
**R^{E3}** is selected from -H, C₁₋₄ alkyl, -CH₂CH₂-O**R^{E1}**;
**R^{E4}, R^{E5}** are independently selected from -H, C₁₋₄ alkyl, 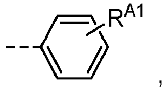 or 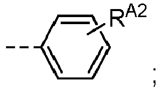 or -N**R^{E4}R^{E5}** forms a cyclic amine;
**R^{E6}** is selected from -H, C₁₋₄ alkyl, -CF₃, -CF₂CF₃, 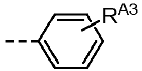 C₁₋₄ alkoxy, -OCH₂CH₂-O**R^{E1}**, -NHCH₂CH₂-O**R^{E1}**, -CH(NH₂)**R^{E12}**; -N**R^{E4}R^{E5}** or -N**R^{E4}R^{E5}** forms a cyclic amine,
**R^{E7}** is selected from -H, C₁₋₄ alkyl, -CF₃, -CF₂CF₃, 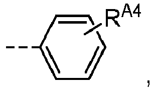
**R^{E8}, R^{E9}, R^{E10}, R^{E11}** are independently -H, -F, or C₁-₄ alkyl;
wherein one or more hydrogens of the C₁₋₄ alkyl, C₁₋₄ alkoxy, and C₁₋₄ alkoxy are optionally substituted by halogen,
**n** is 1 or 2,
**R^{E12}** is selected from the group consisting of: -H, -CH₃, -CH₂OH, -CH₂SH, -CH(OH)CH₃, -CH(CH₃)₂, -CH₂CH(CH₃)₂, -CH(CH₃)CH₂CH₃, -CH₂CO₂H, -CH₂CONH₂, -CH₂CH₂CO₂H, -CH₂CH₂CONH₂, -CH₂CH₂SCH₃, -CH₂CH₂CH₂NH-C(=NH)(NH₂), -CH₂CH₂CH₂CH₂NH₂, 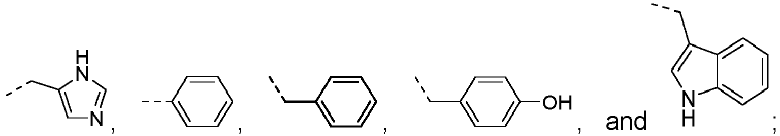
**R^{A1}**, **R^{A2}**, **R^{A3}**, and **R^{A4}**, represent independently of each other -H, -OH, -F, -Br, -CI, -I, -CF₃, -OCF₃, C₁₋₄ alkyl, or C₁₋₄ alkoxy;
with the proviso that when the ring **D** is 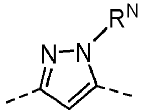 and one of **R₁** - **R₅** is COO**R^{E3}**, COO**R^{E3}** is not substituted at the ortho-position of the phenyl ring of the formula (I),
or tautomers, *N*-oxide, hydrates, solvates, metallic complexes, or acid salt forms thereof.

Halogen represents -F, -CI, -Br, and -I.

C₁₋₄ alkyl represents -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, or -C(CH₃)₃.

C₁₋₄ alkoxy represents -OCH₃, -OCH₂CH₃, -OCH₂CH₂CH₃, -OCH(CH₃)₂, -OCH₂CH₂CH₂CH₃, -OCH(CH₃)CH₂CH₃, -OCH₂CH(CH₃)₂, or -OC(CH₃)₃.

C₁₋₄ alkylene-OH represents -CH₂-OH, -CH₂CH₂-OH, -CH₂CH₂CH₂-OH, -CH(CH₃)CH₂-OH, -CH₂CH₂CH₂CH₂-OH, -CH(CH₃)CH₂CH₂-OH, -CH₂CH(CH₃)CH₂-OH, or -C(CH₃)₂CH₂-OH.

C₁₋₄ alkylene-OCH₃ represents -CH₂-OCH₃, -CH₂CH₂-OCH₃, -CH₂CH₂CH₂-OCH₃, -CH(CH₃)CH₂-OCH₃, -CH₂CH₂CH₂CH₂-OCH₃, -CH(CH₃)CH₂CH₂-OCH₃, -CH₂CH(CH₃)CH₂-OCH₃, or -C(CH₃)₂CH₂-OCH₃.

C₂₋₄ alkenyl represents -CH=CH₂, -CH₂-CH=CH₂, -CH=CH-CH₃, -C(CH₃)=CH₂, -CH=C(CH₃)₂, -CH₂CH₂-CH=CH₂, -CH(CH₃)-CH=CH₂, -CH₂-CH=CH-CH₃, -CH=CH-CH₂CH₃, -C(CH₃)=CH-CH₃, -CH=CH-CH=CH₂. C₁₋₄ alkylthio represents -SCH₃, -SCH₂CH₃, -SCH₂CH₂CH₃, -SCH(CH₃)₂, -SCH₂CH₂CH₂CH₃, -SCH(CH₃)CH₂CH₃, -SCH₂CH(CH₃)₂, -SC(CH₃)₃, -SCH₂CH₂CH₂CH₂CH₃.

The term **cyclic amine** represents 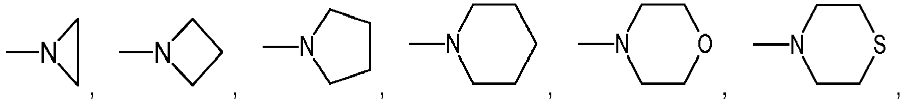 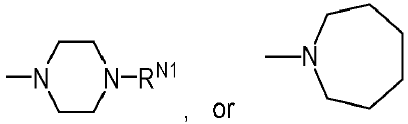 and **R^{N1}** is H or C₁₋₄ alkyl

Acid salt forms: The compounds described herein and, optionally, all their isomers may be obtained in the form of their salts. Because some of the compounds of the formula (I) have a basic center they can, for example, form acid addition salts. Said acid addition salts are, for example, formed with mineral acids, typically sulfuric acid, a phosphoric acid or a hydrogen halide, with organic carboxylic acids, typically acetic acid, oxalic acid, malonic acid, maleic acid, fumaric acid or phthalic acid, with hydroxycarboxylic acids, typically ascorbic acid, lactic acid, malic acid, tartaric acid or citric acid, or with benzoic acid, or with organic sulfonic acids, typically methanesulfonic acid or p-toluenesulfonic acid. Preferably, said acid salt forms are formed with one or more hydrogen halide, in particular, hydrogen bromide, or hydrogen chloride. Within the scope of this invention, agrochemical acceptable salts are preferred.

Metallic complexes preferably refer to transition metal complexes with the compound of the present invention, including but not limited to copper, cobalt, chromium, iron, manganese, nickel, zinc complexes, Preferably, Mn(II), Co(II), Ni(II), Cu(II), Zn(II), Fe(II), Cr(III) complexes and such metal complexes may further contains one or more water molecules.

As described herein, *N*-oxide refers to pyrrolyl *N*-oxide, imidazolyl *N*-oxide, isoxazolyl *N*-oxide, oxazolyl *N*-oxide, oxadiazolyl *N*-oxide, or triazolyl *N*-oxide

Preferred, the invention refers to the use of the compound of the formula (I) as an active ingredient for treatment or protection of plant diseases caused fungi, oomycetes or bacteria. 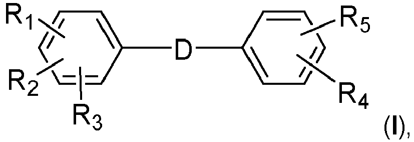 wherein
**D** represents 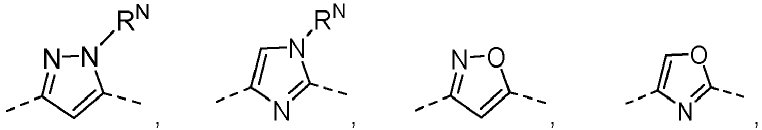 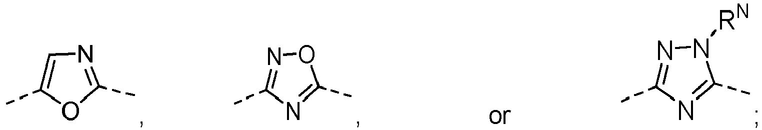
**R₁** - **R₅** represent independently of each other -H, -F, -Br, -CI, -I, -OH, -CF₃, -CH₃, -CH₂CH₃, -CH(CH₃)₂, -OCH₃, -OCH₂CH₃ -OCH(CH₃)₂, -N**R^{E1}R^{E2}**, -NO₂, -SCH₃, -SCH₂CH₃, -NHSO₂CH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃ -NHC(=O)NHCH₂CH₃, -NHC(=O)NHCH(CH₃)₂, -NHC(=O)NHCH₂CH₂OCH₃, -NHC(=O)NHPh, -NHC(=O)NH(4-F-Ph), -NHC(=O)NH(4-MeO-Ph), -NHC(=O)CH(NH₂)CH₂Ph, -NHC(=O)CH(NH₂)CH₃, -NHC(=O)CH(NH₂)CH₂COOH, -NHC(=O)CH(NH₂)CH₂OH, -NHC(=O)CH(NH₂)CH(CH₃)₂, -NHC(=O)CH(NH₂)CH₂CH₂CONH₂, -NHC(=O)CH(NH₂)CH₂CH₂CH₂CH₂NH₂, -NHSO₂Ph, -NHSO₂(4-Cl-Ph), -NHSO₂(4-MeO-Ph), 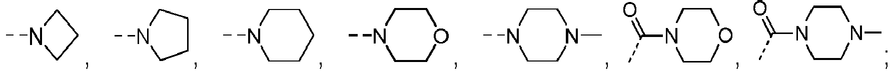 or
wherein at least one of **R₁-R₅** is different from -H; or
**R₁**, and **R₂**, **R₂** and **R₃**, **R₄** and **R₅** form together can non-directionally form a structure -**T**-(C**R^{E8}R^{E9}**)ₙ-**V**- as well as corresponding structures in which one or two double bond(s) is/are present, if they are attached to adjacent carbon atoms and **T** is selected from C**R^{E10}R^{E11}**, N**R^{E1}** and O and **V** is selected from C**R^{E8}R^{E9}**, N**R^{E1}** and O;
**R^{E8}, R^{E9}, R^{E10}, R^{E11}** are independently -H or -F;
**n** is 1 or 2,
**R^{E1}**, and **R^{E2}** are independently selected from -H, C₁₋₄ alkyl; or -N**R^{E1}R^{E2}** forms a cyclic amine;
**R^{N}** is independently selected from hydrogen, C₁₋₄ alkyl, C₂₋₄ alkylene-OH, -C₂₋₄ alkenyl, -CH₂-O**R^{E1}**; -CH₂CH₂-O**R^{E1}** ;
or tautomers, N-oxide, hydrates, solvates, metallic complexes, or acid salt forms thereof.

In one embodiment, the present invention relates to the use of the compound of the formula (**IIa**) as an active ingredient for treatment or protection of plant diseases caused fungi, oomycetes or bacteria: 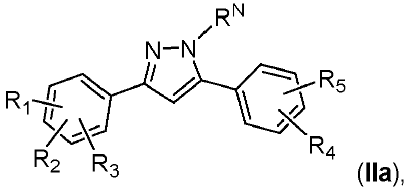 wherein **R₁-R₅** and **R^{N}** have the same meanings as defined herein and when one of **R₁** - **R₅** is COOR^{E3}, COOR^{E3} is not substituted at the ortho-position of the phenyl ring of the formula (**IIa**).

Further, the present invention relates to the use of the compound of the formula (**IIb**) as an active ingredient for treatment or protection of plant diseases caused fungi, oomycetes or bacteria: 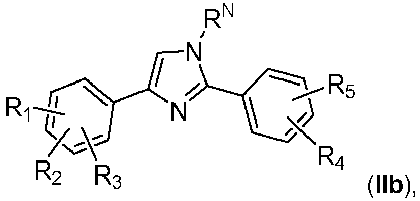 wherein **R₁-R₅** and **R^{N}** have the same meanings as defined above.

Further, the present invention relates to the use of the compound of the formula (**IIc**) as an active ingredient for treatment or protection of plant diseases caused fungi, oomycetes or bacteria: 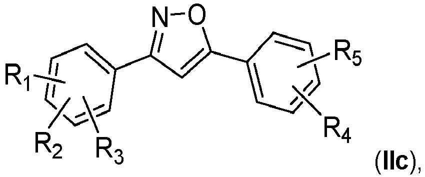 wherein **R₁-R₅** have the same meanings as defined above.

Further, the present invention relates to the use of the compound of the formula (**IId**) as an active ingredient for treatment or protection of plant diseases caused fungi, oomycetes or bacteria: 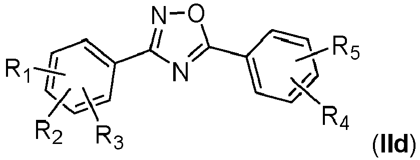 wherein **R₁-R₅** have the same meanings as defined above.

Further, the present invention relates to the use of the compound of the formula (**IIe**) as an active ingredient for treatment or protection of plant diseases caused fungi, oomycetes or bacteria: 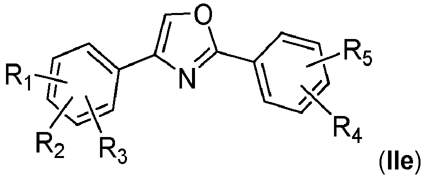 wherein **R₁-R₅** have the same meanings as defined above.

Further, the present invention relates to the use of the compound of the formula (**IIf**) as an active ingredient for treatment or protection of plant diseases caused fungi, oomycetes or bacteria: 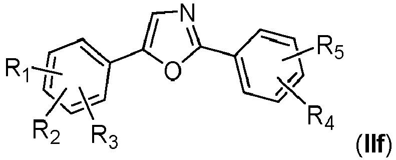 wherein **R₁-R₅** have the same meanings as defined above.

Further, the present invention relates to the use of the compound of the formula (**IIg**) as an active ingredient for treatment or protection of plant diseases caused fungi, oomycetes or bacteria: 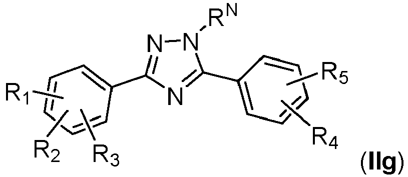 wherein **R₁-R₅** and **R^{N}** have the same meanings as defined above.

Preferred, in any of the formulae (I), (**IIa**) - (**IIg**), **R₁** and **R₂** or **R₂** and **R₃** form together the moiety 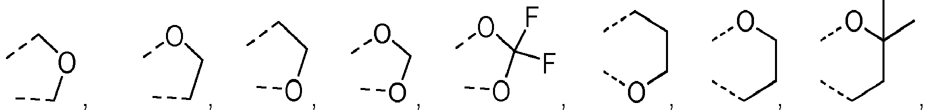 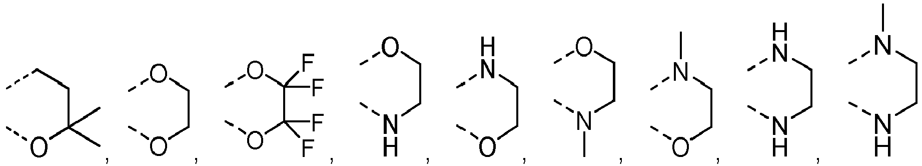 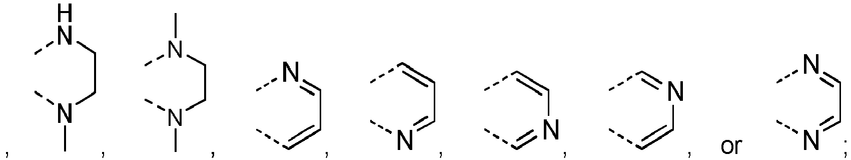 **R₄** and **R₅** forms together the moiety

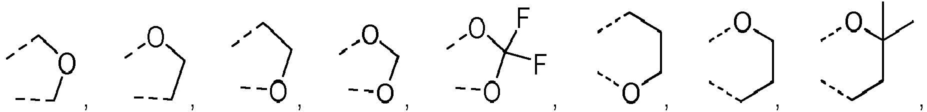

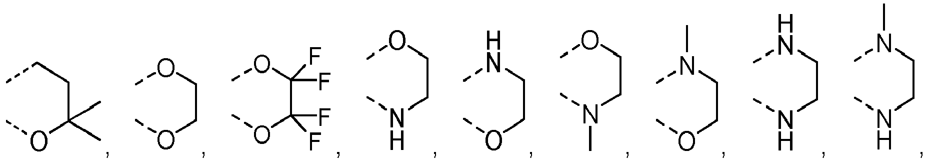

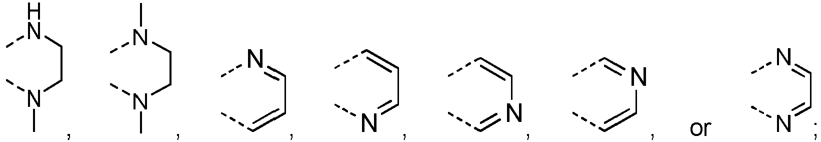

More preferred, **R₁** and **R₂** or **R₂** and **R₃** or **R₄** and **R₅** forms together 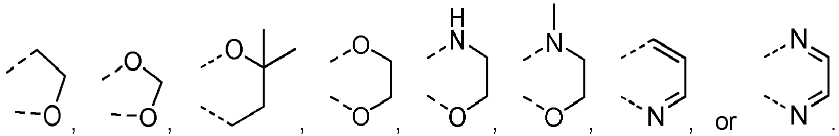 More preferred, in any of the formulae (**I**), (**IIa**) - (**IIg**),
**R₁** - **R₅** represent independently of each other -H, -F, -Br, -CI, -I, -OH, -CF₃, -CH₃, -CH₂CH₃, -CH(CH₃)₂, -OCH₃, -OCH₂CH₃ -OCH(CH₃)₂, -NH₂, -NH(CH₃), -N(CH₃)₂, -NO₂, -SCH₃, -SCH₂CH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃ -NHC(=O)NHCH₂CH₃, -NHC(=O)NHCH(CH₃)₂, -NHC(=O)NHCH₂CH₂OCH₃, -NHC(=O)NHPh, -NHC(=O)NH(4-F-Ph), -NHC(=O)NH(4-MeO-Ph), -NHC(=O)CH(NH₂)CH₂Ph, -NHC(=O)CH(NH₂)CH₃, -NHC(=O)CH(NH₂)CH₂COOH, -NHC(=O)CH(NH₂)CH₂OH, -NHC(=O)CH(NH₂)CH(CH₃)₂, -NHC(=O)CH(NH₂)CH₂CH₂CONH₂, -NHC(=O)CH(NH₂)CH₂CH₂CH₂CH₂NH₂,-NHSO₂CH₃, -NHSO₂Ph, -NHSO₂(4-Cl-Ph), -NHSO₂(4-MeO-Ph), 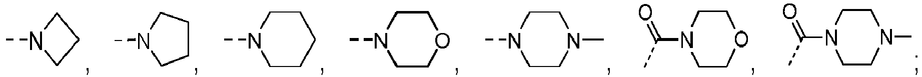
wherein at least one of **R₁** - **R₅** is different from -H; or
**R₁** and **R₂** or **R₂** and **R₃** form together the moiety 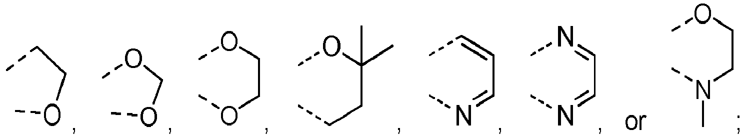 and
**R₄** and **R₅** forms together the moiety 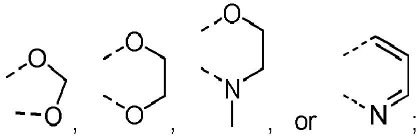
**R^{N}** is independently selected from hydrogen, C₁₋₄ alkyl, C₂₋₄ alkylene-OH, -C₂₋₄ alkenyl, -CH₂-O**R^{E1}**; -CH₂CH₂-O**R^{E1}**; preferably, **R^{N}** represents -H, -CH₃, -CH₂CH₃, or -CH(CH₃)₂, even more preferably **R^{N}** represents -H.

Especially, the present invention is directed to the use of the following compound as an active ingredient for treatment or protection of plant diseases caused fungi, oomycetes or bacteria, and the compound selected from the group consisting of compounds **1 - 144**:

| **Cpd.** | Chemical structure | IUPAC Name |
|---|---|---|
| **1** | 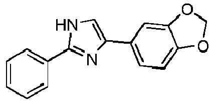 | 4-(1,3-Benzodioxol-5-yl)-2-phenyl-1*H*-imidazole |
| **2** | 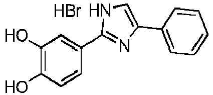 | 4-(3,4-Dihydroxyphenyl)-2-phenyl-1*H*-imidazole hydrobromide |
| **3** | 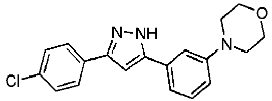 | 4-{3-[3-(4-Chlorophenyl)-1*H*-pyrazol-5-yl]phenyl}morpholine |
| **4** | 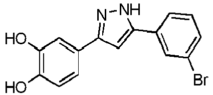 | 3-(3-Bromophenyl)-5-(3,4-dihydroxyphenyl)-1*H-*pyrazole |
| **5** | 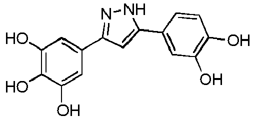 | 3-(3,4-Dihydroxyphenyl)-5-(3,4,5-trihydroxyphenyl)-1*H*-pyrazole |
| **6** | 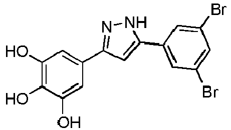 | 3-(3,5-Dibromophenyl)-5-(3,4,5-trihydroxyphenyl)-1*H*-pyrazole |
| **7** | 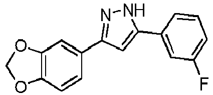 | 3-(1,3-Benzodioxol-5-yl)-5-(3-fluorophenyl)-1*H-*pyrazole |
| **8** | 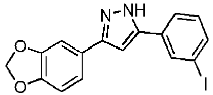 | 3-(1,3-Benzodioxol-5-yl)-5-(3-iodophenyl)-1*H-*pyrazole |
| **9** | 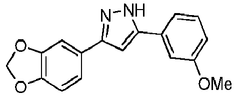 | 3-(1,3-Benzodioxol-5-yl)-5-(3-methoxyphenyl)-1*H-*pyrazole |
| **10** | 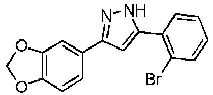 | 3-(1,3-Benzodioxol-5-yl)-5-(2-bromophenyl)-1*H-*pyrazole |
| **11** | 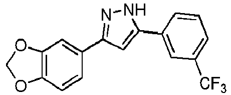 | 3-(1,3-Benzodioxol-5-yl)-5-[3-(trifluoromethyl)phenyl]-1*H*-pyrazole |
| **12** | 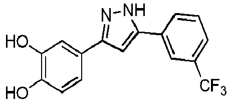 | 3-(3,4-Dihydroxyphenyl)-5-[3-(trifluoromethyl)phenyl]-1*H*-pyrazole |
| **13** | 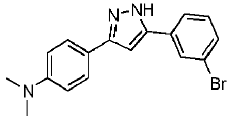 | 3-(3-Bromophenyl)-5-[4-(dimethylamino)phenyl]-1*H-*pyrazole |
| **14** | 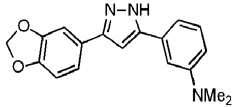 | 3-(1,3-Benzodioxol-5-yl)-5-[3-(dimethylamino)phenyl]-1*H*-pyrazole |
| **15** | 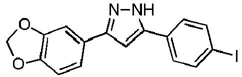 | 3-(1,3-Benzodioxol-5-yl)-5-(4-iodophenyl)-1*H-*pyrazole |
| **16** | 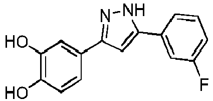 | 3-(3,4-Dihydroxyphenyl)-5-(3-fluorophenyl)-1*H-*pyrazole |
| **17** | 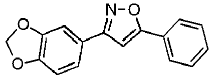 | 3-(1,3-Benzodioxol-5-yl)-5-phenylisoxazole |
| **18** | 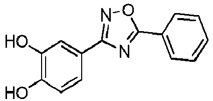 | 3-(3,4-Dihydroxyphenyl)-5-phenyl-1,2,4-oxadiazole |
| **19** | 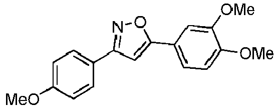 | 5-(3,4-Dimethoxyphenyl)-3-(4-methoxyphenyl) isoxazole |
| **20** | 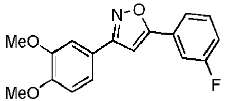 | 3-(3,4-Dimethoxyphenyl)-5-(3-fluorophenyl) isoxazole |
| **21** | 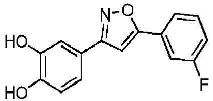 | 3-(3,4-Dihydroxyphenyl)-5-(3-fluorophenyl)isoxazole |
| **22** | 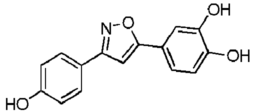 | 5-(3,4-Dihydroxyphenyl)-3-(4-hydroxyphenyl)isoxazole |
| **23** | 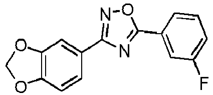 | 3-(1,3-Benzodioxol-5-yl)-5-(3-fluorophenyl)-1,2,4-oxadiazole |
| **24** | 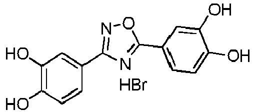 | 3,5-Bis(3,4-Dihydroxyphenyl)-1,2,4-oxadiazole hydrobromide |
| **25** | 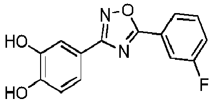 | 3-(3,4-Dihydroxyphenyl)-5-(3-fluorophenyl)-1,2,4-oxadiazole |
| **26** | 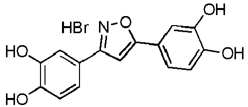 | 3,5-Bis(3,4-Dihydroxyphenyl)isoxazole hydrobromide |
| **27** | 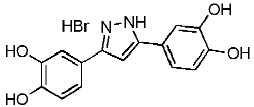 | 3,5-Bis(3,4-Dihydroxyphenyl)-1*H*-pyrazole hydrobromide |
| **28** | 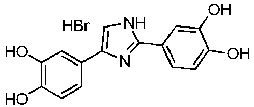 | 2,4-Bis(3,4-dihydroxyphenyl)-1*H*-imidazole hydrobromide |
| **29** | 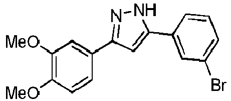 | 3-(3-Bromophenyl)-5-(3,4-dimethoxyphenyl)-1*H-*pyrazole |
| **30** | 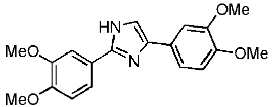 | 2,4-Bis(3,4-dimethoxyphenyl)-1*H*-imidazole |
| **31** | 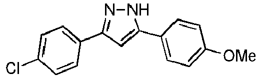 | 3-(4-Chlorophenyl)-5-(4-methoxyphenyl)-1*H-*pyrazole |
| **32** | 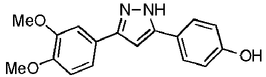 | 3-(3,4-Dimethoxyphenyl)-5-(4-hydroxyphenyl)-1*H-*pyrazole |
| **33** | 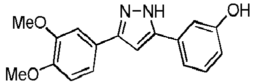 | 3-(3,4-Dimethoxyphenyl)-5-(3-hydroxyphenyl)-1*H-*pyrazole |
| **34** | 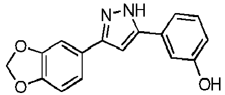 | 3-(1,3-Benzodioxol-5-yl)-5-(3-hydroxyphenyl)-1*H-*pyrazole |
| **35** | 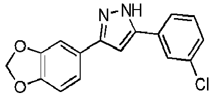 | 3-(1,3-Benzodioxol-5-yl)-5-(3-chlorophenyl)-1*H-*pyrazole |
| **36** | 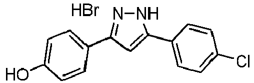 | 3-(4-Chlorophenyl)-5-(4-hydroxyphenyl)-1*H-*pyrazole hydrobromide |
| **37** | 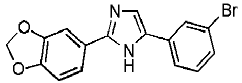 | 2-(1,3-Benzodioxol-5-yl)-4-(3-bromophenyl)-1*H-*imidazole |
| **38** | 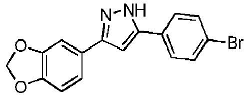 | 3-(1,3-Benzodioxol-5-yl)-5-(4-bromophenyl)-1*H-*pyrazole |
| **39** | 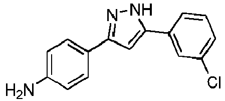 | 3-(4-Aminophenyl)-5-(3-chlorophenyl)-1*H*-pyrazole |
| **40** | 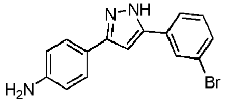 | 3-(4-Aminophenyl)-5-(3-bromophenyl)-1*H*-pyrazole |
| **41** | 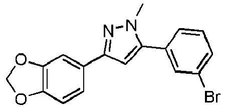 | 3-(1,3-Benzodioxol-5-yl)-5-(3-bromophenyl)-1-methyl-1*H*-pyrazole |
| **42** | 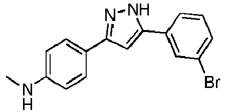 | 3-(3-Bromophenyl)-5-[4-(methylamino)phenyl]-1*H-*pyrazole |
| **43** | 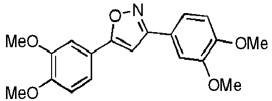 | 3,5-Bis(3,4-dimethoxyphenyl)isoxazole |
| **44** | 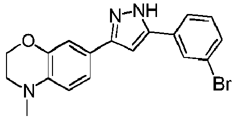 | 7-[5-(3-Bromophenyl)-1*H*-pyrazol-3-yl]-4-methyl-3,4-dihydro-2*H*-1,4-benzoxazine |
| **45** | 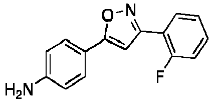 | 5-(4-Aminophenyl)-3-(2-fluorophenyl)-isoxazole |
| **46** | 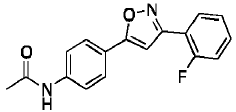 | *N*-{4-[3-(2-Fluorophenyl)-isoxazol-5-yl]phenyl}acetamide |
| **47** | 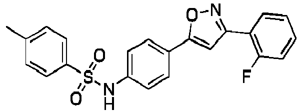 | *N*-{4-[3-(2-Fluorophenyl)-isoxazol-5-yl]phenyl}-4-methylbenzenesulfonamide |
| **48** | 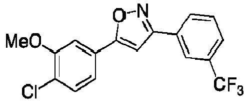 | 5-(4-Chloro-3-methoxyphenyl)-3-[3-(trifluoromethyl)phenyl]-isoxazole |
| **49** | 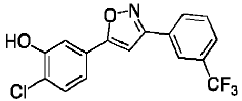 | 5-(4-Chloro-3-hydroxyphenyl)-3-[3-(trifluoromethyl)phenyl]-isoxazole |
| **50** | 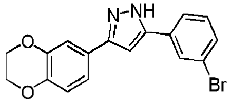 | 3-(3-Bromophenyl)-5-(2,3-dihydro-1,4-benzodioxin-6-yl)-1*H*-pyrazole |
| **51** | 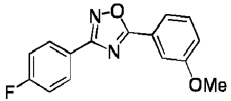 | 3-(4-Fluorophenyl)-5-(3-methoxyphenyl)-1,2,4-oxadiazole |
| **52** | 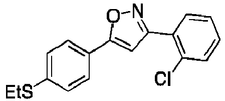 | 3-(2-Chlorophenyl)-5-[4-(ethylsulfanyl)phenyl]-isoxazole |
| **53** | 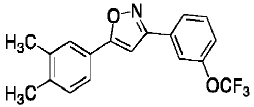 | 5-(3,4-Dimethylphenyl)-3-[3-(trifluoromethoxy)phenyl]-isoxazole |
| **54** | 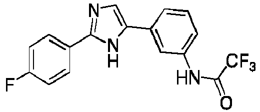 | 2,2,2-Trifluoro-*N*-{3-[2-(4-fluorophenyl)-1*H*-imidazol-5-yl]phenyl}acetamide |
| **55** | 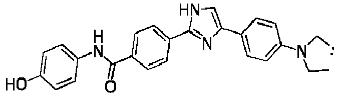 | *N*-(4-Hydroxyphenyl)-4-{4-[4-(pyrrolidin-1-yl)phenyl]-1*H*-imidazol-2-yl}benzamide |
| **56** | 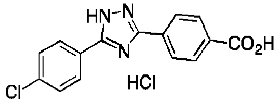 | 4-[5-(4-Chlorophenyl)-1*H*-1,2,4-triazol-3-yl]benzoic acid hydrochloride |
| **57** | 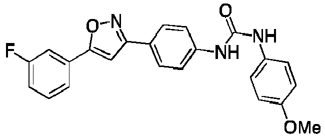 | 1-{4-[5-(3-Fluorophenyl)-1,2-oxazol-3-yl]phenyl}-3-(4-methoxyphenyl)urea |
| **58** | 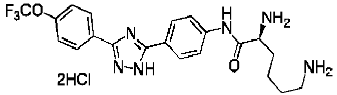 | (2*S*)-2,6-Diamino-*N*-(4-{3-[4-(trifluoromethoxy)phenyl]-1*H*-1,2,4-triazol-5-yl}phenyl)hexanamide dihydrochloride |
| **59** | 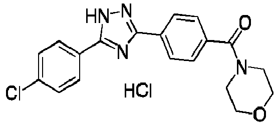 | {4-[5-(4-Chlorophenyl)-1*H*-1,2,4-triazol-3-yl]phenyl}(morpholin-4-yl)methanone hydrochloride |
| **60** | 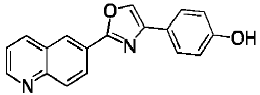 | 6-[4-(4-Hydroxyphenyl)-1,3-oxazol-2-yl]quinoline |
| **61** | 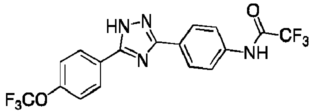 | 2,2,2-Trifluoro-*N*-(4-{5-[4-(trifluoromethoxy)phenyl]-1*H*-1,2,4-triazol-3-yl}phenyl)acetamide |
| **62** | 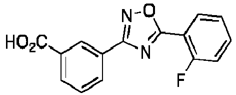 | 3-[5-(2-Fluorophenyl)-1,2,4-oxadiazol-3-yl]benzoic acid |
| **63** | 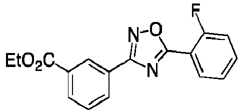 | Ethyl 3-[5-(2-fluorophenyl)-1,2,4-oxadiazol-3-yl]benzoate |
| **64** | 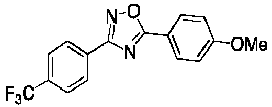 | 5-(4-M ethoxyphenyl)-3-[4-(trifl uorom ethyl) phenyl]-1,2,4-oxadiazole |
| **65** | 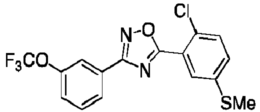 | 5-[2-Chloro-5-(methylsulfanyl)phenyl]-3-[3-(trifluoromethoxy)phenyl]-1,2,4-oxadiazole |
| **66** | 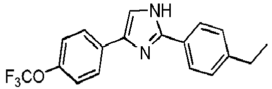 | 2-(4-Ethylphenyl)-4-[4-(trifluoromethoxy)phenyl]-1*H-*imidazole |
| **67** | 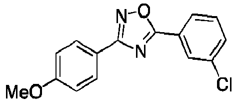 | 5-(3-Chlorophenyl)-3-(4-methoxyphenyl)-1,2,4-oxadiazole |
| **68** | 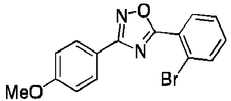 | 5-(2-Bromophenyl)-3-(4-methoxyphenyl)-1,2,4-oxadiazole |
| **69** | 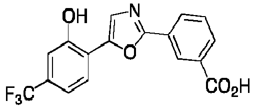 | 3-{5-[2-Hydroxy-4-(trifluoromethyl)phenyl]-1,3-oxazol-2-yl}benzoic acid |
| **70** | 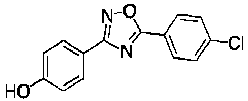 | 5-(4-Chlorophenyl)-3-(4-hydroxyphenyl)-1,2,4-oxadiazole |
| **71** | 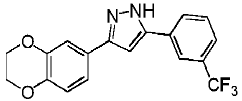 | 3-(2,3-Dihydro-1,4-benzodioxin-6-yl)-5-[3-(trifluoromethyl)phenyl]-1*H*-pyrazole |
| **72** | 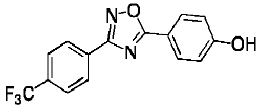 | 5-(4-Hydroxyphenyl]-3-[4-(trifluoromethyl)phenyl]-1,2,4-oxadiazole |
| **73** | 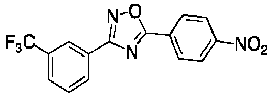 | 5-(4-Nitrophenyl)-3-[3-(trifluoromethyl)phenyl]-1,2,4-oxadiazole |
| **74** | 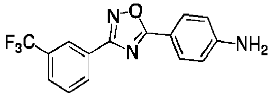 | 5-(4-Aminophenyl)-3-[3-(trifluoromethyl)phenyl]-1,2,4-oxadiazole |
| **75** | 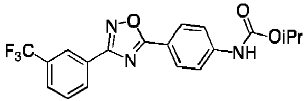 | Propan-2-yl (4-{3-[3-(trifluoromethyl)phenyl]-1,2,4-oxad iazol-5-yl}phenyl)carbamate |
| **76** | 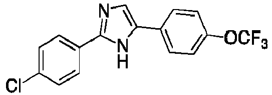 | 2-(4-Chlorophenyl)-5-[4-(trifluoromethoxy)phenyl]-1*H*-imidazole |
| **77** | 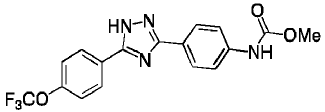 | Methyl (4-{5-[4-(trifluoromethoxy)phenyl]-1*H*-1,2,4-triazol-3-yl}phenyl)carbamate |
| **78** | 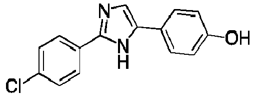 | 2-(4-Chlorophenyl)-5-(4-hydroxyphenyl)-1*H-*imidazole |
| **79** | 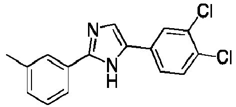 | 5-(3,4-Dichlorophenyl)-2-(3-methylphenyl)-1*H-*imidazole |
| **80** | 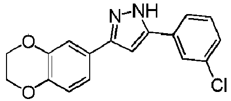 | 5-(3-Chlorophenyl)-3-(2,3-dihydro-1,4-benzodioxin-6-yl)-1*H*-pyrazole |
| **81** | 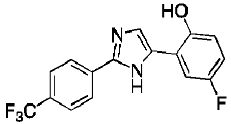 | 5-(2-Hydroxy-5-fluorophenyl)-2-[4-(trifluoromethyl)phenyl]-1*H*-imidazole |
| **82** | 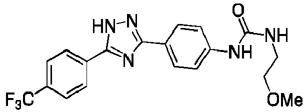 | 1-(2-Methoxyethyl)-3-(4-{5-[4-(trifluoromethyl)phenyl]-1*H*-1,2,4-triazol-3-yl}phenyl)urea |
| **83** | 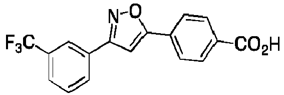 | 4-{3-[3-(Trifluoromethyl)phenyl]-1,2-oxazol-5-yl}benzoic acid |
| **84** | 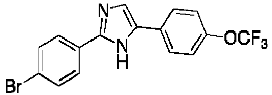 | 2-(4-Bromophenyl)-5-[4-(trifluoromethoxy)phenyl]-1*H*-imidazole |
| **85** | 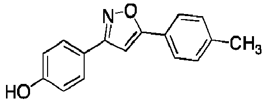 | 3-(4-Hydroxyphenyl)-5-(4-methylphenyl)-1,2-oxazole |
| **86** | 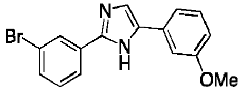 | 2-(3-Bromophenyl)-5-(3-methoxyphenyl)-1*H-*imidazole |
| **87** | 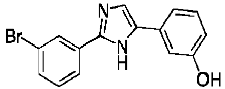 | 2-(3-Bromophenyl)-5-(3-hydroxyphenyl)-1*H-*imidazole |
| **88** | 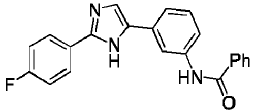 | *N*-{3-[2-(4-fluorophenyl)-1*H*-imidazol-5-yl]phenyl}benzamide |
| **89** | 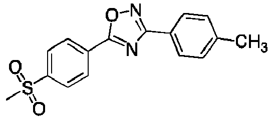 | 3-(4-Methylphenyl)-5-[4-(methylsulfonyl)phenyl]-1,2,4-oxadiazole |
| **90** | 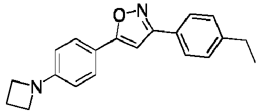 | 5-[4-(Azetidin-1-yl)phenyl]-3-(4-ethylphenyl)-1,2-oxazole |
| **91** | 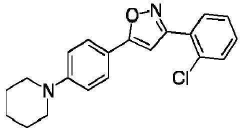 | 1-{4-[3-(2-Chlorophenyl)-1,2-oxazol-5-yl]phenyl}piperidine |
| **92** | 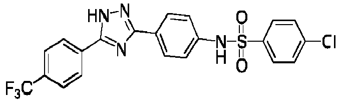 | 4-Chloro-*N*-(4-{5-[4-(trifluoromethyl)phenyl]-1*H-*1,2,4-triazol-3-yl}phenyl)benzenesulfonamide |
| **93** | 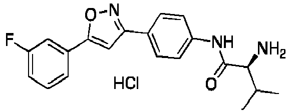 | (2S)-2-Amino-*N*-{4-[5-(3-fluorophenyl)-1,2-oxazol-3-yl] phenyl}-3-methyl butanam ide hydrochloride |
| **94** | 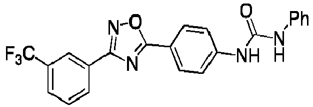 | 1-Phenyl-3-(4-{3-[3-(trifluoromethyl)phenyl]-1,2,4-oxadiazol-5-yl}phenyl)urea |
| **95** | 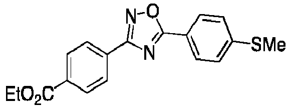 | Ethyl 4-{5-[4-(methylsulfanyl)phenyl]-1,2,4-oxadiazol-3-yl}benzoate |
| **96** | 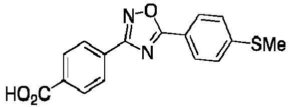 | 4-{5-[4-(Methylsulfanyl)phenyl]-1,2,4-oxadiazol-3-yl}benzoic acid |
| **97** | 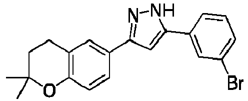 | 5-(3-Bromophenyl)-3-(2,2-dimethyl-3,4-dihydro-2*H-*chromen-6-yl)-1*H*-pyrazole |
| **98** | 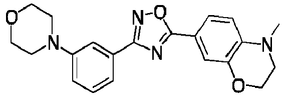 | 4-Methyl-7-{3-[3-(morpholin-4-yl)phenyl]-1,2,4-oxadiazol-5-yl}-3,4-dihydro-2*H*-1,4-benzoxazine |
| **99** | 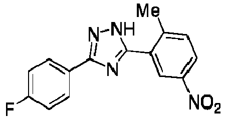 | 3-(4-Fluorophenyl)-5-(2-methyl-5-nitrophenyl)-1*H-*1,2,4-triazole |
| **100** | 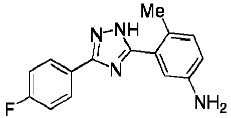 | 5-(3-Amino-6-methylphenyl)-3-(4-fluorophenyl)-1*H-*1,2,4-triazole |
| **101** | 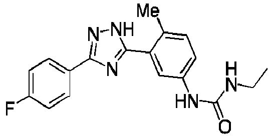 | 1-Ethyl-3-{3-[3-(4-fluorophenyl)-1*H*-1,2,4-triazol-5-yl]-4-methylphenyl}urea |
| **102** | 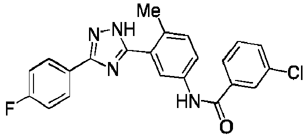 | 3-Chloro-*N*-{3-[3-(4-fluorophenyl)-1*H*-1,2,4-triazol-5-yl]-4-methylphenyl}benzamide |
| **103** | 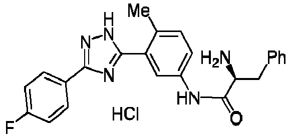 | (2S)-2-Amino-*N*-{3-[3-(4-fluorophenyl)-1*H*-1,2,4-triazol-5-yl]-4-methylphenyl}-3-phenylpropanamide hydrochloride |
| **104** | 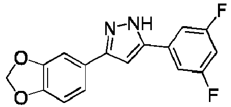 | 3-(1,3-Benzodioxol-5-yl)-5-(3,5-difluorophenyl)-1*H-*pyrazole |
| **105** | 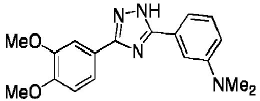 | 3-[3-(3,4-Dimethoxyphenyl)-1*H*-1,2,4-triazol-5-yl]-*N*,*N*-dimethylaniline |
| **106** | 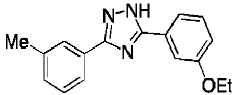 | 5-(3-Ethoxyphenyl)-3-(3-methylphenyl)-1*H*-1,2,4-triazole |
| **107** | 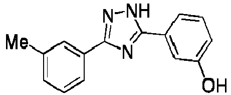 | 5-(3-Hydroxyphenyl)-3-(3-methylphenyl)-1*H*-1,2,4-triazole |
| **108** | 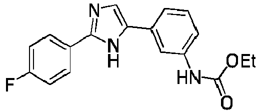 | Ethyl {3-[2-(4-fluorophenyl)-1*H*-imidazol-5-yl]phenyl}carbamate |
| **109** | 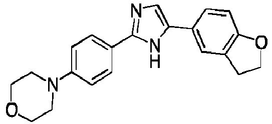 | 4-{4-[5-(2,3-Dihydro-1-benzofuran-5-yl)-1*H-*imidazol-2-yl]phenyl}morpholine |
| **110** | 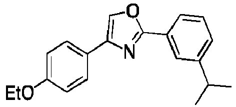 | 4-(4-Ethoxyphenyl)-2-[3-(propan-2-yl)phenyl]-1,3-oxazole |
| **111** | 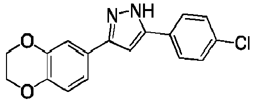 | 5-(4-Chlorophenyl)-3-(2,3-dihydro-1,4-benzodioxin-6-yl)-1*H*-pyrazole |
| **112** | 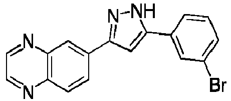 | 6-[5-(3-Bromophenyl)-1*H*-pyrazol-3-yl]quinoxaline |
| **113** | 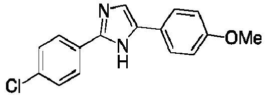 | 2-(4-Chlorophenyl)-5-(4-methoxyphenyl)-1*H-*imidazole |
| **114** | 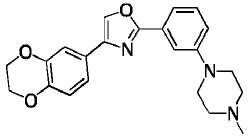 | 1-Methyl-4-{3-[4-(2,3-dihydro-1,4-benzodioxin-6-yl)-1,3-oxazol-2-yl]phenyl}piperazine |
| **115** | 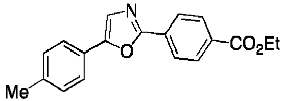 | Ethyl 4-[5-(4-methylphenyl)-1,3-oxazol-2-yl]benzoate |
| **116** | 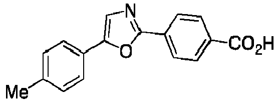 | 4-[5-(4-Methylphenyl)-1,3-oxazol-2-yl]benzoic acid |
| **117** | 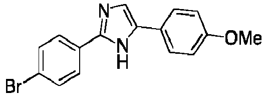 | 2-(4-Bromophenyl)-5-(4-methoxyphenyl)-1*H-*imidazole |
| **118** | 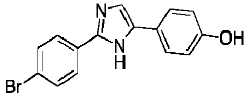 | 2-(4-Bromophenyl)-5-(4-hydroxyphenyl)-1*H-*imidazole |
| **119** | 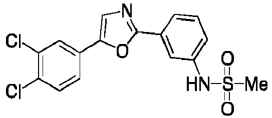 | *N*-{3-[5-(3,4-Dichlorophenyl)-1,3-oxazol-2-yl]phenyl}methanesulfonamide |
| **120** | 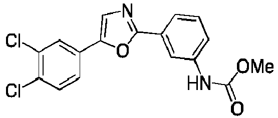 | Methyl {3-[5-(3,4-dichlorophenyl)-1,3-oxazol-2-yl]phenyl}carbamate |
| **121** | 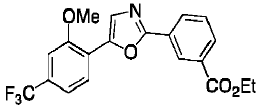 | Ethyl 3-{5-[2-methoxy-4-(trifluoromethyl)phenyl]-1,3-oxazol-2-yl}benzoate |
| **122** | 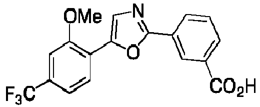 | 3-{5-[2-Methoxy-4-(trifluoromethyl)phenyl]-1,3-oxazol-2-yl}benzoic acid |
| **123** | 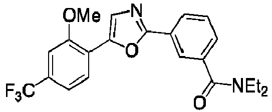 | *N*,*N*-diethyl-3-{5-[2-methoxy-4-(trifluoromethyl)phenyl]-1,3-oxazol-2-yl}benzamide |
| **124** | 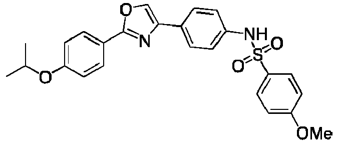 | 4-Methoxy-*N*-{4-[2-[4-(propan-2-yloxy)phenyl]-1,3-oxazol-4-yl]phenyl}benzenesulfonamide |
| **125** | 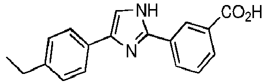 | 3-[4-(4-Ethylphenyl)-1*H*-imidazol-2-yl]benzoic acid |
| **126** | 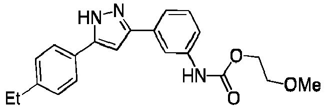 | 2-Methoxyethyl {3-[5-(4-ethylphenyl)-1*H*-pyrazol-3-yl]phenyl}carbamate |
| **127** | 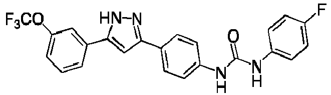 | 1-(4-Fluorophenyl)-3-(4-{5-[3-(trifluoromethoxy)phenyl]-1*H*-pyrazol-3-yl}phenyl)urea |
| **128** | 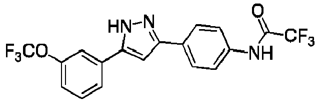 | 2,2,2-Trifluoro-*N*-(4-{5-[3-(trifluoromethoxy)phenyl]-1*H*-pyrazol-3-yl}phenyl)acetamide |
| **129** | 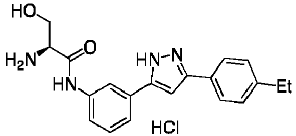 | (2*S*)-2-Amino-*N*-{3-[3-(4-ethylphenyl)-1*H*-pyrazol-5-yl]phenyl}-3-hydroxypropanamide hydrochloride |
| **130** | 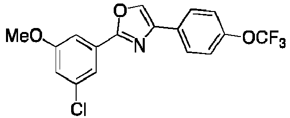 | 2-(3-Chloro-5-methoxyphenyl)-4-[4-(trifluoromethoxy)phenyl]-1,3-oxazole |
| **131** | 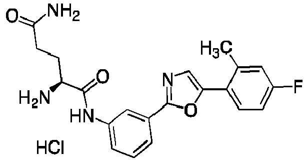 | (2*S*)-2-Amino-*N*¹-{3-[5-(4-fluoro-2-methylphenyl)-1,3-oxazol-2-yl]phenyl}pentanediamide hydrochloride |
| **132** | 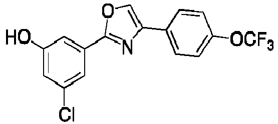 | 2-(3-Chloro-5-hydroxyphenyl)-4-[4-(trifluoromethoxy)phenyl]-1,3-oxazole |
| **133** | 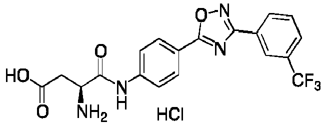 | (3*S*)-3-Amino-4-oxo-4-[(4-{3-[3-(trifluoromethyl)phenyl]-1,2,4-oxadiazol-5-yl}phenyl)amino]butanoic acid hydrochloride |
| **134** | 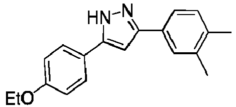 | 3-(3,4-Dimethylphenyl)-5-(4-ethoxyphenyl)-1*H-*pyrazole |
| **135** | 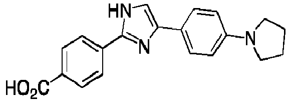 | 4-{4-[4-(Pyrrolidin-1-yl)phenyl]-1*H*-imidazol-2-yl}benzoic acid |
| **136** | 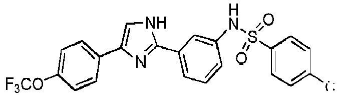 | 4-Chloro-N-(3-{4-[4-(trifluoromethoxy)phenyl]-1*H-*imidazol-2-yl}phenyl)benzenesulfonamide |
| **137** | 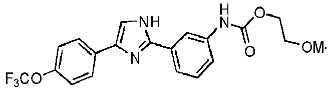 | 2-Methoxyethyl (3-{4-[4-(trifluoromethoxy)phenyl]-1*H*-imidazol-2-yl}phenyl)carbamate |
| **138** | 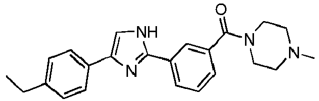 | (4-Methylpiperazin-1-yl){3-[4-(4-methylphenyl)-1*H-*imidazol-2-yl]phenyl}methanone |
| **139** | 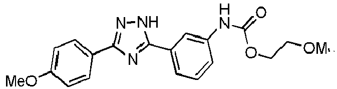 | 2-Methoxyethyl {3-[3-(4-methoxyphenyl)-1*H*-1,2,4-triazol-5-yl]phenyl}carbamate |
| **140** | 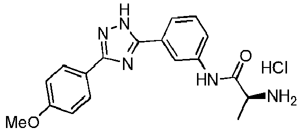 | (2*S*)-2-Amino-*N*-{3-[3-(4-methoxyphenyl)-1*H*-1,2,4-triazol-5-yl]phenyl}propenamide hydrochloride |
| **141** | 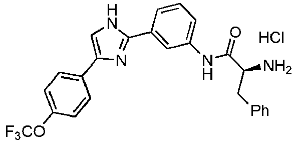 | (2*S*)-2-Amino-*N*-{3-[4-(4-trifluoromethoxyphenyl)-1*H*-imidazol-2-yl]phenyl}-3-phenylpropanamide hydrochloride |
| **142** | 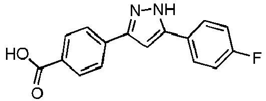 | 4-[5-(4-Fluorophenyl)-1*H*-pyrazol-3-yl]benzoic acid |
| **143** | 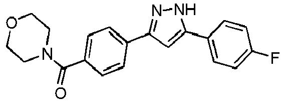 | {4-[5-(4-Fluorophenyl)-1*H*-pyrazol-3-yl]phenyl}(morpholin-4-yl)methanone |
| **144** | 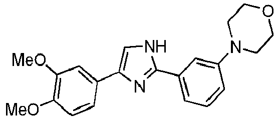 | 4-{3-[4-(3,4-Dimethoxyphenyl)-1*H*-imidazol-2-yl]phenyl}morpholine |

or acid salt forms thereof.

In the present invention, the plant disease caused by fungi, oomycetes or bacteria is selected from the group consisting of: Blumeria diseases, Podosphaera diseases, Sphaerotheca diseases, Uncinula diseases, Gymnosporangium diseases, Hemileia diseases, Phakopsora diseases, Puccinia diseases, Uromyces diseases, Albugo diseases, Bremia diseases, Peronospora diseases, Phytophthora diseases, Plasmopara disease, Pseudoperonospora diseases, Pythium diseases, Altemaria disease, Cercospor diseases, Cladiosporum diseases, Cochliobolus diseases, Colletotrichum diseases, Cycloconium disease, Diaporthe disease, Elsinoe diseases, Gloeosporium diseases, Glomerella diseases, Guignardia, Leptosophaeria diseases, Magnaporthe diseases, Mycosphaerella diseases, Phaeosphaeria diseases, Pyrenophora diseases, Ramularia diseases, Rhynchosporium diseases, Septoria diseases, Typhula diseases, Venturia diseases, Corticium diseases, Fusarium diseases, Gaeumannomyces diseases, Rhizoctonia diseases, Sarocladium diseases, Sclerotium diseases, Tapesia diseases, Thielaviopsis diseases, Alternaria diseases, Aspergillus diseases, Cladosporium diseases, Claviceps diseases, Fusarium diseases, Gibberella diseases, Monographella diseases, Sphacelotheca diseases, Tilletia diseases, Urocystis diseases, Ustilago diseases, Aspergillus diseases, Botrytis diseases, Penicillium diseases, Rhizopus diseases, Sclerotinia diseases, Verticilium diseases, Alternaria diseases, Aphanomyces diseases, Ascochyta diseases, Aspergillus diseases, Cladosporium diseases, Cochliobolus diseases, Colletotrichum diseases, Fusarium diseases, Gibberella diseases, Macrophomina diseases, Monographella diseases, Penicillium diseases, Phoma diseases, Phomopsis diseases, Phytophthora diseases, Pyrenophora diseases, Pyricularia diseases, Pythium diseases, Rhizoctonia diseases, Rhizopus diseases, Sclerotium diseases, Septoria diseases, Typhula diseases, Verticillium diseases, Nectria diseases, Monilinia diseases, Exobasidium diseases, Taphrina diseases, Esca diseases, Eutypa dyeback, Ganoderma diseases, Rigidoporus diseases, Botrytis diseases, Rhizoctonia diseases, Helminthosporium diseases, Hymenoscyphus diseases, and Plasmodiophora diseases.

Preferably, the plant disease caused by fungi, oomycetes or bacteria is selected from the group consisting of:
Blumeria diseases caused by Blumeria graminis, Podosphaera diseases caused by Podosphaera leucotricha, Sphaerotheca diseases caused by Sphaerotheca fuliginea, Uncinula diseases caused by Uncinula necator, Gymnosporangium diseases caused by Gymnosporangium sabinae, Hemileia diseases caused by Hemileia vastatrix, Phakopsora diseases caused by Phakopsora pachyrhizi or Phakopsora meibomiae, Puccinia diseases caused by Puccinia recondite, Puccinia graminis or Puccinia striiformis, Uromyces diseases caused by Uromyces appendiculatus, U. pisi, U fabae or U. striatus, Albugo diseases caused by Albugo candida, Bremia diseases caused by Bremia lactucae, Peronospora diseases caused by Peronospora pisi or P. brassicae, Phytophthora diseases caused by Phytophthora infestans, P. capsica, P. cinnamomi, P. nicotianae, P. palmivora, P. fragariae or P. sojae, Plasmopara disease caused by Plasmopara viticola, Pseudoperonospora diseases caused by Pseudoperonospora humuli or Pseudoperonospora cubensis, Pythium diseases caused by Pythium ultimum, Altemaria disease caused by Alternaria solani, Cercospor diseases caused by Cercospora beticola, Cladiosporum diseases caused by Cladiosporium cucumerinum, Cochliobolus diseases caused by Cochliobolus sativus (Conidiaform Drechslera, Syn: Helminthosporium) or Cochliobolus miyabeanus, Colletotrichum diseases caused by Colletotrichum lindemuthanium, Cycloconium disease caused by by Cycloconium oleaginum, Diaporthe disease caused by Diaporthe citri, Elsinoe diseases caused by Elsinoe fawcettii, Gloeosporium diseases caused by Gloeosporium laeticolor, Glomerella diseases caused by Glomerella cingulata, Guignardia caused by Guignardia bidwelli, Leptosophaeria diseases caused by Leptosphaeria maculans or Leptosphaeria maculans, Magnaporthe diseases caused by Magnaporthe grisea, Mycosphaerella diseases caused by Mycosphaerella graminicola, Mycosphaerella arachidicola or Mycosphaerella fijiensis, Phaeosphaeria diseases caused by Phaeosphaeria nodorum, Pyrenophora diseases caused by Pyrenophora teres, or Pyrenophora tritici repentis, Ramularia diseases caused by Ramularia collo-cygni , or Ramularia areola, Rhynchosporium diseases caused by Rhynchosporium secalis, Septoria diseases caused by Septoria apii or Septoria lycopercisi, Typhula diseases caused by Typhula incarnata, Venturia diseases caused by Venturia inaequalis, Corticium diseases caused by Corticium graminearum, Fusarium diseases caused by Fusarium oxysporum, Gaeumannomyces diseases caused by Gaeumannomyces graminis, Rhizoctonia diseases caused by Rhizoctonia solani, Sarocladium diseases caused by Sarocladium oryzae, Sclerotium diseases caused by Sclerotium oryzae, Tapesia diseases caused by Tapesia acuformis, Thielaviopsis diseases caused by Thielaviopsis basicola, Alternaria diseases caused by Alternaria spp., Aspergillus diseases caused by Aspergillus flavus, Cladosporium diseases caused by Cladosporium spp., Claviceps diseases caused by Claviceps purpurea, Fusarium diseases caused by Fusarium culmorum, Gibberella diseases caused by Gibberella zeae, Monographella diseases caused by Monographella nivalis, Sphacelotheca diseases caused by Sphacelotheca reiliana, Tilletia diseases caused by Tilletia caries, Urocystis diseases caused by Urocystis occulta, Ustilago diseases caused by Ustilago nuda, U. maydis or U. hordei, Aspergillus diseases caused by Aspergillus flavus, Botrytis diseases caused by Botrytis cinerea, Penicillium diseases caused by Penicillium expansum, Rhizopus diseases caused by Rhizopus stolonifer, Sclerotinia diseases caused by Sclerotinia sclerotiorum, Verticilium diseases caused by Verticilium alboatrum, Alternaria diseases caused by Alternaria brassicicola, Aphanomyces diseases caused by Aphanomyces euteiches, Ascochyta diseases caused by Ascochyta lentis Aspergillus diseases caused by Aspergillus flavus, Cladosporium diseases caused by Cladosporium herbarum, Cochliobolus diseases caused by Cochliobolus sativus, Colletotrichum diseases caused by Colletotrichum coccodes, Fusarium diseases caused by Fusarium culmorum, Gibberella diseases caused by Gibberella zeae, Macrophomina diseases caused by Macrophomina phaseolina, Monographella diseases caused by Monographella nivalis, Penicillium diseases caused by Penicillium expansum, Phoma diseases caused by Phoma lingam, Phomopsis diseases caused by Phomopsis sojae, Phytophthora diseases caused by Phytophthora cactorum, Pyrenophora diseases caused by Pyrenophora graminea, Pyricularia diseases caused by Pyricularia oryzae, Pythium diseases caused by Pythium ultimum, Rhizoctonia diseases caused by Rhizoctonia solani, Rhizopus diseases caused by Rhizopus oryzae, Sclerotium diseases caused by Sclerotium rolfsii, Septoria diseases caused by Septoria nodorum, Typhula diseases caused by Typhula incarnata, Verticillium diseases caused by Verticillium dahliae, Nectria diseases caused by Nectria galligena, Monilinia diseases caused by Monilinia laxa, Exobasidium diseases caused by Exobasidium vexans, Taphrina diseases caused by Taphrina deformans, Esca diseases caused by Phaemoniella clamydospora, Eutypa dyeback caused by Eutypa lata, Ganoderma diseases caused by Ganoderma boninense, Rigidoporus diseases caused by Rigidoporus lignosus, Botrytis diseases caused by Botrytis cinerea, Rhizoctonia diseases caused by Rhizoctonia solani, Helminthosporium diseases caused by Helminthosporium solani, Ash dieback caused by Hymenoscyphus sp and Plasmodiophora diseases caused by Plasmodiophora brassicae.

Preferably, the compound of the formula (I) can be used for treatment or protection of plant disease caused by oomycetes, wherein the plant disease caused by oomycetes is Albugo disease caused by Albugo Candida or Albugo laibachii, Bremia disease caused by Bremia lactucae, Peronospora disease caused by Peronospora pisi or P. brassicae, Phytophthora disease caused by Phytophthora infestans, Plasmopara disease caused by Plasmopara viticola, and Pseudoperonospora disease caused by Pseudoperonospora humuli or Pseudoperonospora cubensis.

The present invention is directed to a compound of formula (I) 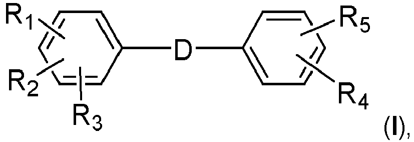 wherein
**D** represents 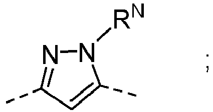
**R₁** and **R₂** are hydrogen and **R₃** represents -CI, hydroxyl, -OCF₃, -OC₂H₅, -C₂H₅, -NH-CO-CH(NH₂)-CH₂-OH, or
**R₁** is hydrogen and **R₂** and **R₃** form together the residue -O-CH₂-O-;
**R₄** is hydrogen and **R₅** represents -C₂H₅, -N(CH₃)₂, -NH-CO-CF₃, -I, -NH-C=OO-CH₂CH₂-OCH₃, -NH-C=O-NH-**R^{E5}** or
**R₄** and **R₅** represent -CH₃, or 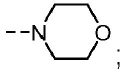
**R^{E5}** represents 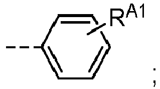
**R^{A1}** represents -F;
**R^{N}** represents hydrogen;
or tautomers, *N*-oxide, hydrates, solvates, metallic complexes, or acid salt forms thereof.

Thus, the present invention is related to the following preferred compounds:

| **Cpd** | **Name** |
|---|---|
| **3** | 4-{3-[3-(4-Chlorophenyl)-1*H*-pyrazol-5-yl]phenyl}morpholine |
| **14** | 3-(1,3-Benzodioxol-5-yl)-5-[3-(dimethylamino)phenyl]-1*H*-pyrazole |
| **15** | 3-(1,3-Benzodioxol-5-yl)-5-(4-iodophenyl)-1*H*-pyrazole |
| **126** | 2-Methoxyethyl{3-[5-(4-ethylphenyl)-1*H*-pyrazol-3-yl]phenyl}carbamate |
| **127** | 1-(4-Fluorophenyl)-3-(4-{5-[3-(trifluoromethoxy)phenyl]-1*H*-pyrazol-3-yl}phenyl)urea |
| **128** | 2,2,2-Trifluoro-*N*-(4-{5-[3-(trifluoromethoxy)phenyl]-1*H*-pyrazol-3-yl}phenyl)acetamide |
| **129** | (2*S*)-2-Amino-*N*-{3-[3-(4-ethylphenyl)-1*H*-pyrazol-5-yl]phenyl}-3-hydroxypropanamide hydrochloride |
| **134** | 3-(3,4-Dimethylphenyl)-5-(4-ethoxyphenyl)-1*H*-pyrazole |

The present invention is also directed to a compound of formula (I) 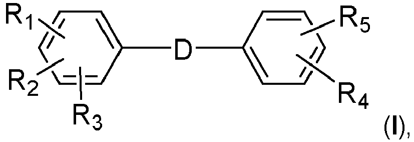 wherein
**D** represents 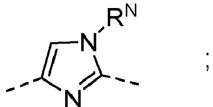
**R₁** and **R₂** are hydrogen and **R₃** represents -NH-CO-CF₃, -OCF₃, 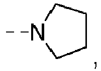 -OH, -NH-CO-Ph, -NH-CO-OC₂H₅, -C₂H₅, or
**R₁** is hydrogen and **R₂** and **R₃** represent independently of each other -CI, -OCH₃, -OH, -F; or
**R₁** is hydrogen and **R₂** and **R₃** form together the residue -CH₂-CH₂-O-;
**R₄** is hydrogen and **R₅** represents -CH₃, -C₂H₅, -F, -CI, -Br, -CF₃, -C=ONH**R^{E5}**, 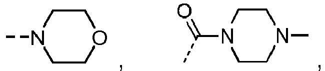 -NH-COO-CH₂CH₂-OCH₃ -NHS(=O)₂**R^{E7}**, -NH-CO-CH(NH₂)-CH₂Ph, -N(CH₃)-CH₂CH₂-OCH₃, -COOH; or
**R₄** and **R₅** represent -OCH₃;
**R^{E5}** represents 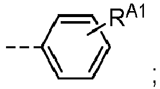
**R^{E7}** represents 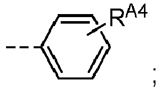
**R^{A1}** represents -OH;
**R^{A4}** represents -Cl;
**R^{N}** represents hydrogen;
or tautomers, *N*-oxide, hydrates, solvates, metallic complexes, or acid salt forms thereof.

Thus, the present invention is related to the following preferred compounds:

| **Cpd** | **Name** |
|---|---|
| **30** | 2,4-Bis(3,4-dimethoxyphenyl)-1*H*-imidazole |
| **54** | 2,2,2-Trifluoro-*N*-{3-[2-(4-fluorophenyl)-1*H*-imidazol-5-yl]phenyl}acetamide |
| **55** | *N*-(4-Hydroxyphenyl)-4-{4-[4-(pyrrolidin-1-yl)phenyl]-1*H*-imidazol-2-yl}benzamide |
| **66** | 2-(4-Ethylphenyl)-4-[4-(trifluoromethoxy)phenyl]-1*H*-imidazole |
| **76** | 2-(4-Chlorophenyl)-5-[4-(trifluoromethoxy)phenyl]-1*H*-imidazole |
| **78** | 2-(4-Chlorophenyl)-5-(4-hydroxyphenyl)-1*H*-imidazole |
| **79** | 5-(3,4-Dichlorophenyl)-2-(3-methylphenyl)-1*H*-imidazole |
| **81** | 5-(2-Hydroxy-5-fluorophenyl)-2-[4-(trifluoromethyl)phenyl]-1*H*-imidazole |
| **84** | 2-(4-Bromophenyl)-5-[4-(trifluoromethoxy)phenyl]-1*H*-imidazole |
| **87** | 2-(3-Bromophenyl)-5-(3-hydroxyphenyl)-1*H*-imidazole |
| **88** | *N*-{3-[2-(4-fluorophenyl)-1*H*-imidazol-5-yl]phenyl}benzamide |
| **108** | Ethyl {3-[2-(4-fluorophenyl)-1*H*-imidazol-5-yl]phenyl}carbamate |
| **109** | 4-{4-[5-(2,3-Dihydro-1-benzofuran-5-yl)-1*H*-imidazol-2-yl]phenyl}morpholine |
| **118** | 2-(4-Bromophenyl)-5-(4-hydroxyphenyl)-1*H*-imidazole |
| **125** | 3-[4-(4-Ethylphenyl)-1*H*-imidazol-2-yl]benzoic acid |
| **135** | 4-{4-[4-(Pyrrolidin-1-yl)phenyl]-1*H*-imidazol-2-yl}benzoic acid |
| **136** | 4-Chloro-N-(3-{4-[4-(trifluoromethoxy)phenyl]-1*H*-imidazol-2-yl}phenyl)benzenesulfonamide |
| **137** | 2-Methoxyethyl(3-{4-[4-(trifluoromethoxy)phenyl]-1*H*-imidazol-2-yl}phenyl)carbamate |
| **138** | (4-Methylpiperazin-1-yl){3-[4-(4-methylphenyl)-1*H*-imidazol-2-yl]phenyl}methanone |
| **141** | (2*S*)-2-Amino-*N*-{3-[4-(4-trifluoromethoxyphenyl)-1*H*-imidazol-2-yl]phenyl}-3-phenylpropanamide hydrochloride |
| **144** | 4-{3-[4-(3,4-Dimethoxyphenyl)-1*H*-imidazol-2-yl]phenyl}morpholine |

The present invention is also directed to a compound of formula (I) 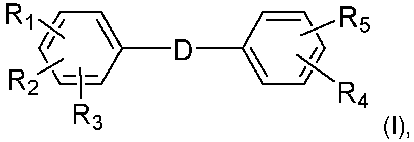 wherein
**D** represents 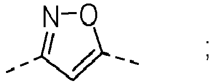
**R₁** and **R₂** are hydrogen and **R₃** represents -CI, -C₂H₅, -CF₃, -NHC(=O)-NH**R^{E5}**, -F, -OCF₃, -NH-CO-CH(NH₂)-CH(CH₃)₂;
**R₄** is hydrogen and **R₅** represents -NH₂, -SC₂H₅, -F, 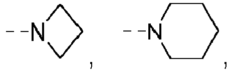 -NHS(=O)₂**R^{E7}**, -COOH, -NH-CO-CH₃; or
**R₄** and **R₅** represent independently of each other -CI, -OCH₃, -OH, -CH₃;
**R^{E5}** represents 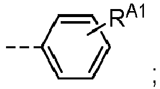
**R^{E7}** represents 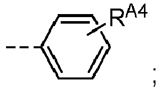
**R^{A1}** represents -OCH₃;
**R^{A4}** represents -CH₃;
or tautomers, *N*-oxide, hydrates, solvates, metallic complexes, or acid salt forms thereof.

Thus the present invention is related to the following preferred compounds:

| **Cpd** | **Name** |
|---|---|
| **90** | 5-[4-(Azetidin-1-yl)phenyl]-3-(4-ethylphenyl)-1,2-oxazole |
| **91** | 1-{4-[3-(2-Chlorophenyl)-1,2-oxazol-5-yl]phenyl}piperidine |
| **83** | 4-{3-[3-(Trifluoromethyl)phenyl]-1,2-oxazol-5-yl}benzoic acid |
| **57** | 1-{4-[5-(3-Fluorophenyl)-1,2-oxazol-3-yl]phenyl}-3-(4-methoxyphenyl)urea |
| **45** | 5-(4-Aminophenyl)-3-(2-fluorophenyl)-isoxazole |
| **46** | *N*-{4-[3-(2-Fluorophenyl)-isoxazol-5-yl]phenyl}acetamide |
| **47** | *N*-{4-[3-(2-Fluorophenyl)-isoxazol-5-yl]phenyl}-4-methylbenzenesulfonamide |
| **48** | 5-(4-Chloro-3-methoxyphenyl)-3-[3-(trifluoromethyl)phenyl]-isoxazole |
| **49** | 5-(4-Chloro-3-hydroxyphenyl)-3-[3-(trifluoromethyl)phenyl]-isoxazole |
| **52** | 3-(2-Chlorophenyl)-5-[4-(ethylsulfanyl)phenyl]-isoxazole |
| **53** | 5-(3,4-Dimethylphenyl)-3-[3-(trifluoromethoxy)phenyl]-isoxazole |
| **93** | (2*S*)-2-Amino-*N*-{4-[5-(3-fluorophenyl)-1,2-oxazol-3-yl]phenyl}-3-methylbutanamide hydrochloride |

The present invention is also directed to a compound of formula (I) 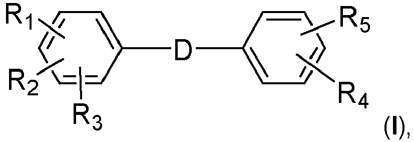 wherein
**D** represents 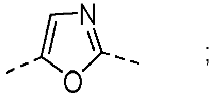
**R₁** and **R₂** are hydrogen and **R₃** represents -CH₃, or
**R₁** is hydrogen and **R₂** and **R₃** represent independently of each other -OH, -CF₃, -CI, -OCH₃, -CH₃, -F;
**R₄** is hydrogen and **R₅** represents -COOH, -COOC₂H₅, -NH-SO₂-CH₃, -NH-CO-OCH₃, -CO-N(C₂H₅)₂, -NH-CO-CH(NH₂)-CH₂CH₂-CO-NH₂;
or tautomers, *N*-oxide, hydrates, solvates, metallic complexes, or acid salt forms thereof.

Thus, the present invention is related to the following preferred compounds:

| **Cpd** | **Name** |
|---|---|
| **69** | 3-{5-[2-Hydroxy-4-(trifluoromethyl)phenyl]-1,3-oxazol-2-yl}benzoic acid |
| **115** | Ethyl 4-[5-(4-methylphenyl)-1,3-oxazol-2-yl]benzoate |
| **119** | *N*-{3-[5-(3,4-Dichlorophenyl)-1,3-oxazol-2-yl]phenyl}methanesulfonamide |
| **120** | Methyl {3-[5-(3,4-dichlorophenyl)-1,3-oxazol-2-yl]phenyl}carbamate |
| **121** | Ethyl 3-{5-[2-methoxy-4-(trifluoromethyl)phenyl]-1,3-oxazol-2-yl}benzoate |
| **122** | 3-{5-[2-Methoxy-4-(trifluoromethyl)phenyl]-1,3-oxazol-2-yl}benzoic acid |
| **123** | *N*,*N*-diethyl-3-{5-[2-methoxy-4-(trifluoromethyl)phenyl]-1,3-oxazol-2-yl}benzamide |
| **131** | (2*S*)-2-Amino-*N*¹-{3-[5-(4-fluoro-2-methylphenyl)-1,3-oxazol-2-yl]phenyl}pentanediamide hydrochloride |

The present invention is also directed to a compound of formula (I) 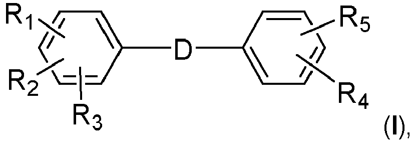 wherein
D represents 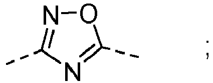
**R₁** and **R₂** are hydrogen and **R₃** represents -OCF₃, -COOC₂H₅, -CF₃, -CH₃, -COOH, 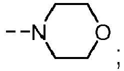
**R₄** is hydrogen and **R₅** represents -SO₂CH₃, -SCH₃, -F, -NH-CO-CH(CH₃)₂, -NH-CO-NH-Ph, -NH-CO-CH(NH₂)-CH₂-COOH; or
**R₄** and **R₅** represent independently of each other -CI, -SCH₃; or
**R₄** and **R₅** form together the residue -O-CH₂-CH₂-N(CH₃)-;
or tautomers, *N*-oxide, hydrates, solvates, metallic complexes, or acid salt forms thereof.

Thus, the present invention is related to the following preferred compounds:

| **Cpd** | **Name** |
|---|---|
| **63** | Ethyl 3-[5-(2-fluorophenyl)-1,2,4-oxadiazol-3-yl]benzoate |
| **65** | 5-[2-Chloro-5-(methylsulfanyl)phenyl]-3-[3-(trifluoromethoxy)phenyl]-1,2,4-oxadiazole |
| **75** | Propan-2-yl (4-{3-[3-(trifluoromethyl)phenyl]-1 ,2,4-oxadiazol-5-yl}phenyl)carbamate |
| **89** | 3-(4-Methylphenyl)-5-[4-(methylsulfonyl)phenyl]-1,2,4-oxadiazole |
| **94** | 1-Phenyl-3-(4-{3-[3-(trifluoromethyl)phenyl]-1,2,4-oxadiazol-5-yl}phenyl)urea |
| **95** | Ethyl 4-{5-[4-(methylsulfanyl)phenyl]-1,2,4-oxadiazol-3-yl}benzoate |
| **96** | 4-{5-[4-(Methylsulfanyl)phenyl]-1,2,4-oxadiazol-3-yl}benzoic acid |
| **98** | 4-Methyl-7-{3-[3-(morpholin-4-yl)phenyl]-1,2,4-oxadiazol-5-yl}-3,4-dihydro-2*H-*1,4-benzoxazine |
| **133** | (3*S*)-3-Amino-4-oxo-4-[(4-{3-[3-(trifluoromethyl)phenyl]-1,2,4-oxadiazol-5-yl}phenyl)amino]butanoic acid hydrochloride |

The present invention is also directed to a compound of formula (I) 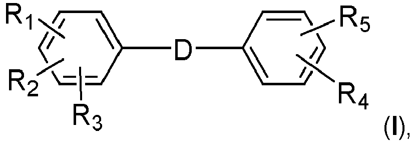 wherein
**D** represents 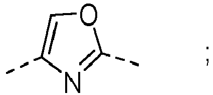
**R₁** and **R₂** are hydrogen and **R₃** represents hydroxyl, -OCF₃, -OC₂H₅, -NHS(=O)₂**R^{E7}**, or
**R₁** is hydrogen and **R₂** and **R₃** form together the residue -O-(CH₂CH₂)ₙ-O-;
**R₄** represents hydrogen and **R₅** represents -OCH(CH₃)₂, -CH(CH₃)₂, 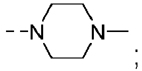 or
**R₄** and **R₅** represent -CI, -OCH₃, -OH, or
**R₄** and **R₅** form together the residue -N=CH-CH=CH-;
**R^{E7}** represents 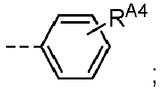
**R^{A4}** represents -OCH₃;
or tautomers, *N*-oxide, hydrates, solvates, metallic complexes, or acid salt forms thereof.

Thus, the present invention is related to the following preferred compounds:

| **Cpd** | **Name** |
|---|---|
| **60** | 6-[4-(4-Hydroxyphenyl)-1,3-oxazol-2-yl]quinoline |
| **130** | 2-(3-Chloro-5-methoxyphenyl)-4-[4-(trifluoromethoxy)phenyl]-1,3-oxazole |
| **132** | 2-(3-Chloro-5-hydroxyphenyl)-4-[4-(trifluoromethoxy)phenyl]-1,3-oxazole |
| **124** | 4-Methoxy-*N*-{4-[2-[4-(propan-2-yloxy)phenyl]-1,3-oxazol-4-yl]phenyl}benzenesulfonamide |
| **110** | 4-(4-Ethoxyphenyl)-2-[3-(propan-2-yl)phenyl]-1,3-oxazole |
| **114** | 1-Methyl-4-{3-[4-(2,3-dihydro-1,4-benzodioxin-6-yl)-1,3-oxazol-2-yl]phenyl}piperazine |

The present invention is also directed to a compound of formula (I) 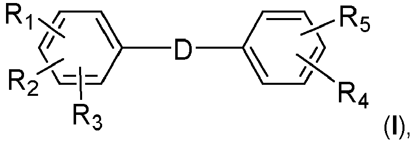 wherein
**D** represents 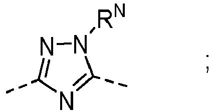
**R₁** and **R₂** are hydrogen and **R₃** represents -F, -CI, -OCF₃, -CF₃, -N(CH₃)₂, -CH₃, -OCH₃;
**R₄** represents hydrogen and **R₅** represents -COOH, -NH-CO-CH(NH₂)-(CH₂)₄-NH₂, 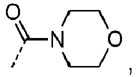 -NH-CO-CF₃, -NH-CO-OCH₃, -NH-CO-NH-CH₂CH₂-OCH₃, -NHS(=O)₂**R^{E7}**, -OC₂H₅, -NH-CO-O-CH₂CH₂-OCH₃; or
**R₄** and **R₅** represent independently of each other -CH₃, -NH₂, -NO₂, -NH-CO-NH-CH₂CH₃, -NHC(=O)**R^{E6}**, -NH-CO-CH(NH₂)-CH₂-Ph, -OCH₃, -NH-CO-CH(NH₂)-CH₃;
**R^{E6}** represents 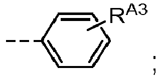
**R^{E7}** represents 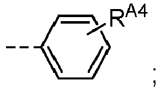
**R^{A3}** represents -Cl;
**R^{A4}** represents -Cl;
or tautomers, *N*-oxide, hydrates, solvates, metallic complexes, or acid salt forms thereof;

Thus, the present invention is related to the following preferred comoounds:

| **Cpd** | **Name** |
|---|---|
| **56** | 4-[5-(4-Chlorophenyl)-1*H*-1,2,4-triazol-3-yl]benzoic acid hydrochloride |
| **58** | (2*S*)-2,6-Diamino-*N*-(4-{3-[4-(trifluoromethoxy)phenyl]-1*H*-1,2,4-triazol-5-yl}phenyl)hexanamide dihydrochloride |
| **59** | {4-[5-(4-Chlorophenyl)-1*H*-1,2,4-triazol-3-yl]phenyl}(morpholin-4-yl)methanone hydrochloride |
| **61** | 2,2,2-Trifluoro-*N*-(4-{5-[4-(trifluoromethoxy)phenyl]-1*H*-1,2,4-triazol-3-yl}phenyl)acetamide |
| **77** | Methyl (4-{5-[4-(trifluoromethoxy)phenyl]-1*H*-1,2,4-triazol-3-yl}phenyl)carbamate |
| **82** | 1-(2-Methoxyethyl)-3-(4-{5-[4-(trifluoromethyl)phenyl]-1*H*-1,2,4-triazol-3-yl}phenyl)urea |
| **92** | 4-Chloro-*N*-(4-{5-[4-(trifluoromethyl)phenyl]-1*H*-1,2,4-triazol-3-yl}phenyl)benzenesulfonamide |
| **99** | 3-(4-Fluorophenyl)-5-(2-methyl-5-nitrophenyl)-1*H*-1,2,4-triazole |
| **100** | 5-(3-Amino-6-methylphenyl)-3-(4-fluorophenyl)-1*H*-1,2,4-triazole |
| **101** | 1-Ethyl-3-{3-[3-(4-fluorophenyl)-1*H*-1,2,4-triazol-5-yl]-4-methylphenyl}urea |
| **102** | 3-Chloro-*N*-{3-[3-(4-fluorophenyl)-1*H*-1,2,4-triazol-5-yl]-4-methylphenyl}benzamide |
| **103** | (2*S*)-2-Amino-*N*-{3-[3-(4-fluorophenyl)-1*H*-1,2,4-triazol-5-yl]-4-methylphenyl}-3-phenylpropanamide hydrochloride |
| **105** | 3-[3-(3,4-Dimethoxyphenyl)-1*H*-1,2,4-triazol-5-yl]-*N*,*N*-dimethylaniline |
| **106** | 5-(3-Ethoxyphenyl)-3-(3-methylphenyl)-1*H*-1,2,4-triazole |
| **139** | 2-Methoxyethyl {3-[3-(4-methoxyphenyl)-1*H*-1,2,4-triazol-5-yl]phenyI}carbamate |
| **140** | (2*S*)-2-Amino-*N*-{3-[3-(4-methoxyphenyl)-1*H*-1,2,4-triazol-5-yl]phenyl}propenamide hydrochloride |

In one embodiment, the present invention is directed to a composition comprising as an active ingredient, an effective amount of a compound of formula (I) and an agriculturally acceptable support, carrier or filler:

The present invention is directed to a composition comprising as an active ingredient, an effective amount of a compound of formula (**I**) and an agriculturally acceptable support, carrier or filler: 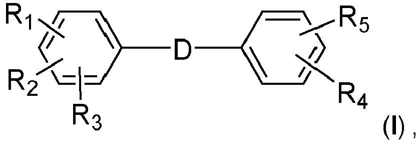 wherein
**D** represents 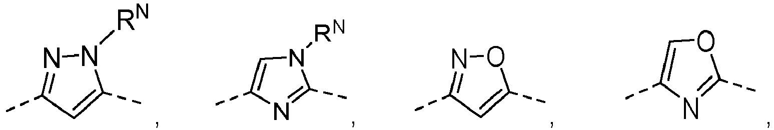 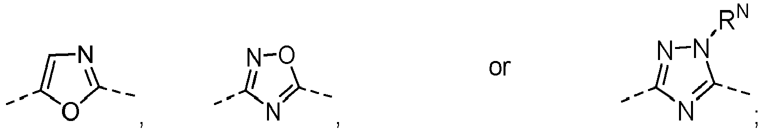
**R₁, R₂, R₃, R₄, R₅** are independently selected from hydrogen, halogen, hydroxy, C₁₋₄ alkoxy, C₁₋₄ alkyl, C₁₋₄ alkylene-OH, C₁₋₄ alkylene-OCH₃, -N**R^{E1}R^{E2}**, -OCF₃, -OCF₂CF₃, -NO₂, -CF₃, -CF₂CF₃, C₁₋₄ alkylthio, -C(=O)CH₃, -C(=O)CF₃, -COO**R^{E3}**, -C(=O)N**R^{E4}R^{E5}**, -NHC(=O)**R^{E6}**, -NHS(=O)₂**R^{E7}**, wherein at least one of **R₁-R₅** is different from -H; or
**R₁**, and **R₂**, **R₂** and **R₃**, **R₄** and **R₅** together can non-directionally form a structure -**T**-(C**R^{E8}R^{E9}**)ₙ-**V**- as well as corresponding structures in which one or two double bond(s) is/are present, if they are attached to adjacent carbon atoms; and **T** is independently selected from C**R^{E10}R^{E11}**, N**R^{E1}** and O; and **V** is independently selected from C**R^{E8}R^{E9}**, N**R^{E1}** and O;
**R^{N}** is independently selected from hydrogen, C₁₋₄ alkyl, C₂₋₄ alkylene-OH, C₂₋₄ alkenyl, -CH₂-O**R^{E1}**; -CH₂CH₂-O**R^{E1}**
**R^{E1}, R^{E2}** are independently of each other selected from -H, C₁₋₄ alkyl; or -N**R^{E1}R^{E2}** forms a cyclic amine;
**R^{E3}** is selected from -H, C₁₋₄ alkyl, -CH₂CH₂-O**R^{E1}**;
**R^{E4}, R^{E5}** are independently selected from -H, C₁₋₄ alkyl, 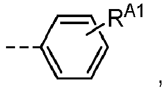 or 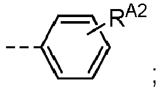 or -N**R^{E4}R^{E5}** forms a cyclic amine;
**R^{E6}** is selected from -H, C₁₋₄ alkyl, -CF₃, -CF₂CF₃, 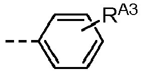 C₁₋₄ alkoxy, -OCH₂CH₂-O**R^{E1}**, -NHCH₂CH₂-O**R^{E1}**, -CH(NH₂)**R^{E12}**; -N**R^{E4}R^{E5}** or -N**R^{E4}R^{E5}** forms a cyclic amine.
**R^{E7}** is selected from -H, C₁₋₄ alkyl, -CF₃, -CF₂CF₃, 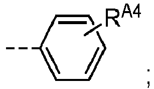
**R^{E8}, R^{E9}, R^{E10}, R^{E11}** are independently -H, -F, or C₁₋₄ alkyl;
wherein one or more hydrogens of the C₁₋₄ alkyl, C₁₋₄ alkoxy, and C₁₋₄ alkoxy are optionally substituted by halogen,
**n** is 1 or 2,
**R^{E12}** is selected from the group consisting of: -H, -CH₃, -CH₂OH, -CH₂SH, -CH(OH)CH₃, -CH(CH₃)₂, -CH₂CH(CH₃)₂, -CH(CH₃)CH₂CH₃, -CH₂CO₂H, -CH₂CONH₂, -CH₂CH₂CO₂H, -CH₂CH₂CONH₂, -CH₂CH₂SCH₃, -CH₂CH₂CH₂NH-C(=NH)(NH₂), -CH₂CH₂CH₂CH₂NH₂, 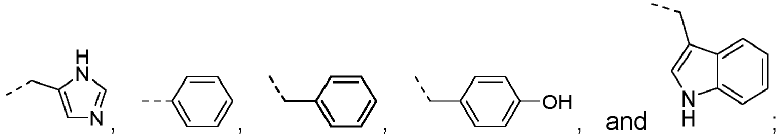
**R^{A1}**, **R^{A2}**, **R^{A3}**, and **R^{A4}**, represent independently of each other -H, -OH, -F, -Br, -CI, -I, -CF₃, -OCF₃, C₁₋₄ alkyl, or C₁₋₄ alkoxy; with the proviso that when the ring D is 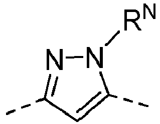 and one of **R₁** - **R₅** is COO**R^{E3}**, COO**R^{E3}** is not substituted at the ortho-position of the phenyl ring of the formula (I),
   or tautomers, *N*-oxide, hydrates, solvates, metallic complexes, or acid salt forms thereof.

Preferably, the composition comprises as an active ingredient, an effective amount of the compound of formula (**I**) 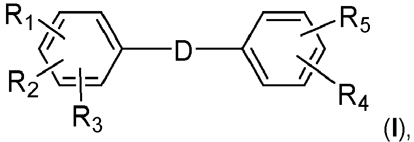 wherein
**D** represents 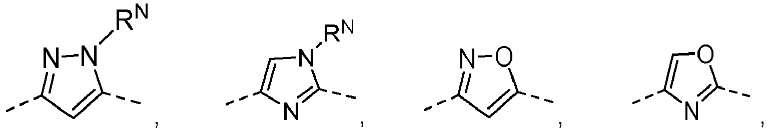 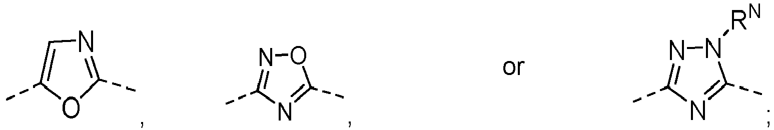
**R₁** - **R₅** represent independently of each other -H, -F, -Br, -CI, -I, -OH, -CF₃, -CH₃, -CH₂CH₃, -CH(CH₃)₂, -OCH₃, -OCH₂CH₃, -OCH(CH₃)₂, -N**R^{E1}R^{E2}**, -NO₂, -SCH₃, -SCH₂CH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃ -NHC(=O)NHCH₂CH₃, -NHC(=O)NHCH(CH₃)₂, -NHC(=O)NHCH₂CH₂OCH₃, -NHC(=O)NHPh, -NHC(=O)NH(4-F-Ph), -NHC(=O)NH(4-MeO-Ph), -NHC(=O)CH(NH₂)CH₂Ph, -NHC(=O)CH(NH₂)CH₃, -NHC(=O)CH(NH₂)CH₂COOH, -NHC(=O)CH(NH₂)CH₂OH, -NHC(=O)CH(NH₂)CH(CH₃)₂, -NHC(=O)CH(NH₂)CH₂CH₂CONH₂, -NHC(=O)CH(NH₂)CH₂CH₂CH₂CH₂NH₂, -NHSO₂CH₃, -NHSO₂Ph, -NHSO₂(4-Cl-Ph), -NHSO₂(4-MeO-Ph), 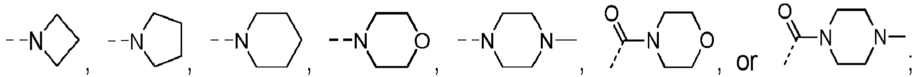 wherein at least one of **R₁-R₅** is different from -H; or
**R₁**, and **R₂**, **R₂** and **R₃**, **R₄** and **R₅** form together can non-directionally form a structure **-T-**(C**R^{E8}R^{E9}**)ₙ**-V-** as well as corresponding structures in which one or two double bond(s) is/are present, if they are attached to adjacent carbon atoms; and **T** is selected from C**R^{E10}R^{E11}**, N**R^{E1}** and O; and **V** is selected from C**R^{E8}R^{E9}**, N**R^{E1}** and O;
**R^{E8}**, **R^{E9}**, **R^{E10}**, **R^{E11}** are independently -H, -F, or C₁₋₄ alkyl;
**n** is 1 or 2,
**R^{E1}**, and **R^{E2}** are independently selected from -H, C₁₋₄ alkyl; or -N**R^{E1}R^{E2}** forms a cyclic amine;
**R^{N}** is independently selected from hydrogen, C₁₋₄ alkyl, C₂₋₄ alkylene-OH, -C₂₋₄ alkenyl, -CH₂-O**R^{E1}**, -CH₂CH₂-O**R^{E1}**;
or tautomers, N-oxide, hydrates, solvates, metallic complexes, or acid salt forms thereof.

More preferably, the composition comprises as an active ingredient, an effective amount of the compound of formula (**I**) 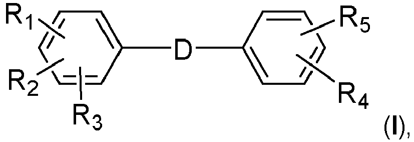 wherein
**D** represents 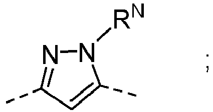
**R₁** and **R₂** are hydrogen and **R₃** represents -CI, hydroxyl, -OCF₃, -OC₂H₅, -C₂H₅, -NH-CO-CH(NH₂)-CH₂-OH, or
**R₁** is hydrogen and **R₂** and **R₃** form together the residue -O-CH₂-O-;
**R₄** is hydrogen and **R₅** represents -C₂H₅, -N(CH₃)₂, -NH-CO-CF₃, -I, -NH-COO-CH₂CH₂-OCH₃, -NH-C=O-NH-**R^{E5}** or
**R₄** and **R₅** represent -CH₃, or 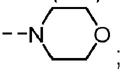
**R^{E5}** represents 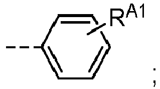
**R^{A1}** represents -F;
**R^{N}** represents hydrogen;
or tautomers, *N*-oxide, hydrates, solvates, metallic complexes, or acid salt forms thereof;
or
**D** represents 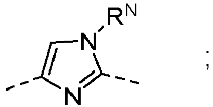
**R₁** and **R₂** are hydrogen and **R₃** represents -NH-CO-CF₃, -OCF₃, 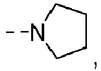 -OH, -NH-CO-Ph, -NH-CO-OC₂H₅, -C₂H₅, or
**R₁** is hydrogen and **R₂** and **R₃** represent independently of each other -CI, -OCH₃, -OH, -F;
**R₁** is hydrogen and **R₂** and **R₃** form together the residue -CH₂-CH₂-O-;
**R₄** is hydrogen and **R₅** represents -CH₃, -C₂H₅, -F, -CI, -Br, -CF₃, -C(=O)NH**R^{E5}**, 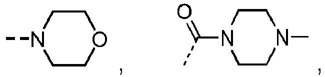 -NH-COO-CH₂CH₂-OCH₃, -NHS(=O)₂**R^{E7}**, -NH-CO-CH(NH₂)-CH₂Ph, -N(CH₃)-CH₂CH₂-OCH₃, -COOH; or
**R₄** and **R₅** represent -OCH₃;
**R^{E5}** represents 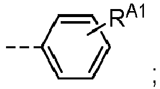
**R^{E7}** represents 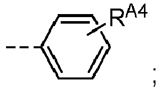
**R^{A1}** represents -OH;
**R^{A4}** represents -Cl;
**R^{N}** represents hydrogen; or tautomers, *N*-oxide, hydrates, solvates, metallic complexes, or acid salt forms thereof;
or
**D** represents 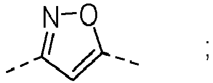
**R₁** and **R₂** are hydrogen and **R₃** represents -CI, -C₂H₅, -CF₃, -NHC(=O)-NH**R^{E5}**, -F, -OCF₃, -NH-CO-CH(NH₂)-CH(CH₃)₂;
**R₄** is hydrogen and **R₅** represents -NH₂, -SC₂H₅, -F, 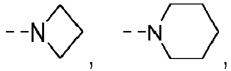 -NHS(=O)₂**R^{E7}**, -COOH, -NH-CO-CH₃; or
**R₄** and **R₅** represent independently of each other -CI, -OCH₃, -OH, -CH₃;
**R^{E5}** represents 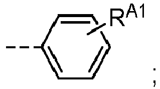
**R^{E7}** represents 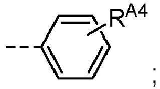
**R^{A1}** represents -OCH₃;
**R^{A4}** represents -CH₃;
or tautomers, *N*-oxide, hydrates, solvates, metallic complexes, or acid salt forms thereof;
or
**D** represents 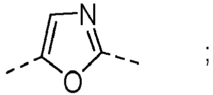
**R₁** and **R₂** are hydrogen and **R₃** represents -CH₃,
**R₁** is hydrogen and **R₂** and **R₃** represent independently of each other -OH, -CF₃, -CI, -OCH₃, -CH₃, -F;
**R₄** is hydrogen and **R₅** represents -COOH, -COOC₂H₅, -NH-SO₂-CH₃, -NH-CO-OCH₃, -CO-N(C₂H₅)₂, -NH-CO-CH(NH₂)-CH₂CH₂-CO-NH₂; or
or tautomers, *N*-oxide, hydrates, solvates, metallic complexes, or acid salt forms thereof;
or
**D** represents 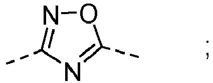
**R₁** and **R₂** are hydrogen and **R₃** represents -OCF₃, -COOC₂H₅, -CF₃, -CH₃, -COOH, 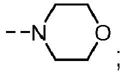
**R₄** is hydrogen and **R₅** represents -SO₂CH₃, -SCH₃, -F, -NH-CO-CH(CH₃)₂, -NH-CO-NH-Ph, -NH-CO-CH(NH₂)-CH₂-COOH; or
**R₄** and **R₅** represent independently of each other -CI, -SCH₃; or
**R₄** and **R₅** form together the residue -O-CH₂-CH₂-N(CH₃)-;
or tautomers, *N*-oxide, hydrates, solvates, metallic complexes, or acid salt forms thereof;
or
**D** represents 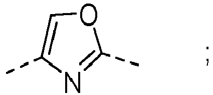
**R₁** and **R₂** are hydrogen and **R₃** represents hydroxyl, -OCF₃, -OC₂H₅, -NHS(=O)₂**R^{E7}**, or
**R₁** is hydrogen and **R₂** and **R₃** form together the residue -O-(CH₂CH₂)ₙ-O-;
**R₄** represents hydrogen and **R₅** represents -OCH(CH₃)₂, -CH(CH₃)₂, 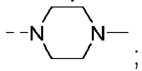 or
**R₄** and **R₅** represent -CI, -OCH₃, -OH, or
**R₄** and **R₅** form together the residue -N=CH-CH=CH-;
**R^{E7}** represents 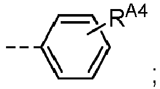
**R^{A4}** represents -OCH₃;
or tautomers, *N*-oxide, hydrates, solvates, metallic complexes, or acid salt forms thereof;
or
**D** represents 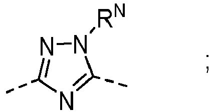
**R₁** and **R₂** are hydrogen and **R₃** represents -F, -CI, -OCF₃, -CF₃, -N(CH₃)₂, -CH₃, -OCH₃;
**R₄** represents hydrogen and **R₅** represents -COOH, -NH-CO-CH(NH₂)-(CH₂)₄-NH₂, 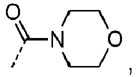 -NH-CO-CF₃, -NH-CO-OCH₃, -NH-CO-NH-CH₂CH₂-OCH₃, -NHS(=O)₂**R^{E7}**, -OC₂H₅, -NH-CO-O-CH₂CH₂-OCH₃; or
**R₄** and **R₅** represent independently of each other -CH₃, -NH₂, -NO₂, -NH-CO-NH-CH₂CH₃, -NHC(=O)**R^{E6}**, -NH-CO-CH(NH₂)-CH₂-Ph, -OCH₃, -NH-CO-CH(NH₂)-CH₃;
**R^{E6}** represents 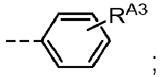
**R^{E7}** represents 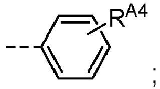
**R^{A3}** represents -Cl;
**R^{A4}** represents -Cl;
or tautomers, *N*-oxide, hydrates, solvates, metallic complexes, or acid salt forms thereof.

Also preferred, the composition comprises as an active ingredient, an effective amount of the compound of any of following formulae **(IIa)** to **(IIg)** as an active ingredient:

More preferred, the composition comprises as an active ingredient, an effective amount of a compound of formula (**IIa**): 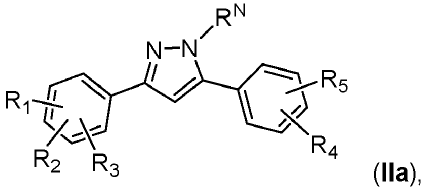 wherein **R₁-R₅** and **R^{N}** have the same meanings as defined herein and when one of **R₁** - **R₅** is -COOR^{E3}, -COOR^{E3} is not substituted at the ortho-position of the phenyl ring of the formula (**IIa**).

Still more preferred, the composition comprises as an active ingredient, an effective amount of a compound of formula (**IIb**): 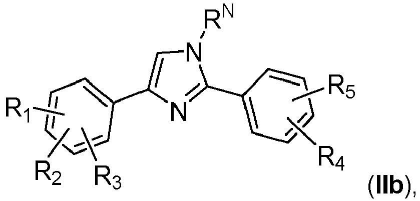 wherein **R₁-R₅** and **R^{N}** have the same meanings as defined herein.

Still more preferred, the composition comprises as an active ingredient, an effective amount of a compound of formula (**IIc**): 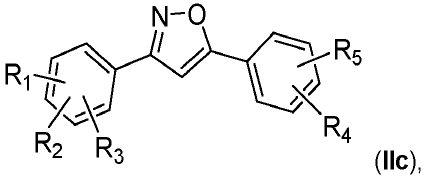 wherein **R₁-R₅** have the same meanings as defined herein.

Still more preferred, the composition comprises as an active ingredient, an effective amount of a compound of formula (**IId**): 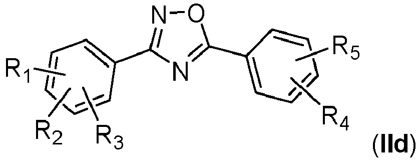 wherein **R₁-R₅** have the same meanings as defined herein.

Still more preferred, the composition comprises as an active ingredient, an effective amount of a compound of formula (**IIe**): 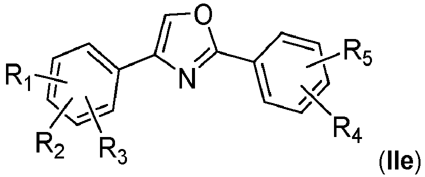 wherein **R₁-R₅** have the same meanings as defined herein.

Still more preferred, the composition comprises as an active ingredient, an effective amount of a compound of formula (**IIf**): 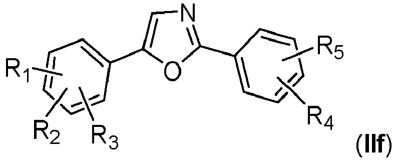 wherein **R₁-R₅** have the same meanings as defined herein.

Still more preferred, the composition comprises as an active ingredient, an effective amount of a compound of formula (**IIg**): 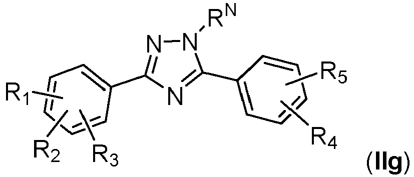 wherein **R₁-R₅** and **R^{N}** have the same meanings as defined herein.

Preferred, in any of the formulae (**I**), **(IIa)** - (**IIg**), **R₁** and **R₂** or **R₂** and **R₃** form together the following moiety 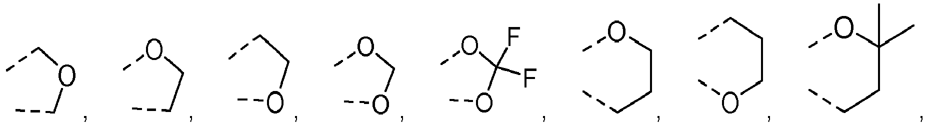 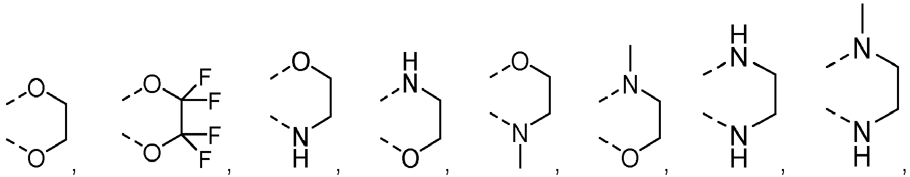 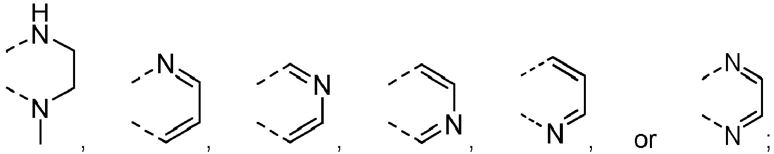 **R₄** and **R₅** forms together the following moiety

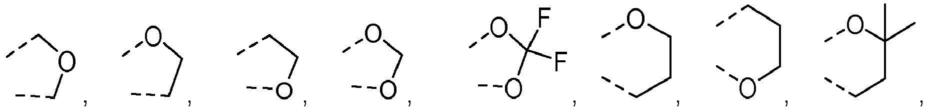

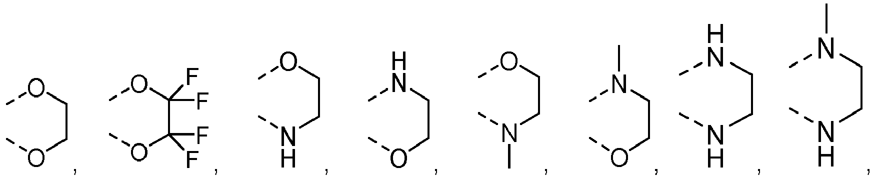

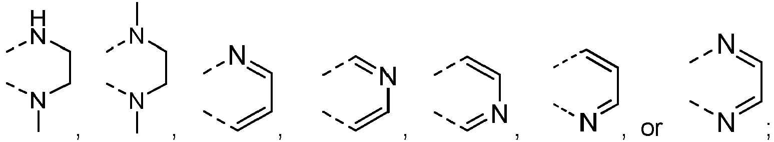

More preferred, the composition comprises as an active ingredient, an effective amount of the compound of any one of formulae (**I**), **(IIa)** - (**IIg**), wherein
**R₁** - **R₅** represent independently of each other -H, -F, -Br, -CI, -I, -OH, -CF₃, -CH₃, -CH₂CH₃, -CH(CH₃)₂, -OCH₃, -OCH₂CH₃, -OCH(CH₃)₂, -NH₂, -NH(CH₃), -N(CH₃)₂, -NO₂, -SCH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃ -NHC(=O)NHCH₂CH₃, -NHC(=O)NHCH(CH₃)₂, -NHC(=O)NHCH₂CH₂OCH₃, -NHC(=O)NHPh, -NHC(=O)NH(4-F-Ph), -NHC(=O)NH(4-MeO-Ph), -NHC(=O)CH(NH₂)CH₂Ph, -NHC(=O)CH(NH₂)CH₃, -NHC(=O)CH(NH₂)CH₂COOH, -NHC(=O)CH(NH₂)CH₂OH, -NHC(=O)CH(NH₂)CH(CH₃)₂, -NHC(=O)CH(NH₂)CH₂CH₂CONH₂, -NHC(=O)CH(NH₂)CH₂CH₂CH₂CH₂NH₂, -SCH₂CH₃, -NHSO₂CH₃, -NHSO₂Ph, -NHSO₂(4-Cl-Ph), -NHSO₂(4-MeO-Ph), 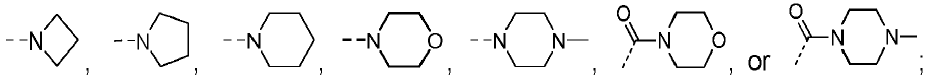
wherein at least one of **R₁ - R₅** is different from -H; or
**R₁** and **R₂** or **R₂** and **R₃** form together the moiety 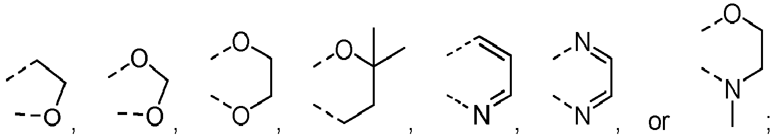
**R₄** and **R₅** forms together the moiety 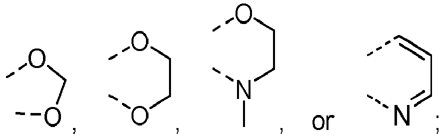 and
**R^{N}** is independently selected from hydrogen, C₁₋₄ alkyl, -C₂₋₄ alkylene-OH, -C₂₋₄ alkenyl, -CH₂-O**R^{E1}**; -CH₂CH₂-O**R^{E1}**; preferably, **R^{N}** represents -H, -CH₃, -CH₂CH₃, or -CH(CH₃)₂, even more preferably **R^{N}** represents -H.

Most preferred, the composition comprises as an active ingredient, an effective amount of a compound selected from the group consisting of compounds 1-144, in particular, compounds 3, 14, 15, 30, 45, 46, 47, 48, 49, 52, 53, 54, 55, 56, 57, 58, 59, 60, 61, 63, 65,66, 75, 76, 77, 78, 79, 81, 82, 83, 84 , 87, 88, 89, 90, 91, 92, 93 ,94, 95, 96, 98, 99, 100, 101, 102, 103, 105, 106, 108, 109, 110, 114, 115, 118, 119, 120, 121, 122, 123, 124, 125, 126, 127, 128, 129, 130, 131, 132, 133, 134, 135, 136, 137, 138, 139, 140, 141, and 144.

The expression "effective and non-phytotoxic amount" means an amount of composition according to the invention that is sufficient to control or destroy the fungi present or liable to appear on the cropsand that does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials that are within the capabilities of a person skilled in the art.

According to the invention, the term " an agriculturally acceptable support" denotes a natural or synthetic, organic or inorganic compound with that the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilizers, water, alcohols, in particular butanol, organic solvents, mineral and plant oils and derivatives thereof. Mixtures of such supports can also be used.

The carrier is inert and includes for examples, talc, lime, quartz, pumice, soybean flour, alumina trihydrate, lignin, diatomaceous earth, calcium carbonate, silica, wheat flour, and tripoli.

The filler includes for examples, urea, melamine, dicyandiamide, melamine cyanurate, amino phosphates, aminopolyphosphates, aminoplasts, phenoplasts, powdered synthetic resins, sawdust, carbohydrates, ammonium sulfate, ammonium phosphate, amino phosphates, potassium phosphate, amino sulfates, silica, diatomaceous earth, alkali metal silicates, alkaline earth metal silicates, metals, metal silicates, oxides, alumina, various clays, diatomaceous earth, carbonates, sulphates, phosphates and borates, potassium hydrogen phosphate and other like inert materials, wollastonite, mica, flint powder, kryolite, alumina trihydrate, talc, sand, pyrophyllite, blanc fixe, granulated polyethylene, zinc oxide, and mixtures thereof.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulfonic acid salts, phenolsulfonic or naphthalenesulfonic acid salts, polycondensates of ethylene oxide with fatty alcohols or with fatty acids or with fatty amines, substituted phenols (in particular alkylphenols or arylphenols), salts of sulfosuccinic acid esters, taurine derivatives (in particular alkyl taurates), phosphoric esters of polyoxyethylated alcohols or phenols, fatty acid esters of polyolsand derivatives of the above compounds containing sulfate, sulfonate and phosphate functions. The presence of at least one surfactant is generally essential when the active compound and/or the inert support are water-insoluble and when the vector agent for the application is water. Preferably, surfactant content can be comprised from 5% to 40% by weight of the composition. Optionally, additional components can also be included, e.g. protective colloids, adhesives, thickeners, thixotropic agents, penetration agents, stabilisers, sequestering agents. More generally, the active compounds can be combined with any solid or liquid additive that complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure), gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

The composition according to the invention can further comprise one or more insecticide, fungicide, bactericide, attractant, acaricide or pheromone active substance or other compounds with biological activity. The mixtures thus obtained have normally a broadened spectrum of activity.

Thus, one embodiment of the invention is directed to according to the invention further comprising at least one fungicide compounds.

Examples of suitable fungicide can be selected in the following lists:
(1) Inhibitors of the ergosterol biosynthesis, for example aldimorph (1704-28-5), azaconazole (60207-31-0), bitertanol (55179-31 -2), bromuconazole (116255-48-2), cyproconazole (113096-99-4), diclobutrazole (75736-33-3), difenoconazole (119446-68-3), diniconazole (83657-24-3), diniconazole-M (83657-18-5), dodemorph (1593-77-7), dodemorph acetate (31717-87-0), epoxiconazole (106325-08-0), etaconazole (60207-93-4), fenarimol (60168-88-9), fenbuconazole (114369-43-6), fenhexamid (126833-17-8), fenpropidin (67306-00-7), fenpropimorph (67306-03-0), fluquinconazole (136426-54-5), flurprimidol (56425-91 -3), flusilazole (85509-19-9), flutriafol (76674-21-0), furconazole (112839-33-5), furconazole-cis (112839-32-4), hexaconazole (79983-71-4), imazalil (60534-80-7), imazalil sulfate (58594-72-2), imibenconazole (86598-92-7), ipconazole (125225-28-7), metconazole (1251 16-23-6), myclobutanil (88671 -89-0), naftifine (65472-88-0), nuarimol (63284-71-9), oxpoconazole (174212-12-5), paclobutrazol (76738-62-0), pefurazoate (101903-30-4), penconazole (66246-88-6), piperalin (3478-94-2), prochloraz (67747-09-5), propiconazole (60207-90-1), prothioconazole (178928-70-6), pyributicarb (88678-67-5), pyrifenox (88283-41-4), quinconazole (103970-75-8), simeconazole (149508-90-7), spiroxamine (118134-30-8), tebuconazole (107534-96-3), terbinafine (91161-71-6), tetraconazole (112281-77-3), triad imefon (43121-43-3), triadimenol (89482-17-7), tridemorph (81412-43-3), triflumizole (68694-11-1), triforine (26644-46-2), triticonazole (131983-72-7), uniconazole (83657-22-1), uniconazole-p (83657-17-4), viniconazole (77174-66-4), voriconazole (137234-62-9), 1-(4-chlorophenyl)-2-(1H-1,2,4-triazol-1-yl)cycloheptanol (129586-32- 9), methyl 1-(2,2-dimethyl-2,3-dihydro-1 H-inden-1 - yl)-1 H-imidazole-5-carboxylate (110323-95-0), N'-{5-(difluoromethyl)-2-methyl-4-[3-(trimethylsilyl)propoxy]phenyl}-N-ethyl-N-methylimidoformamide, N-ethyl-N-methyl-N'-{2-methyl-5-(trifluoromethyl)-4-[3-(trimethylsilyl)propoxy]phenyl} imidoformamide and 0-[1-(4-methoxyphenoxy)-3,3-dimethylbutan-2-yl] 1H-imidazole-1 -carbothioate (111226-71-2).
(2) Inhibitors of the respiratory chain at complex I or II, for example bixafen (581809-46-3), boscalid (188425-85-6), carboxin (5234-68-4), diflumetorim (130339-07-0), fenfuram (24691-80-3), fluopyram (658066-35-4), flutolanil (66332-96-5), fluxapyroxad (907204-31-3), furametpyr (123572-88-3), furmecyclox (60568-05-0), isopyrazam (mixture of syn- epimeric racemate 1 RS,4SR,9RS and anti-epimeric racemate 1 RS,4SR,9SR) (881685-58-1), isopyrazam (anti-epimeric racemate 1 RS,4SR,9SR), isopyrazam (anti-epimeric enantiomer 1 R,4S,9S), isopyrazam (anti-epimeric enantiomer 1 S,4R,9R), isopyrazam (syn epimeric racemate 1 RS,4SR,9RS), isopyrazam (syn-epimeric enantiomer 1 R,4S,9R), isopyrazam (syn-epimeric enantiomer 1 S,4R,9S), mepronil (55814-41-0), oxycarboxin (5259-88-1), penflufen (494793-67-8), penthiopyrad (183675-82-3), sedaxane (874967-67-6), thifluzamide (130000-40-7), 1-methyl-N-[2-(1,1,2,2-tetrafluoroethoxy)phenyl]-3-(trifluoromethyl)-1H-pyrazole-4-carboxamide, 3-(difluoromethyl)-1-methyl-N-[2-(1,1,2,2- tetrafluoroethoxy)phenyl]-1 H-pyrazole-4-carboxamide, 3-(difluoromethyl)-N-[4-fluoro-2-(1,1,2,3,3,3- hexafluoropropoxy) phenyl]-1-methyl-1H-pyrazole-4-carboxamide, N-[1-(2,4-dichlorophenyl)-1 - methoxypropan-2-yl]-3-(difluoromethyl)-1 -methyl-1 H-pyrazole-4-carboxamide (1092400-95-7), 1-methyl-3-(trifluoromethyl)-N-[2'-(trifluoromethyl)biphenyl-2-yl]-1H-pyrazole-4-carboxamide, N-(4'-chlorobiphenyl-2-yl)-3-(difluoromethyl)-1-methyl-1H-pyrazole-4-carboxamide, N-(2',4'-dichlorobiphenyl-2-yl)-3-(difluoromethyl)-1-methyl-1H-pyrazole-4-carboxamide, 3-(difluoromethyl)-1-methyl-N-[4'-(trifluoromethyl)biphenyl-2-yl]-1H-pyrazole-4-carboxamide, N-(2',5'-difluorobiphenyl-2-yl)-1-methyl-3-(trifluoromethyl)-1H-pyrazole-4-carboxamide, 3-(difluoromethyl)-1-methyl-N-[4'-(prop-1-yn-1-yl)biphenyl-2-yl]-1H-pyrazole-4-carboxamide, 5-fluoro-1,3-dimethyl-N-[4'-(prop-1-yn-1-yl)biphenyl-2-yl]-1H-pyrazole-4-carboxamide, 2-chloro-N-[4'-(prop-1 -yn-1 -yl)biphenyl-2-yl]pyridine-3-carboxamide, 3-(difluoromethyl)-N-[4'-(3,3-dimethylbut-1 -yn-1 -yl)biphenyl-2-yl]-1-methyl-1H-pyrazole-4-carboxamide, N-[4'-(3,3-dimethylbut-1-yn-1-yl)biphenyl-2-yl]-5-fluoro-1,3-dimethyl-1H-pyrazole-4-carboxamide, 3-(difluoromethyl)-N-(4'-ethynylbiphenyl-2-yl)-1-methyl-1 H-pyrazole-4-carboxamide, N-(4'-ethynylbiphenyl-2-yl)-5-fluoro-1,3-dimethyl-1H-pyrazole-4-carboxamide, 2-chloro-N-(4'-ethynylbiphenyl-2-yl)pyridine-3-carboxamide, 2-chloro-N-[4'-(3,3-dimethylbut-1-yn-1-yl)biphenyl-2-yl]pyridine-3-carboxamide, 4-(difluoromethyl)-2-methyl-N-[4'-(trifluoromethyl)biphenyl-2-yl]-1,3-thiazole-5-carboxamide, 5-fluoro-N-[4'-(3-hydroxy-3-methylbut-1-yn-1-yl)biphenyl-2-yl]-1,3-dimethyl-1H-pyrazole-4-carboxamide, 2-chloro-N-[4'-(3-hydroxy-3-methylbut-1-yn-1-yl)biphenyl-2-yl]pyridine-3-carboxamide, 3-(difluoromethyl)-N-[4'-(3-methoxy-3-methylbut-1-yn-1-yl)biphenyl-2-yl]-1-methyl-1 H-pyrazole-4-carboxamide, 5-fluoro-N-[4'-(3-methoxy-3-methylbut-1-yn-1-yl)biphenyl-2-yl]-1,3-dimethyl-1H-pyrazole-4-carboxamide, 2-chloro-N-[4'-(3-methoxy-3-methylbut-1-yn-1-yl)biphenyl-2-yl]pyridine-3-carboxamide and salts thereof
(3) Inhibitors of the respiratory chain at complex III, for example ametoctradin (865318-97-4), amisulbrom (348635-87-0), azoxystrobin (131860-33-8), cyazofamid (120116-88-3), dimoxystrobin (141600-52-4), enestroburin (238410-11-2), famoxadone (131807-57-3), fenamidone (161326-34-7), fluoxastrobin (361377-29-9), kresoxim-methyl (143390-89-0), metominostrobin (133408-50-1), orysastrobin (189892- 69-1), picoxystrobin (117428-22-5), pyraclostrobin (175013-18-0), pyrametostrobin (915410-70-7), pyraoxystrobin (862588-11-2), pyribencarb (799247-52-2), trifloxystrobin (141517-21-7), (2E)-2-(2-{[6-(3-chloro-2-methylphenoxy)-5-fluoropyrimidin-4-yl]oxy}phenyl)-2-(methoxyimino)-N-methylethanamide, (2E)-2-(methoxyimino)-N-methyl-2-(2-{[({(1E)-1-[3-(trifluoromethyl)phenyl]ethylidene}amino)oxy]methyl}phenyl)ethanamide and salts thereof., (2E)-2-(methoxyimino)-N-methyl-2-{2-[(E)-({1-[3-(trifluoromethyl)phenyl]ethoxy}imino)methyl]phenyl}ethanamide (158169-73-4), (2E)-2-{2-[({[(1E)-1-(3-{[(E)-1 -fluoro-2-phenylethenyl]oxy}phenyl)ethylidene] amino}oxy)methyl]phenyl}-2-(methoxyimino)-N- methylethanamide (326896-28-0), (2E)-2-{2-[({[(2E,3E)-4-(2,6-dichlorophenyl)but-3-en-2-ylidene]amino}oxy)methyl] phenyl}-2-(methoxyimino)-N-methylethanamide, 2-chloro-N-(1,1,3-trimethyl-2,3-dihydro-1 H-inden-4-yl)pyridine-3-carboxamide (119899-14-8), 5-methoxy-2-methyl-4-(2-{[({(1 E)-1-[3-(trifluoromethyl)phenyl]ethylidene}amino)oxy]methyl} phenyl)-2,4-dihydro-3H-1,2,4-triazol-3-one, methyl(2E)-2-{2-[({cyclopropyl[(4-methoxyphenyl)imino]methyl}sulfanyl)methyl]phenyl}-3-methoxyprop-2-enoate (149601-03-6), N-(3-ethyl-3,5,5-trimethylcyclohexyl)-3-(formylamino)-2-hydroxybenzamide (226551-21-9), 2-{2-[(2,5-dimethylphenoxy)methyl]phenyl}-2-methoxy-N-methylacetamide (173662-97-0), (2R)-2-{2-[(2,5-dimethylphenoxy)methyl]phenyl}-2-methoxy-N-methylacetamide (394657-24-0) and salts thereof.
(4) Inhibitors of the mitosis and cell division, for example benomyl (17804-35-2), carbendazim (10605-21-7), chlorfenazole (3574-96-7), diethofencarb (87130-20-9), ethaboxam (162650-77-3), fluopicolide (2391 10-15-7), fuberidazole (3878-19-1), pencycuron (66063- 05-6), thiabendazole (148-79-8), thiophanate-methyl (23564-05-8), thiophanate (23564-06-9), zoxamide (156052-68-5), 5-chloro-7-(4-methylpiperidin-1-yl)-6-(2,4,6-trifluorophenyl)[1,2,4]triazolo[1,5-a]pyrimidine (214706-53-3), 3-chloro-5-(6-chloropyridin-3-yl)-6-methyl-4-(2,4,6-trifluorophenyl) pyridazine (1002756-87-7) and salts thereof.
(5) Compounds capable to have a multisite action, like for example bordeaux mixture (801 1 -63-0), captafol (2425-06-1), captan (133-06-2), chlorothalonil (1897-45-6), copper hydroxide (20427-59-2), copper naphthenate (1338-02-9), copper oxide (1317-39-1), copper oxychloride (1332-40-7), copper(2+) sulfate (7758-98-7), dichlofluanid (1085-98- 9), dithianon (3347-22-6), dodine (2439-10-3), dodine free base, ferbam (14484-64-1), fluorofolpet (719-96-0), folpet (133-07-3), guazatine (108173-90-6), guazatine acetate, iminoctadine (13516-27-3), iminoctadine albesilate (169202-06-6), iminoctadine triacetate (57520-17-9), mancopper (53988-93-5), mancozeb (8018-01-7), maneb (12427-38-2), metiram (9006-42-2), metiram zinc (9006-42-2), oxine-copper (10380-28-6), propamidine (104-32-5), propineb (12071 -83-9), sulfur and sulfur preparations including calcium polysulfide (7704-34-9), thiram (137-26-8), tolylfluanid (731 -27-1), zineb (12122-67-7), ziram (137-30-4) and salts thereof.
(6) Compounds capable to induce a host defence, like for example acibenzolar-S-methyl (135158- 54-2), isotianil (224049-04-1), probenazole (27605-76-1), tiadinil (223580-51 -6) and salts thereof.
(7) Inhibitors of the amino acid and/or protein biosynthesis, for example , andoprim (23951 -85-1), blasticidin-S (2079-00-7), cyprodinil (121552-61 -2), kasugamycin (6980-18-3), kasugamycin hydrochloride hydrate (19408-46-9), mepanipyrim (1 10235-47-7), pyrimethanil (531 12-28-0) and salts thereof.
(8) Inhibitors of the ATP production, for example fentin acetate (900-95-8), fentin chloride (639- 58-7), fentin hydroxide (76-87-9) and silthiofam (175217-20-6).
(9) Inhibitors of the cell wall synthesis, for example benthiavalicarb (177406-68-7), dimethomorph (110488-70-5), flumorph (21 1867-47-9), iprovalicarb (140923-17-7), mandipropamid (374726-62-2), polyoxins (11113-80-7), polyoxorim (22976-86-9), (9.8) validamycin A (37248-47-8) and valifenalate (283159-94-4; 283159-90-0).
(10) Inhibitors of the lipid and membrane synthesis, for example biphenyl (92-52-4), chloroneb (2675-77-6), dicloran (99-30-9), edifenphos (17109-49-8), etridiazole (2593-15-9), iodocarb (55406-53-6), iprobenfos (26087-47-8), isoprothiolane (50512-35-1), propamocarb (25606-41-1), propamocarb hydrochloride (25606-41-1), prothiocarb (19622-08-3), pyrazophos (13457-18-6), quintozene (82-68-8), tecnazene (117-18-0) and tolclofos-methyl (57018-04-9).
(11) Inhibitors of the melanine biosynthesis, for example carpropamid (104030-54-8), diclocymet (139920-32-4), fenoxanil (115852-48-7), phthalide (27355-22-2), pyroquilon (57369-32-1) and tricyclazole (41814-78-2).
(12) Inhibitors of the nucleic acid synthesis, for example benalaxyl (71626-11 -4), benalaxyl-M (kiralaxyl) (98243-83-5), bupirimate (41483-43-6), clozylacon (67932-85-8), dimethirimol (5221-53-4), ethirimol (23947-60-6), furalaxyl (57646-30-7), hymexazol (10004-44-1), metalaxyl (57837-19-1), metalaxyl-M (mefenoxam) (70630-17-0), ofurace (58810-48-3), oxadixyl (77732-09-3) and oxolinic acid (14698-29-4).
(13) Inhibitors of the signal transduction, for example chlozolinate (84332-86-5), fenpiclonil (74738-17-3), fludioxonil (131341-86-1), iprodione (36734-19-7), procymidone (32809-16-8), quinoxyfen (124495-18-7) and vinclozolin (50471-44-8).
(14) Compounds capable to act as an uncoupler, like for example binapacryl (485-31-4), dinocap (131-72-6), ferimzone (89269-64-7), fluazinam (79622-59-6) and meptyldinocap (131 -72-6).
(15) Further compounds, like for example benthiazole (21564-17-0), bethoxazin (163269-30-5), capsimycin (70694-08-5), carvone (99-49-0), chinomethionat (2439-01-2), chlazafenone (688046-61-9), cufraneb (11096-18-7), cyflufenamid (180409-60-3), cymoxanil (57966-95-7), cyprosulfamide (221667-31-8), dazomet (533-74-4), debacarb (62732-91-6), dichlorophen (97-23-4), diclomezine (62865-36-5), difenzoquat (49866-87-7), difenzoquat methylsulfate (43222-48-6), diphenylamine (122-39-4), ecomate, fenpyrazamine (473798-59-3), flumetover (154025-04-4), fluoroimide (41205-21-4), flusulfamide (106917-52-6), flutianil (304900-25-2), fosetyl-aluminium (39148-24-8), fosetyl-calcium, fosetyl-sodium (39148-16-8), hexachlorobenzene (118-74-1), irumamycin (81604-73-1), methasulfocarb (66952-49-6), methyl isothiocyanate (556-61-6), metrafenone (220899-03-6), mildiomycin (67527-71-3), natamycin (7681-93-8), nickel dimethyldithiocarbamate (15521-65-0), nitrothal-isopropyl (10552-74-6), octhilinone (26530-20-1), oxamocarb (917242- 12-7), oxyfenthiin (34407-87-9), pentachlorophenol and salts (87-86-5), phenothrin, phosphorous acid and its salts (13598-36-2), propamocarb-fosetylate, propanosine-sodium (88498-02-6), proquinazid (189278-12-4), pyrrolnitrine (1018-71-9), tebufloquin (376645-78-2), tecloftalam (76280-91-6), tolnifanide (304911 -98-6), triazoxide (72459-58-6), trichlamide (70193-21-4), zarilamid (84527-51-5), 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-79-6), 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003319-80-9), 1-(4-{4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone (1003318-67-9), 1-(4-methoxyphenoxy)-3,3-dimethylbutan-2-yl 1H-imidazole-1-carboxylate (111227-17-9), 2,3,5,6-tetrachloro-4-(methylsulfonyl)pyridine (13108-52-6), 2,3-dibutyl-6-chlorothieno[2,3-d]pyrimidin-4(3H)-one (221451-58-7), 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone (1003316-53-7), 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone (1003316-54-8), 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone (1003316-51-5), 2-butoxy-6-iodo-3-propyl-4H-chromen-4-one, 2-chloro-5-[2-chloro-1-(2,6-difluoro-4-methoxyphenyl)-4-methyl-1H-imidazol-5-yl]pyridine, 2-phenylphenol and salts (90-43-7), 3,4,5-trichloropyridine-2,6-dicarbonitrile (17824-85-0), 3-[5-(4-chlorophenyl)-2,3-dimethyl-1 ,2-oxazolidin-3-yl]pyridine, 3-chloro-5-(4-chlorophenyl)-4-(2,6-difluorophenyl)-6-methylpyridazine, 4-(4-chlorophenyl)-5-(2,6- difluorophenyl)-3,6-dimethylpyridazine, 5-amino-1 ,3,4-thiadiazole-2-thiol, 5-chloro-N'- phenyl-N'-(prop-2-yn-1-yl)thiophene-2-sulfonohydrazide (134-31-6), 5-methyl-6-octyl[1 ,2,4]triazolo[1 ,5-a]pyrimidin-7-amine, ethyl (2Z)-3-amino-2-cyano-3-phenylprop-2-enoate, N-(4-chlorobenzyl)-3-[3-methoxy-4-(prop-2-yn-1-yloxy)phenyl]propanamide, N-[(4- chlorophenyl)(cyano)methyl]-3-[3-methoxy-4-(prop-2-yn-1 -yloxy)phenyl]propanamide, N-[(5- bromo-3-chloropyridin-2-yl)methyl]-2,4-dichloropyridine-3-carboxamide, N-[1 -(5-bromo-3- chloropyridin-2-yl)ethyl]-2,4-dichloropyridine-3-carboxamide, N-[1 -(5-bromo-3-chloropyridin-2-yl)ethyl]-2-fluoro-4-iodopyridine-3-carboxamide, N-{(E)-[(cyclopropylmethoxy) imino][6-(difluoromethoxy)-2,3-difluorophenyl]methyl}-2-phenylacetamide (221201 -92-9), N-{(Z)-[(cyclopropylmethoxy)imino][6-(difluoromethoxy)-2,3-difluorophenyl] methyl}-2-phenylacetamide (221201-92-9), N-methyl-2-(1-{[5-methyl-3-(trifluoromethyl)-1H-pyrazol-1-yl]acetyl}piperidin-4-yl)-N-(1,2,3,4-tetrahydro naphthalen-1-yl)-1,3-thiazole-4-carboxamide (922514-49-6), N-methyl-2-(1-{[5-methyl-3-(trifluoromethyl)-1 H-pyrazol-1-yl]acetyl}piperidin-4-yl)-N-[(1 R)-1,2,3,4-tetrahydronaphthalen-1-yl]-1,3-thiazole-4-carboxamide (922514-07-6), N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide (922514-48-5), pentyl {6-[({[(1-methyl-1 H-tetrazol-5-yl)(phenyl)methylidene]amino}oxy)methyl] pyridin-2-yl}carbamate, phenazine-1-carboxylic acid, quinolin-8-ol (134-31-6) and quinolin-8-ol sulfate (2:1) (134-31-6), (5-bromo-2-methoxy-4-methylpyridin-3-yl)(2,3,4-trimethoxy-6-methylphenyl)methanone and N-[2-(4-{[3-(4-chlorophenyl)prop-2-yn-1-yl]oxy}-3-methoxyphenyl)ethyl]-N2-(methylsulfonyl) valinamide (220706-93-4).

In one embodiment, the composition according to the invention further comprises at least one bactericide.

Examples of suitable bactericides can be selected in the following list: bronopol, dichlorophen, nitrapyrin, nickel dimethyldithiocarbamate, kasugamycin, octhilinone, furancarboxylic acid, oxytetracycline, probenazole, streptomycin, tecloftalam, copper sulfate and other copper preparations.

The composition according to the invention can be used for treatment or protection of plant diseases caused fungi, oomycetes or bacteria, wherein the plant disease caused by fungi, oomycetes or bacteria is selected from the group consisting of: Blumeria diseases, Podosphaera diseases, Sphaerotheca diseases, Uncinula diseases, Gymnosporangium diseases, Hemileia diseases, Phakopsora diseases, Puccinia diseases, Uromyces diseases, Albugo diseases, Bremia diseases, Peronospora diseases, Phytophthora diseases, Plasmopara disease, Pseudoperonospora diseases, Pythium diseases, Altemaria disease, Cercospor diseases, Cladiosporum diseases, Cochliobolus diseases, Colletotrichum diseases, Cycloconium disease, Diaporthe disease, Elsinoe diseases, Gloeosporium diseases, Glomerella diseases, Guignardia, Leptosophaeria diseases, Magnaporthe diseases, Mycosphaerella diseases, Phaeosphaeria diseases, Pyrenophora diseases, Ramularia diseases, Rhynchosporium diseases, Septoria diseases, Typhula diseases, Venturia diseases, Corticium diseases, Fusarium diseases, Gaeumannomyces diseases, Rhizoctonia diseases, Sarocladium diseases, Sclerotium diseases, Tapesia diseases, Thielaviopsis diseases, Alternaria diseases, Aspergillus diseases, Cladosporium diseases, Claviceps diseases, Fusarium diseases, Gibberella diseases, Monographella diseases, Sphacelotheca diseases, Tilletia diseases, Urocystis diseases, Ustilago diseases, Aspergillus diseases, Botrytis diseases, Penicillium diseases, Rhizopus diseases, Sclerotinia diseases, Verticilium diseases, Alternaria diseases, Aphanomyces diseases, Ascochyta diseases, Aspergillus diseases, Cladosporium diseases, Cochliobolus diseases, Colletotrichum diseases, Fusarium diseases, Gibberella diseases, Macrophomina diseases, Monographella diseases, Penicillium diseases, Phoma diseases, Phomopsis diseases, Phytophthora diseases, Pyrenophora diseases, Pyricularia diseases, Pythium diseases, Rhizoctonia diseases, Rhizopus diseases, Sclerotium diseases, Septoria diseases, Typhula diseases, Verticillium diseases, Nectria diseases, Monilinia diseases, Exobasidium diseases, Taphrina diseases, Esca diseases, Eutypa dyeback, Ganoderma diseases, Rigidoporus diseases, Botrytis diseases, Rhizoctonia diseases, Helminthosporium diseases, Hymenoscyphus diseases, and Plasmodiophora diseases.

Preferably, the plant disease caused by fungi, oomycetes or bacteria is selected from the group consisting of:
Blumeria diseases caused by Blumeria graminis, Podosphaera diseases caused by Podosphaera leucotricha, Sphaerotheca diseases caused by Sphaerotheca fuliginea, Uncinula diseases caused by Uncinula necator, Gymnosporangium diseases caused by Gymnosporangium sabinae, Hemileia diseases caused by Hemileia vastatrix, Phakopsora diseases caused by Phakopsora pachyrhizi or Phakopsora meibomiae, Puccinia diseases caused by Puccinia recondite, Puccinia graminis or Puccinia striiformis, Uromyces diseases caused by Uromyces appendiculatus, U. pisi, U. fabae or U. striatus, Albugo diseases caused by Albugo candida, Bremia diseases caused by Bremia lactucae, Peronospora diseases caused by Peronospora pisi or P. brassicae, Phytophthora diseases caused by Phytophthora infestans, P. capsica, P. cinnamomi, P. nicotianae, P. palmivora, P. fragariae or P. sojae, Plasmopara disease caused by Plasmopara viticola, Pseudoperonospora diseases caused by Pseudoperonospora humuli or Pseudoperonospora cubensis, Pythium diseases caused by Pythium ultimum, Altemaria disease caused by Alternaria solani, Cercospor diseases caused by Cercospora beticola, Cladiosporum diseases caused by Cladiosporium cucumerinum, Cochliobolus diseases caused by Cochliobolus sativus (Conidiaform Drechslera, Syn: Helminthosporium) or Cochliobolus miyabeanus, Colletotrichum diseases caused by Colletotrichum lindemuthanium, Cycloconium disease caused by by Cycloconium oleaginum, Diaporthe disease caused by Diaporthe citri, Elsinoe diseases caused by Elsinoe fawcettii, Gloeosporium diseases caused by Gloeosporium laeticolor, Glomerella diseases caused by Glomerella cingulata, Guignardia caused by Guignardia bidwelli, Leptosophaeria diseases caused by Leptosphaeria maculans or Leptosphaeria maculans, Magnaporthe diseases caused by Magnaporthe grisea, Mycosphaerella diseases caused by Mycosphaerella graminicola, Mycosphaerella arachidicola or Mycosphaerella fijiensis, Phaeosphaeria diseases caused by Phaeosphaeria nodorum, Pyrenophora diseases caused by Pyrenophora teres, or Pyrenophora tritici repentis, Ramularia diseases caused by Ramularia collo-cygni , or Ramularia areola, Rhynchosporium diseases caused by Rhynchosporium secalis, Septoria diseases caused by Septoria apii or Septoria lycopercisi, Typhula diseases caused by Typhula incarnata, Venturia diseases caused by Venturia inaequalis, Corticium diseases caused by Corticium graminearum, Fusarium diseases caused by Fusarium oxysporum, Gaeumannomyces diseases caused by Gaeumannomyces graminis, Rhizoctonia diseases caused by Rhizoctonia solani, Sarocladium diseases caused by Sarocladium oryzae, Sclerotium diseases caused by Sclerotium oryzae, Tapesia diseases caused by Tapesia acuformis, Thielaviopsis diseases caused by Thielaviopsis basicola, Alternaria diseases caused by Alternaria spp., Aspergillus diseases caused by Aspergillus flavus, Cladosporium diseases caused by Cladosporium spp., Claviceps diseases caused by Claviceps purpurea, Fusarium diseases caused by Fusarium culmorum, Gibberella diseases caused by Gibberella zeae, Monographella diseases caused by Monographella nivalis, Sphacelotheca diseases caused by Sphacelotheca reiliana, Tilletia diseases caused by Tilletia caries, Urocystis diseases caused by Urocystis occulta, Ustilago diseases caused by Ustilago nuda, U. maydis or U. hordei, Aspergillus diseases caused by Aspergillus flavus, Botrytis diseases caused by Botrytis cinerea, Penicillium diseases caused by Penicillium expansum, Rhizopus diseases caused by Rhizopus stolonifer, Sclerotinia diseases caused by Sclerotinia sclerotiorum, Verticilium diseases caused by Verticilium alboatrum, Alternaria diseases caused by Alternaria brassicicola, Aphanomyces diseases caused by Aphanomyces euteiches, Ascochyta diseases caused by Ascochyta lentis Aspergillus diseases caused by Aspergillus flavus, Cladosporium diseases caused by Cladosporium herbarum, Cochliobolus diseases caused by Cochliobolus sativus, Colletotrichum diseases caused by Colletotrichum coccodes, Fusarium diseases caused by Fusarium culmorum, Gibberella diseases caused by Gibberella zeae, Macrophomina diseases caused by Macrophomina phaseolina, Monographella diseases caused by Monographella nivalis, Penicillium diseases caused by Penicillium expansum, Phoma diseases caused by Phoma lingam, Phomopsis diseases caused by Phomopsis sojae, Phytophthora diseases caused by Phytophthora cactorum, Pyrenophora diseases caused by Pyrenophora graminea, Pyricularia diseases caused by Pyricularia oryzae, Pythium diseases caused by Pythium ultimum, Rhizoctonia diseases caused by Rhizoctonia solani, Rhizopus diseases caused by Rhizopus oryzae, Sclerotium diseases caused by Sclerotium rolfsii, Septoria diseases caused by Septoria nodorum, Typhula diseases caused by Typhula incarnata, Verticillium diseases caused by Verticillium dahliae, Nectria diseases caused by Nectria galligena, Monilinia diseases caused by Monilinia laxa, Exobasidium diseases caused by Exobasidium vexans, Taphrina diseases caused by Taphrina deformans, Esca diseases caused by Phaemoniella clamydospora, Eutypa dyeback caused by Eutypa lata, Ganoderma diseases caused by Ganoderma boninense, Rigidoporus diseases caused by Rigidoporus lignosus, Botrytis diseases caused by Botrytis cinerea, Rhizoctonia diseases caused by Rhizoctonia solani, Helminthosporium diseases caused by Helminthosporium solani, Ash dieback caused by Hymenoscyphus sp and Plasmodiophora diseases caused by Plasmodiophora brassicae.

Preferalby, the composition according to the invention can be used for treatment or protection of plant disease caused by oomycetes, wherein the plant disease caused by oomycetes is Albugo disease caused by Albugo candida or Albugo laibachii, Bremia disease caused by Bremia lactucae, Peronospora disease caused by Peronospora pisi or P. brassicae, Phytophthora disease caused by Phytophthora infestans, P. capsica, P. cinnamomi, P. nicotianae, P. palmivora, P. fragariae or P. sojae, Plasmopara disease caused by Plasmopara viticola, and Pseudoperonospora disease caused by Pseudoperonospora humuli or Pseudoperonospora cubensis.

A method for treatment or protection of a plant disease caused fungi, oomycetes or bacteria, characterized in that an agronomically effective and non-phytotoxic quantity of a compound of the formula (I) or a composition according to the invention is applied to the soil where plants grow or are capable of growing, to the leaves and/or the fruit of plants or to the seeds of plants.

The dose of active compound usually applied in the method of treatment according to the invention is generally and advantageously from 10 to 800 g/ha, preferably from 10 to 300 g/ha, more preferably, 20 to 100 g/ha, most preferably 20-50 g/ha for applications in foliar treatment. The dose of active substance applied is generally and advantageously from 2 to 200 g per 100 kg of seed, preferably from 3 to 150 g per 100 kg of seed in the case of seed treatment.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain almost identical or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### General methods

### 1. Plant growth conditions

*A. thaliana* seeds were stratified on moist soil (Einheitserde type A240, Stender, Germany) for seven days at 4 °C in darkness, before transfer into growth chambers with short day conditions (10 h light, 14 h darkness, 23/20 °C, 60 % humidity). After three weeks, seedlings were singularised and grown for three weeks further. After six weeks, *A. thaliana* plants were used for experiments and after infection they were transferred into SANYO (Panasonic MLR-352, Japan) growth cabinets (10 h light, 14 h darkness, 22/16 °C). Potato plants were grown from tubers by covering them with soil (Einheitserde type A240, Stender, Germany). The leaves of adult plants (~4-6 weeks old) were cut off and kept in a moist environment for infection assays.

### 2. Testing of putative amyloid inhibitor compounds on potato leaves against P. infestans

*Phytophthora infestans* was cultivated on Rye B agar (Caten and Jinks, Spontaneous variability of single isolates of Phytophthora infestans. I. Cultural variation, Can. J. Bot. 1968, 46, 329-348) at 18 °C in the dark. Spore solutions were made by washing them off the plate with ice-cold water and incubation at 4 °C, 1 h. To induce hedging of the washed-off spores, the solution was incubated on a light table for 30-40 min. The test compounds were dissolved in DMSO (10 mM stocks) and diluted to 100 µM in *P. infestans* spore solution (1 % DMSO as mock control). The mixture of test compound and spore solution was drop-inoculated on the left side of the middle vain of detached potato leaves (3 × 10 µl), while the mock control was drop-inoculated on the right side of the same leaf. The inoculated leaves were kept under moist conditions (12 h light, 12 h darkness, 18/16 °C) for five days before the infection area was measured using UV illumination and ImageJ (Rueden et al., BMC Bioinformatics, 2017, 18(1), 529) analyses software.

### 3. Albugo sp. infections of A. thaliana

For regular *Albugo* sp. infections *A. thaliana* Ws-0 was sprayed with an *Albugo* sp. conidiospores solution. These spore solutions were prepared by harvesting sporulating leaf material at 14 days post infection (dpi) and adding water for rehydration of the spores. Spore solutions were hold on ice for ~1 h, before filtering through miracloth (Merck Millipore, Germany) to remove dirt. Spores were counted and the solutions diluted to 22.2 × 10⁴ conidiospores/ml. *Albugo* sp. spore solutions were sprayed uniformly on *A. thaliana* leaves (4.5 ml per six plants) using an airbrush gun. During spraying, plants were placed in a plastic bag and afterwards transferred into a cold room to incubate at 4 °C in darkness (~ 16 h). The next morning, plants were transferred into plant growth cabinets, but left in clear bags to keep humidity high, which supports *Albugo* sp. infections. ~24 h later plants were unbagged and further incubated under normal plant growth conditions.

### 4. Testing of putative amyloid inhibitor compounds on A. thaliana leaves against Albugo sp.

For experiment 2 and 3, the test compounds were dissolved in DMSO (10 mM stocks) and diluted to 100 µM in sterile ddH₂O (1 % DMSO as mock control). The inhibitors were carefully infiltrated in ten *A. thaliana* Ws-0 leaves without causing any wounding using a needleless syringe until the leaves were completely water-soaked. Afterwards, the plants were kept in the lab for ~1 h to let the leaves equilibrate again. The infiltrated plants were infected with *Albugo* sp., following the above-mentioned protocol. At 7 dpi, infiltrated leaves were phenotypically scored and harvested. The samples were frozen in liquid nitrogen and stored at -80 °C until DNA extraction for growth quantification via qPCR. For experiment 4, *A. candida* spore solutions were prepared as before and mixed with 10 mM stock solution of the test compound (100 µM end conc.). The mixture of spore solution and test compound was sprayed and incubated as for experiment 2 and 3.

### 5. DNA extraction and Albugo growth quantification via quantitative PCR (qPCR)

Infected plants were harvested at 7 dpi *(A. laibachii*/*A. candida* infections) and were frozen in liquid nitrogen. Three adult plants (or five seedlings) were pooled and ground to powder using liquid nitrogen cooled mortar and pestle and DNA was extracted following a Phenol/Chloroform- extraction protocol (McKinney et al., Sequence-based identification of T-DNA insertion mutations in Arabidopsis: actin mutants act2-1 and act4-1, The Plant Journal, 1995, 8, 613-622). In short, ground powder was taken up in extraction buffer (50 mM Tris pH 8.0, 200 mM NaCl, 0.2 mM EDTA, 0.5 % SDS, 0.1 mg/ml proteinase K (Sigma-Aldrich, USA) and incubated at 37 °C for 30 min. 1 volume phenol was added, centrifuged and the top layer was mixed with 1 volume chloroform/isoamylalcohol (24:1). After centrifugation, the top layer was mixed with 3 M sodium acetate and two volumes pure ethanol to precipitate the nucleic acids. DNA was pelleted by centrifugation and washed twice with 70 % ethanol. DNA was resuspended in nuclease free water (NFW) and used for qPCR. DNA concentrations were determined via NanoDrop (Thermo Scientific, USA) and diluted to 1 ng/µl. One qPCR reaction contained 7.5 µl SsoAdvanced universal SYBR Green supermix (Bio-Rad, USA), 0.3 µl of each primer (10 mM), 1.9 µl NFW and 5 µl DNA. Samples were measured in triplicates in a CFX Connect real-time PCR detection system (Bio-Rad, USA) using the following program: (1) 95 °C, 2 min; (2) [95 °C, 20 sec, then 59 °C, 20 sec, then 72 °C, 30 sec] x40, 72 °C, 5 min followed by a temperature gradient from 65 °C to 95 °C. To quantify the amount of oomycete DNA per plant sample, two standard genes were used: *A. thaliana* EF1-α (forward: AAGGAGGCTGCTGAGATGAA - SEQ ID NO: 1, reverse: TGGTGGTCTCGAACTTCCAG- SEQ ID NO: 2) and oomycete ITS 5.8s (forward: ACTTTCAGCAGTGGATGTCTA- SEQ ID NO: 3, reverse: GATGACTCACTGAATTCTGCA- SEQ ID NO: 4). The amount of oomycete DNA was normalized to the respective plant DNA content and test compound-treated plants were normalized to mock-treated plants via calculating the ΔΔCq.

### 6. Testing of putative amyloid inhibitor compounds in bacterial liquid cultures

To assess the growth of different bacteria in the presence of putative amyloid inhibitor compounds bacterial overnight cultures of a *Rhodococcus* sp. and *Pseudomonas syringae* pv. *tomato* (*Pto*) DC3000 were diluted to an OD₆₀₀ of 0.05 in KB medium (20.0 g Peptone, 1.5 g K₂HPO₄, 5 ml 1 M MgSO_{4*}7H₂O, 10 ml Glycerol, H₂O at 1000 ml, pH 7.2; experiments with *Pto* DC3000 contained 100 mg/L Rifampicin). 100 µl bacterial liquid culture were mixed with 1 µl 10 mM inhibitor compound (or DMSO as mock) in clear 96-well flat bottom plates in duplicates. Bacterial growth curves were measured in a Synergy 4 plate reader (Biotek Instruments, USA) over a time course of 18.5 h, measuring OD₆₀₀ of bacterial solutions every 30 min at 22 °C under constant shaking. To determine the absolute growth rates, OD-values were averaged for duplicates and (ODₘₐₓ-ODₘᵢₙ)/(tₘₐₓ-tₘᵢₙ) was calculated. All values were normalized to the mock control to see inhibitory effects.

### Experiment 1:

Antimicrobial activity for the compounds described in this invention was determined via in vivo tests. 100 µM compounds in 1 % DMSO (end conc.) were mixed with *Phytophthora infestans* spore solutions and drop-inoculated on adult, detached potato leaves (three 10 µl drops). Test compounds were inoculated on the left side of the middle vain, while the control (1 % DMSO) was inoculated on the right side so that test compound and control were on the same leaf. The leaves were incubated for five days, before pictures were taken under UV illumination to measure the infection area relative to control. 19 test compounds showed > 50 % inhibition of *P. infestans,* while 14 of these compounds inhibited the pathogen growth even > 70 % (Table 1 and Table 7).

**Table 1 P. infestans growth inhibition after drop inoculation of spore solutions mixed with test compounds.**

| *P. infestans* | |
|---|---|
| Compounds | % inhibition |
| **1** | 74 |
| **2** | 78 |
| **5** | 89 |
| **10** | 82 |
| **12** | 76 |
| **14** | 71 |
| **15** | 56 |
| **17** | 13 |
| **19** | 26 |
| **20** | 21 |
| **21** | 70 |
| **22** | 63 |
| **23** | 76 |
| **24** | 80 |
| **25** | 79 |
| **26** | 65 |
| **27** | 62 |
| **28** | 30 |
| **30** | 46 |
| **31** | 77 |
| **32** | 77 |
| **33** | 85 |
| **36** | 95 |
| **37** | 87 |

### Experiment 2:

Fungicidal activity against other oomycetes was tested using isolates of the obligate biotrophic pathogen genus *Albugo* on *Arabidopsis thaliana.* The test compounds, 100 µM in 1 % DMSO, were syringe infiltrated into adult *A. thaliana* and subsequently sprayed with aqueous spore solutions of *Albugo laibachii* or A. *candida.* In primary screenings, disease severity was visually screened and scored at nine days post infection (dpi) (Table 2 + 3).

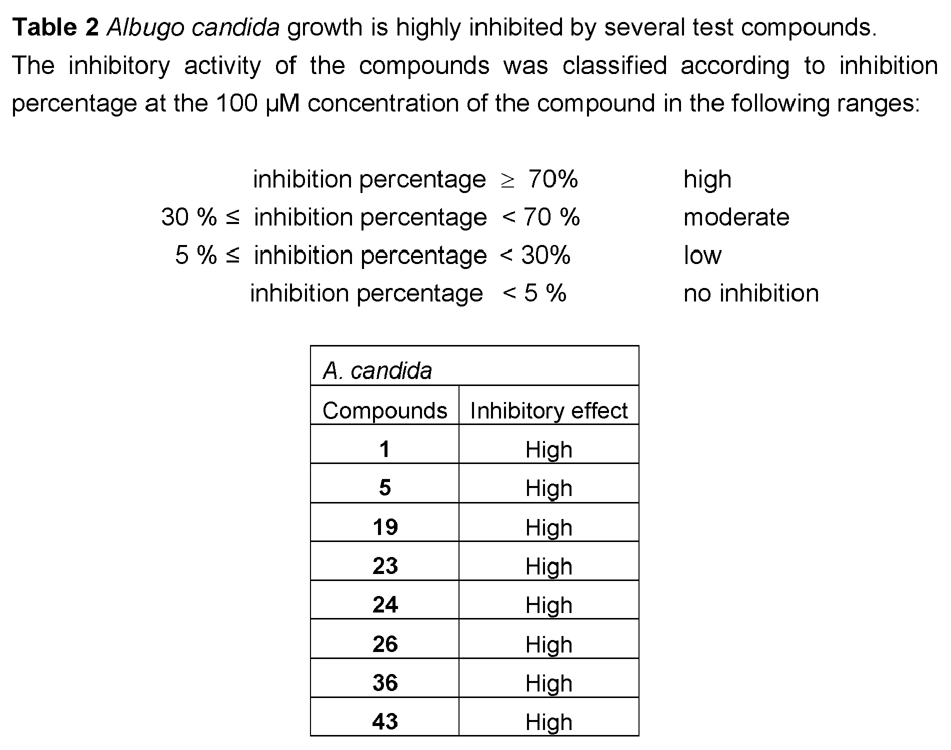

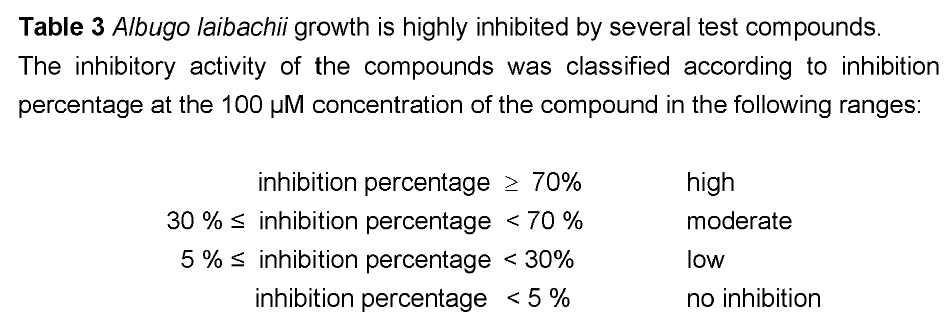

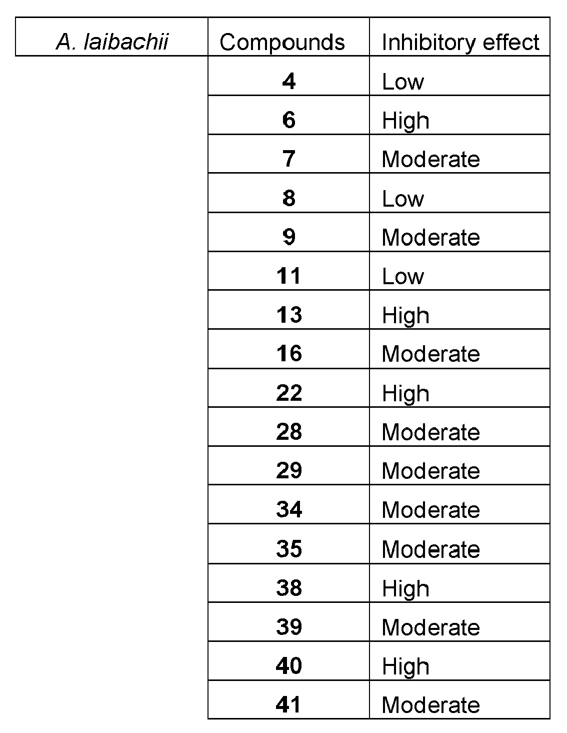

### Experiment 3:

Additional quantitative data was obtained by measuring *Albugo* sp. growth via molecular technique (qPCR) at 7 dpi. Compound 19 and 24 treated samples served as reference for the reliability of visual scoring of infection symptoms (Experiment 2). Table 4 shows the strong inhibition of *A. candida* growth by compounds 10, 19, 24, 22 and 38, while *A. laibachii* was significantly inhibited by 19, 22 and 38 (p ≤ 0.05).

**Table 4 Albugo candida and Albugo laibachii growth is significantly inhibited by several test compounds after syringe infiltration.**

| *A. candida* | |
|---|---|
| Compounds | % inhibition |
| **10** | 68 |
| **19** | 70 |
| **22** | 53 |
| **24** | 80 |
| **28** | 49 |
| **38** | 53 |

| *A. laibachii* | |
|---|---|
| Compounds | % inhibition |
| **10** | 36 |
| **19** | 59 |
| **22** | 49 |
| **24** | 16 |
| **28** | 4 |
| **38** | 50 |

### Experiment 4:

Efficacy of spray application was assessed by mixing *A. candida* spore solutions with 100 µM test compound in 1 % DMSO (control accordingly) and spraying the solution on adult *A. thaliana* plants. At 7 dpi, leaves were harvested and *Albugo* growth was quantified via qPCR. Exemplary compounds were tested that were predicted to have suitable chemical properties for spray applications. Table 5 shows strong growth inhibition of *A. candida* after mixing spore solutions with compounds 36 and 37.

**Table 5 A. candida is significantly inhibited after spray application of test compounds.**

| *A. candida* | |
|---|---|
| Compounds | % inhibition |
| **24** | 41 |
| **36** | 94 |
| **37** | 80 |

In conclusion, the presented test compounds are strong inhibitors of plant pathogen growth and can be applied in different ways. The compounds can have a high specificity and can even distinguish between two pathogen species of the same genus, while they can also have broader activity against different pathogens. As most pathogens that exhibit a biotrophic life style of variable length during host colonization possess high amounts of amyloid-like proteins, aggregation inhibitors have a great potential for the development of effective antifungal and anti-oomycete agents.

### Experiment 5: Testing of putative amyloid inhibitor compounds in bacterial liquid cultures

To assess the efficiency of the test compounds against bacterial growth, *Rhodococcus* sp. and pyhtopathogenic *Pto* DC3000 were grown in liquid culture. After addition of the test compounds (100 µM end conc.) bacterial growth curves were measured. 21 of the 50 tested compounds showed strong inhibitory effects (≥ 70 %) and eight moderate effects (30 % ≤ inhibition percentage < 70 %) against *Rhodococcus* sp. growth, while 6 compounds showed moderate inhibition of *Pto* DC3000.

**Table 6 Absolute growth rates relative to mock control (1 = mock) of Rhodococcus sp. and Pto DC3000 bacteria in the presence of test compounds. Absolute growth rates were calculated as (ODₘₐₓ-ODₘᵢₙ)/(tₘₐₓ-tₘᵢₙ).**

| Compound | *Rhodococcus* sp. | *Pto* DC3000 |
|---|---|---|
| **4** | -0.01 | 0.48 |
| **5** | 0.64 | 0.40 |
| **6** | 0.01 | 0.73 |
| **7** | 0.14 | 0.98 |
| **8** | 0.35 | 1.00 |
| **9** | 0.08 | 1.00 |
| **10** | 0.24 | 1.11 |
| **11** | 1.10 | 0.81 |
| **12** | -0.01 | 1.04 |
| **14** | 0.11 | 1.00 |
| **16** | 0.00 | 0.66 |
| **19** | 0.58 | 0.95 |
| **21** | 0.02 | 0.93 |
| **23** | 0.01 | 1.02 |
| **24** | 0.19 | 0.95 |
| **25** | 0.01 | 1.05 |
| **26** | 1.06 | 0.73 |
| **27** | 0.98 | 0.77 |
| **28** | 0.71 | 0.49 |
| **29** | 0.62 | 0.88 |
| **30** | 0.35 | 1.09 |
| **32** | 0.10 | 0.90 |
| **33** | 0.06 | 0.90 |
| **34** | -0.01 | 0.56 |
| **35** | 0.11 | 0.85 |
| **36** | 0.09 | 1.04 |
| **37** | 0.07 | 1.02 |
| **39** | -0.01 | 0.94 |
| **40** | -0.01 | 0.91 |
| **41** | 0.62 | 1.06 |
| **42** | 0.72 | 0.73 |
| **43** | 0.75 | 1.14 |
| **104** | 0.51 | 1.01 |

**Table 7. P. infestans growth is significantly inhibited after spray application of test compounds in potato.**

| ***P. infestans*** | | Compounds | % inhibition | | |
|---|---|---|---|---|---|
| Compounds | % inhibition | **75** | 31 | Compounds | % inhibition |
| **3** | 20 | **76** | 99 | **111** | 24 |
| **33** | 84 | **77** | 6 | **112** | 44 |
| **36** | 85 | **78** | 79 | **113** | 90 |
| **44** | 5 | **79** | 81 | **114** | 93 |
| **45** | 76 | **80** | 77 | **115** | 12 |
| **46** | 7 | **81** | 10 | **116** | 72 |
| **47** | 19 | **82** | 18 | **117** | 97 |
| **48** | 21 | **83** | 26 | **118** | 83 |
| **49** | 3 | **84** | 96 | **119** | 15 |
| **50** | 28 | **85** | 10 | **120** | 12 |
| **51** | 8 | **86** | 75 | **121** | 28 |
| **52** | 9 | **87** | 99 | **122** | 36 |
| **53** | 23 | **88** | 28 | **123** | 33 |
| **54** | 91 | **89** | 8 | **124** | 4 |
| **55** | 85 | **90** | 13 | **125** | 30 |
| **56** | 17 | **91** | 29 | **126** | 81 |
| **57** | 8 | **92** | 84 | **127** | 18 |
| **58** | 89 | **93** | 95 | **128** | 6 |
| **59** | 87 | **94** | 9 | **129** | 97 |
| **60** | 9 | **95** | 9 | **130** | 9 |
| **61** | 71 | **96** | 4 | **131** | 47 |
| **62** | 64 | **97** | 10 | **132** | 66 |
| **63** | 5 | **98** | 22 | **133** | 85 |
| **64** | 23 | **99** | 64 | **134** | 19 |
| **65** | 31 | **100** | 51 | **135** | 51 |
| **66** | 29 | **101** | 69 | **136** | 7 |
| **67** | 55 | **102** | 30 | **137** | 77 |
| **68** | 17 | **103** | 99 | **138** | 68 |
| **69** | 99 | **105** | 77 | **139** | 48 |
| **70** | 33 | **106** | 44 | **140** | 39 |
| **71** | 36 | **107** | 91 | **141** | 93 |
| **72** | 83 | **108** | 51 | **142** | 46 |
| **73** | 2 | **109** | 11 | **143** | 94 |
| **74** | 18 | **110** | 18 | **144** | 81 |

### Synthesis

### Chemical synthetic procedures

The following methods are presented with details as to the preparation of compounds of the invention and the illustrative examples. A compound of the invention can be prepared from known or commercially available starting materials and reagents by one skilled in the art of organic synthesis.

All starting materials and solvents were of commercial grade and were used as received unless noted otherwise.

**Thin layer chromatography (TLC)** was conducted using Macherey-Nagel precoated sheets, 0.25 mm ALUGRAM^{®} SIL G/UV254 plates, detection with UV and/or by charring with 10 wt% ethanolic phosphomolybdic acid reagent followed by heating at 200 °C.

**Flash column chromatography** was performed using Merck silica gel 60 (0.063-0.100 mm).

**Analytical high performance liquid chromatography (HPLC)** was performed by using a Waters HPLC system with a Waters 996 Photodiode Array Detector. All separations involved a mobile phase of 0.1% trifluoroacetic acid (TFA) (v/v) in water and 0.1% TFA in acetonitrile. HPLC was performed using a reversed-phase (RP) column Eurospher RP 18, 100 Å, 5 µm, 250x4.6 mm at flow rate of 1 mL·min⁻¹. Gradient A: gradient 5% CH₃CN / 95% water → 100% CH₃CN in 30 minutes. Gradient B: gradient 50% CH₃CN / 50% water → 100% CH₃CN in 30 minutes.

**Electrospray ionization mass spectrometry (ESI-MS)** and **liquid chromatography** / **mass spectrometry (LC/MS) analyses** were obtained by using a Waters Micromass ZQ 4000 mass spectrometer in conjunction with the Waters HPLC apparatus described above.

**NMR spectra** were recorded using a 400 MHz Bruker Avance spectrometer (Bruker AG, Rheinstetten, Germany) equipped with a TXI HCN z-gradient probe. All spectra were processed using TOPSPIN 3.1 (Bruker AG, Karlsruhe, Germany). ¹H NMR chemical shifts (δ) are reported in parts per million (ppm) relative to CHCl₃, DMSO-d₅ and TFA-d₁ as internal standards. Data are reported as follows: chemical shift, multiplicity (s = singlet, d = doublet, t = triplet, q = quartet, qi = quintet, dd = doublet of doublets, dt = doublet of triplets, b = broadened, m = multiplet), coupling constants (*J*, given in Hz), integration. ¹³C NMR chemical shifts (δ) are reported in parts per million (ppm) relative to CDCl₃, DMSO-d₆ and TFA-d₁ as internal standards. The following experiments were used to record the resonances of the compounds: ¹H-1D, ¹³C-1D NMR spectra and ¹³C-APT (attached proton test with a single J-evolution time of 1/145 s, spectra were processed such that quaternary and methylene groups have a positive sign and methyl and methine groups have a negative sign). To resolve overlap of resonances and recover undetectable resonances in ¹H and APT spectra, 2D-[¹³C,¹H]-HSQC (heteronuclear single quantum coherence), 2D-[¹³C,¹H]-HMBC (heteronuclear multiple bond correlation) and 2D-NOESY were recorded for some compounds.

The synthesis and characterization of compounds 1, 2, 4-13, 16-29, 31-41, 43, 50 was reported previously *(*Acta Neuropathol., 2013, 125(6), 795-813).

The synthesis and characterization of compounds 44, 67, 71, 80, 86, 97, 112 was reported previously (WO2018206778A1).

The synthesis and characterization of compound 42 was reported previously *(*ChemMedChem, 2020, 15(5), 411-415).

Compound 62 is commercially available.

### Method A1: Synthesis of 2,4-diaryl-1H-imidazoles.

### Illustrative example: 2,4-Bis(3,4-dimethoxyphenyl)-1H-imidazole, compound 30

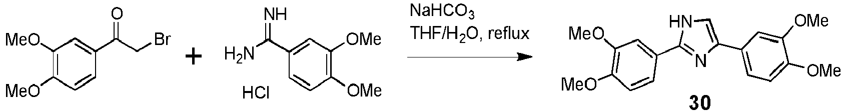

The title compound was prepared according to the published protocol (Org. Process Res. Dev., 2002, 6, 682-683) with minor modifications. A mixture of 3,4-dimethoxybenzamidine hydrochloride (320 mg, 1.5 mmol) and sodium bicarbonate (497 mg, 5.9 mmol) in THF (5 mL) and water (1.2 mL) was heated under reflux. A solution of 2-bromo-1-(3,4-dimethoxyphenyl)ethanone (381 mg, 1.5 mmol) in THF (1.5 mL) was added over a period of 30 min, while keeping the reaction under reflux. After addition, the reaction was heated under reflux for 2 h, THF was evaporated under reduced pressure. Ethyl acetate (20 mL) was added to the mixture, organic phase was separated, washed with the brine (10 mL), dried over Na₂SO₄ and evaporated under reduced pressure. The resulting crude product was purified by column chromatography on silica gel (CHCl₃/MeOH = 100/1) to provide compound 30 (360 mg, 72%) as a white solid.

### Method A2: Synthesis of 2,4-diaryl-1H-imidazoles.

### Illustrative example: 2-(4-Ethylphenyl)-4-[4-(trifluoromethoxy)phenyl]-1H-imidazole, compound 66

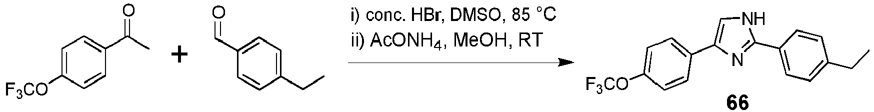

The title compound was prepared according to the published protocol (J. Org. Chem., 2019, 84, 14187-14201) and purified by column chromatography on silica gel (gradient ethyl acetate/n-hexane = 1/5 to ethyl acetate/n-hexane = 1/3) to provide the title compound 66 with the yield 21 % as a light yellow solid.

### Method A3: Synthesis of 2,4-diaryl-1H-imidazoles.

### Illustrative example: 4-{3-[4-(3,4-Dimethoxyphenyl)-1H-imidazol-2-yl]phenyl}morpholine, compound 144

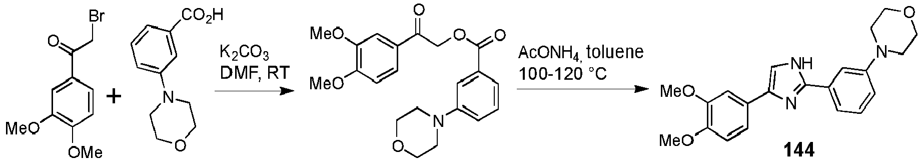

A suspension of 2-bromo-1-(3,4-dimethoxyphenyl)ethanone (311 mg, 1.5 mmol), 3-(morpholin-4-yl)benzoic acid (389 mg, 1.5 mmol) and K₂CO₃ (311 mg, 2.25 mmol) in DMF (4 mL) was stirred at room temperature for 4 hours. The mixture was poured into water (50 mL), stirred for 10 minutes and the resulting precipitate was collected by filtration, washed with water (20 mL) on the filter and dried to provide an intermediate compound 2-(3,4-dimethoxyphenyl)-2-oxoethyl 3-(morpholin-4-yl)benzoate (551 mg) which was used in the next step without further purification. A suspension of intermediate compound 2-(3,4-dimethoxyphenyl)-2-oxoethyl 3-(morpholin-4-yl)benzoate (365 mg, 1 mmol) and AcONH₄ (924 mg, 12 mmol) in toluene (7 mL) was heated at 100-120 °C with intensive stirring for 14 h. After cooling down, the mixture was partitioned between water (20 mL), saturated NH₄Cl solution (5 mL) and chloroform (30 mL). Aqueous phase was extracted with chloroform (20 mL), combined organic phases were dried over Na₂SO₄ and concentrated *in vacuo.* The residue was purified by two column chromatography on silica gel (CHCl₃/MeOH = 100/1 and acetone/n-hexane = 1/2 gradient to 1/1) to provide the title compound 144 (100 mg, 27%) as a light-red solid.

### Method B1: Synthesis of 3,5-diarylisoxazoles

### Illustrative example: 3-(2-Fluorophenyl)-5-(4-nitrophenyl)-isoxazole

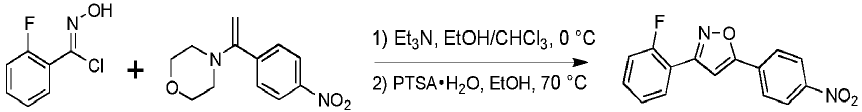

To a suspension of 4-[1-(4-nitrophenyl)ethenyl]morpholine (1.06 g, 4.5 mmol) (J. Org. Chem., 2005, 70(14), 5760-5763), Et₃N (455 mg, 4.5 mmol) in a mixture of ethanol (10 mL) and chloroform (5 mL), a solution of 2-fluoro-*N-*hydroxybenzenecarboximidoyl chloride (0.715 g, 4.1 mmol) (J. Org. Chem., 1980, 45, 3916-3918) in ethanol (10 mL) was added dropwise at 0 °C in 15 minutes with vigorous stirring and the resulting mixture was stirred at room temperature overnight. The solvents were removed under reduced pressure and the crude material was recrystallized from ethanol. The isolated intermediate (1.09 g) was treated with p-toluenesulfonic acid monohydrate (0.615 g, 3.2 mmol) in ethanol (40 mL) and the resulting mixture was stirred at 70 °C for 7 hours. After cooling down of the mixture in the ice bath, the precipitate was collected by filtration and dried on air to afford 3-(2-fluorophenyl)-5-(4-nitrophenyl)-isoxazole (775 mg, 66%) as a white solid.

¹H NMR (400 MHz, CDCl₃) *δ* = 8.37 (d, *J* = 10.0 Hz, 2H), 8.09-8.00 (m, 3H), 7.52-7.45 (m, 1H), 7.29 (td, *J* = 7.6, 1.2 Hz, 1H), 7.23 (dd, *J* = 11.0, 8.4 Hz, 1H), 7.17 (d, *J* = 3.4 Hz, 1H).

### Method B2: Synthesis of 3,5-diarylisoxazoles

### Illustrative example: 5-(3,4-Dimethylphenyl)-3-[3-(trifluoromethoxy)phenyl]-isoxazole, compound 53

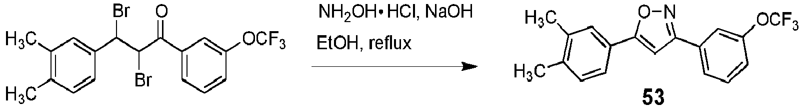

The starting material and the title compound were prepared according to the published protocol *(*Acta Neuropathol., 2013, 125(6), 795-813). A mixture of 2,3-dibromo-3-(3,4-dimethylphenyl)-1-[3-(trifluoromethoxy)phenyl]propane-1-one (960 mg, 2.0 mmol), hydroxylamine hydrochloride (620 mg, 8.9 mmol), NaOH (880 mg, 22 mmol) and water (3 mL) in ethanol (12 mL) was heated under reflux 2 h with stirring. The reaction mixture was diluted with water (10 mL) and cooled in the ice bath for 2h. The resulting precipitate was collected by filtration, washed with water (2x7 mL) and dried on air to provide the title compound 53 (310 mg, 46%) as a white solid.

### Method B3: Synthesis of 3,5-diarylisoxazoles

### Illustrative example: 4-{3-[3-(Trifluoromethyl)phenyl]-1,2-oxazol-5-yl}benzoic acid, compound 83

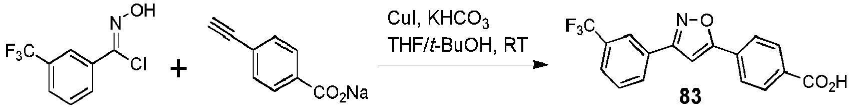

The title compound was prepared according to the published protocol *(*Tetrahedron Lett., 2009, 50(8), 905-908) with minor modifications. A mixture of sodium 4-ethynylbenzoate (0.50 g, 3.0 mmol), CuI (76 mg, 0.4 mmol) in THF (4 mL) and *t*-BuOH (12 mL) was stirred at room temperature for 10 minutes. N-hydroxy-3-(trifluromethyl)benzenecarboximidoyl chloride (0.7 g, 3.1 mmol) (J. Org. Chem., 1980, 45, 3916-3918) was added in one portion and the resulting mixture was stirred at room temperature. An additional amount of 3-(trifluromethyl)-*N-*hydroxybenzenecarboximidoyl (0.45 g, 2 mmol) was added after 6 hours and the stirring was continued for another 36 hours. The solvents were removed under reduced pressure, phosphate buffer (1M, pH 6, 50 mL) was added and the product was extracted with ethyl acetate (50 mL). The organic fraction was washed with aqueous saturated NH₄Cl solution (20 mL), dried over Na₂SO₄ and concentrated in *vacuo.* The crude product was purified by recrystallization from acetonitrile to afford the title compound **83** (555 mg, 55 %) as a white powder.

### Method C1: Synthesis of 3,5-diaryl-1,2,4-oxadiazoles

### Illustrative example: 3-(4-Fluorophenyl)-5-(3-methoxyphenyl)-1,2,4-oxadiazole, compound 51

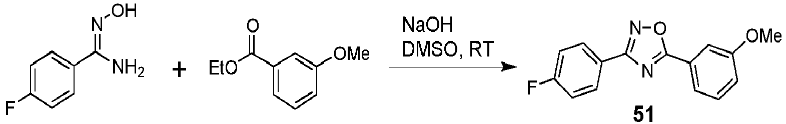

The title compound was prepared according to the published protocol *(*Tetrahedron, 2017, 73, 945-951) with minor modifications. To a solution of N-hydroxy-4-fluorobenzenecarboximidamide (206 mg, 1.3 mmol) and ethyl 3-methoxybenzoate (356 mg, 2.0 mmol) in DMSO (2 mL) powdered NaOH (80 mg, 2.0 mmol) was rapidly added. The heterogeneous mixture was stirred at room temperature for 2 h. The reaction mixture was diluted with cold water (30 mL). The resulting precipitate was collected by filtration, washed with water (2x15 mL) and dried on air to afford the title compound **51** (230 mg, 64%) as a white crystalline solid.

If the formation of the precipitate was not observed or the final product was not pure, the crude mixture was purified by column chromatography on silica gel.

### Method C2: Synthesis of 3,5-diaryl-1,2,4-oxadiazoles

### Illustrative example: 5-(4-Nitrophenyl)-3-[3-(trifluoromethyl)phenyl]-1,2,4-oxadiazole, compound 73

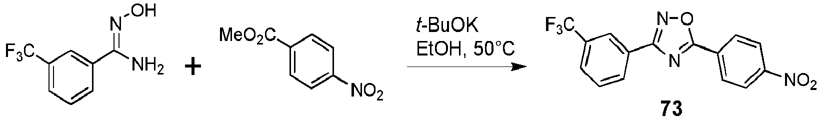

To a mixture of *N*-hydroxy-3-(trifluoromethyl)benzenecarboximidamide (3.06 g, 15 mmol) and methyl 4-nitrobenzoate (3.08 g, 17 mmol) in ethanol (50 mL) *t*-BuOK (1.72 g, 15.4 mmol) was rapidly added. The heterogeneous mixture was stirred at room temperature for 3 hours and overnight at 50 °C. After cooling down and incubation at 0 °C for 1 hour, the resulting precipitate was collected by filtration, washed with water (2x15 mL) and dried on air to afford the title compound **73** (1.6 g, 33%) as a white crystalline solid.

### Method C3: Synthesis of 3,5-diaryl-1,2,4-oxadiazoles

### Illustrative example: Ethyl 4-{5-[4-(methylsulfanyl)phenyl]-1,2,4-oxadiazol-3-yl}benzoate, compound 95

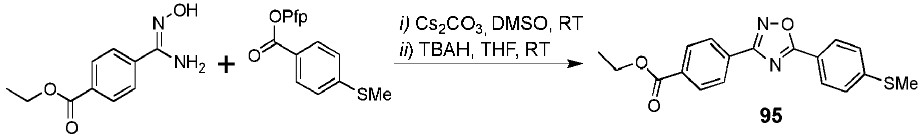

To a stirring solution of 4-(*N*-hydroxycarbamimidoyl)benzoic acid ethyl ester (208 mg, 1 mmol) and pentafluorophenyl 4-(methylthio)benzoate (668 mg, 2 mmol) (WO2018206778A1) in DMSO (4 mL) cesium carbonate (652 mg, 2 mmol) was added in one portion. After stirring for 48 hours at room temperature the reaction mixture was quenched with water (30 mL) and the resulting precipitate was collected by filtration. The crude intermediate was dissolved in THF (5 mL), treated with aqueous TBAH solution (40 %, 130 mg) and stirred at room temperature for 1 hour. The solvents were removed under reduced pressure, phosphate buffer (1M, pH 7, 20 mL) was added and the product was extracted with ethyl acetate (2x15 mL). The combined organic fractions were washed with brine (5 mL), dried over Na₂SO₄ and concentrated *in vacuo.* The crude product was purified by column chromatography on silica gel (CH₂Cl₂/*n*-hexane = 1/1) to provide compound 95 (180 mg, 53%) as a white solid.

### Method D1: Synthesis of 3,5-diaryl-1,2,4-triazoles

### Illustrative example: 3-(4-Fluorophenyl)-5-(2-methyl-5-nitrophenyl)-1H-1,2,4-triazole, compound 99

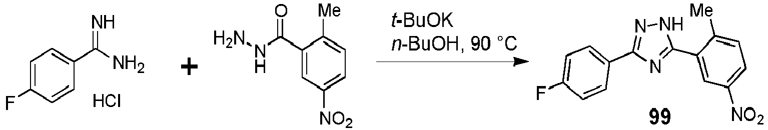

A solution of *t*-BuOK (1.12 g, 10 mmol) in n-BuOH (30 mL) was cooled in ice bath, 4-fluorobenzamidine hydrochloride (1.16 g, 6.6 mmol) was added and the resulting mixture was stirred 30 min at RT. After addition of 1-methyl-3-nitrobenzoic acid hydrazide (1.31 g, 6.7 mmol) the mixture was stirred at 90 °C for 14 hours. If LC-MS analysis showed the presence of corresponding amidrazone intermediate, the mixture was heated under reflux until disappearance of amidrazone. The reaction mixture was cooled down and concentrated *in vacuo.* The residue was dissolved in ethyl acetate (40 mL), insoluble impurities were removed by filtration and the filtrate was concentrated *in vacuo.* The crude product was purified by recrystallization from acetonitrile to provide compound **99** (1.26 g, 64%) as a white solid.

### Method E1: Synthesis of 2,4-diaryl-1,3-oxazoles

### Illustrative example: 4-(4-Ethoxyphenyl)-2-[3-(propan-2-yl)phenyl]-1,3-oxazole, compound 110

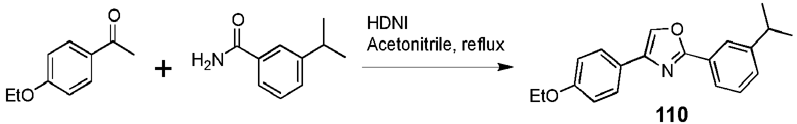

The title compound was prepared according to the published protocol *(*Synth. Commun., 2003, 33(9), 1611-1614) with minor modifications. A mixture of 1-(4-ethoxyphenyl)ethan-1-one (328 mg, 2 mmol) and [hydroxy(2,4-dinitrobenzenesulfonyloxy)iodo]benzene (1.12 g, 2.4 mmol) in acetonitrile (20 mL) was stirred under reflux for 2 hours. 3-Isopropylbenzamide (978 mg, 6 mmol) was added in one portion and the reaction mixture was stirred under reflux overnight. After cooling down, the solvent was evaporated *in vacuo,* the residue was treated with saturated aqueous NaHCO₃ solution (200 mL) and extracted with DCM (2x75 mL). The combined organic fractions were washed with water (30 mL), brine (20 mL), dried over Na₂SO₄ and concentrated *in vacuo.* The crude product was purified by column chromatography on silica gel (CH₂Cl₂/cyclohexane = 1/1) to provide compound **110** (290 mg, 47%) as a white solid.

### Method E2: Synthesis of 2,5-diaryl-1,3-oxazoles

### Illustrative example: Ethyl 4-[5-(4-methylphenyl)-1,3-oxazol-2-yl]benzoate, compound 115

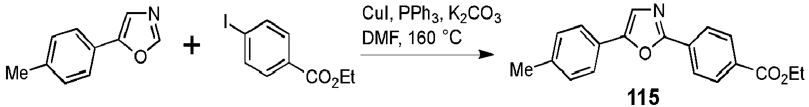

The title compound was prepared according to the published protocol *(*Tetrahedron Lett., 2009, 50(26), 3273-3276). A mixture of 5-(4-methylphenyl)-1,3-oxazole (159 mg, 1.0 mmol), which was prepared according to published protocol *(*J. Org. Chem., 2008, 73(8), 3278-3280), ethyl 4-iodobenzoate (331 mg, 1.2 mmol), CuI (190 mg, 1.0 mmol), triphenylphosphine (53 mg, 0.2 mmol) and sodium carbonate (212 mg, 2.0 mmol) in DMF (2 mL) was stirred at 160 °C for 6 hours. The reaction mixture was cooled down, quenched by addition of water/ethylenediamine (20 mL/0.4 mL) and extracted with DCM (30 mL). The organic fraction was washed with water (20 mL), brine (15 mL), dried over Na₂SO₄ and concentrated *in vacuo.* The crude product was purified by recrystallization from ethanol to afford the title compound **115** (265 mg, 86%) as a white solid.

### Method F1: Synthesis of 3,5-diaryl-1H-pyrazoles

### Illustrative example: 5-(4-Chlorophenyl)-3-(2,3-dihydro-1,4-benzodioxin-6-yl)-1H-pyrazole, compound 111

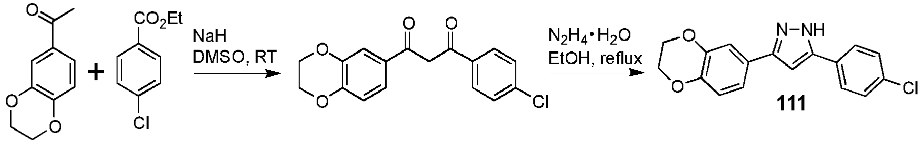

The title compound was prepared according to the published protocol *(*Acta Neuropathol., 2013, 125(6), 795-813). A 60 % suspension of sodium hydride in mineral oil (0.2 g, 5.0 mmol) was washed with petroleum benzine (20 mL) twice, anhydrous DMSO (3 mL) was added. After being stirred for 30 min at room temperature under argon, the flask was cooled down to 15 °C and a solution of methyl 4-chlorobenzoate (0.923 g, 5.0 mmol) and 1-(2,3-dihydro-1,4-benzodioxin-6-yl)ethanone (0.534 g, 3.0 mmol) in DMSO (3 mL) was added dropwise. Upon completion of addition, the reaction mixture was stirred 24 hours at room temperature, then poured slowly into crushed ice (50 g) containing 85 % phosphoric acid (1 mL). The resulting precipitate was collected by filtration, washed with water (50 ml) and air dried. Following the recrystallization from methanol, intermediate 1-(4-chlorophenyl)-3-(2,3-dihydro-1,4-benzodioxin-6-yl)propane-1,3-dione (540 mg, 1.7 mmol) was treated with hydrazine hydrate (130 mg, 2.6 mmol) in ethanol (10 mL) and reaction mixture was heated under reflux 14 hours with stirring. After cooling down to the room temperature the reaction mixture was kept at -20 °C for 1 hour. The resulting precipitate was collected by filtration, washed with water (5 mL), dried to afford the title compound 111 (470 mg, 50 % over two steps) as a white powder.

If necessary, the crude 3,5-diaryl-1*H*-pyrazoles were purified by recrystallization from ethanol or by column chromatography on silica gel.

### Method F2: Synthesis of 3,5-diaryl-1H-pyrazoles

### Illustrative example: 3-(4-ethylphenyl)-5-(3-nitrophenyl)-1H-pyrazole

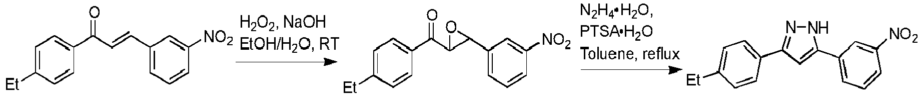

A suspension of (E)-1-(4-ethylphenyl)-3-(3-nitrophenyl)-2-propene-1-one (2.25 g, 8 mmol), which was prepared according to the published protocol (Arch. Pharm. Res., 2004, 27, 581-588), in ethanol (20 mL) was treated with aqueous NaOH solution (1 M, 1.6 mL, 1.6 mmol) followed by aqueous hydrogen peroxide solution (30%, 1.6 mL) at 0 °C and intensive stirring. After stirring at room temperature, the mixture was poured into ice cold water (200 mL) and the resulting precipitate was collected by filtration and air dried. The intermediate *trans*-1-(4-ethylphenyl)-3-(3-nitrophenyl)-2,3-epoxypropane-1-one was added to a mixture of p-toluenesulfonic acid hydrate (0.16 g, 0.8 mmol) and hydrazine hydrate (1.05 g, 21 mmol) in toluene (35 mL). The heterogeneous mixture was heated under reflux for 3 hours and concentrated *in vacuo.* The crude product was purified by recrystallization from a mixture ethanol/water (2/1) to afford 3-(4-ethylphenyl)-5-(3-nitrophenyl)-1*H-*pyrazole (1.5 g, 64%) as a white powder.

### Method F3: Synthesis of 3,5-diaryl-1H-pyrazoles

### Illustrative example: Methyl 4-[5-(4-fluorophenyl)-1H-pyrazol-3-yl]benzoate

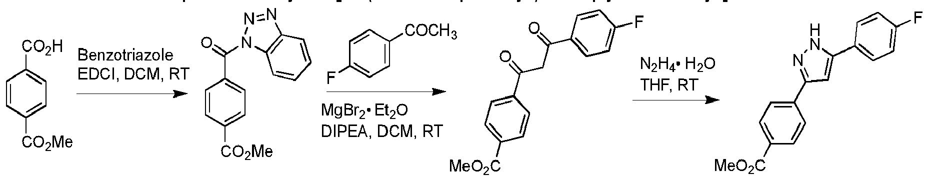

A mixture of 1-(4-fluorophenyl)ethan-1-one (345 mg, 2.5 mmol), MgBr₂· Et₂O (1.61 g, 6.25 mmol) in DCM (15 mL) was treated with DIPEA (968 mg, 7.5 mmol) and stirred at room temperature for 10 minutes. Next, the crude mixture of methyl 4-(1*H*-benzotriazol-1-ylcarbonyl)benzoate, which was prepared separately by stirring of monomethyl ester of 1,4-benzenedicarboxylic acid (540 mg, 3.0 mmol), benzotriazole (357 mg, 3 mmol) and EDCI hydrochloride (575 mg, 3.0 mmol) in dry DCM (10 mL) at 25 °C for 3 h, was added dropwise over 5 minutes. The resulting mixture was stirred at 25 °C for 12 hours, then treated with aqueous 1 M HCl (5 mL) and stirred for another 10 minutes at 25 °C. After addition of water (20 mL), the mixture was extracted with DCM (2x15 mL). Combined organic fractions were washed with brine, dried over Na₂SO₄ and concentrated *in vacuo.* The crude product was purified by recrystallization from methanol to provide intermediate methyl 4-[3-(4-flourophenyl)-1,3-dioxopropyl]benzoate (500 mg) as an beige solid which was used in the next step without further purification. This intermediate was suspended in THF (10 mL) and hydrazine monohydrate (110 mg, 2.2 mmol) was added. The reaction mixture was stirred at room temperature for 22 hours and concentrated *in vacuo.* The residue was triturated in water (10 mL), the precipitate was collected by filtration and dried on air to provide methyl 4-[5-(4-fluorophenyl)-1*H*-pyrazol-3-yl]benzoate (480 mg, 65% over two steps) as a white solid.

### Method G1: Formation of amines through the reduction of nitro groups

### Illustrative example: 5-(4-Aminophenyl)-3-(2-fluorophenyl)-isoxazole, compound 45

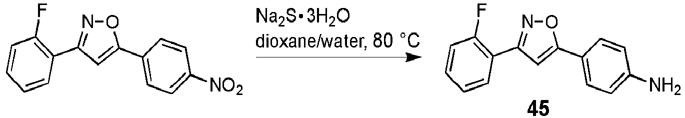

To a suspension of 3-(2-fluorophenyl)-5-(4-nitrophenyl)-isoxazole (770 mg, 2.7 mmol) in dioxane (10 mL) a warm (ca. 60 °C) solution of sodium sulfide trihydrate (1.11 g, 6.8 mmol) in water (10 mL) was added in one portion at 80 °C. The mixture was stirred for 10 h at 80 °C, cooled down to room temperature and poured into ice water (40 mL). After 30 min stirring at 0 °C the resulting precipitate filtered off, washed with cold water (3x5 mL) and air dried. The crude product was treated with ethanol (25 mL) and the resulting suspension was incubated at 50 °C for 10 minutes. After cooling down in the ice bath, insoluble material was removed by filtration and the filtrate was concentrated *in vacuo* to afford the title compound **45** (540 mg, 78%) as a light orange solid.

### Method G2: Synthesis of amides, carbamates and sulfonamides from anilines

### Illustrative example: N-{4-[3-(2-Fluorophenyl)-isoxazol-5-yl]phenyl}acetamide, compound 46

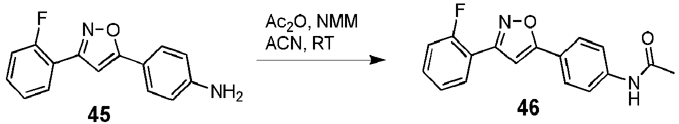

To a stirring suspension of compound 45 (152 mg, 0.6 mmol) and *N-*methylmorpholine (83 mg, 0.72 mmol) in acetonitrile (1 mL) a solution of acetic anhydride (67 mg, 0.66 mmol) in acetonitrile (10 mL) was slowly added at room temperature. After stirring for 24 h at room temperature the mixture was incubated at 0 °C for 1 h and the resulting precipitate filtered off, washed with cold water (2x3 mL) and air dried to afford the title compound 46 (142 mg, 80%) as a white solid.

If the formation of the precipitate was not observed or the final product was not pure, the crude mixture was purified by column chromatography on silica gel. In case of imidazole, pyrazoles and triazoles, the crude reaction mixture was treated with an excess of MeOH, incubated at room temperature overnight or 30 minutes under reflux, concentrated *in vacuo* and the residue was purified by column chromatography on silica gel.

### Method G3: Synthesis of amides from corresponding carboxylic acids

### Illustrative example: N-(4-Hydroxyphenyl)-4-{4-[4-(pyrrolidin-1-yl)phenyl]-1H-imidazol-2-yl}benzamide, compound 55

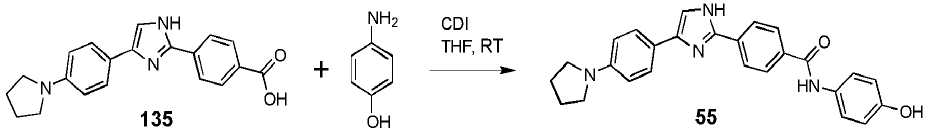

A mixture of compound 135 (96 mg, 0.3 mmol) and THF (3 mL) was treated with CDI (117 mg, 0.72 mmol) and stirred at room temperature overnight. After addition of 4-aminophenol (250 mg, 2.5 mmol) the reaction mixture was stirred for another 5 hours at room temperature. The solvents were removed under vacuum and the residue was purified by column chromatography on silica gel (gradient CHCl₃/MeOH = 100/1 to CHCl₃/MeOH = 100/5) to afford the title compound **55** (50 mg, 41%) as a dark green solid.

### Method G4: Synthesis of esters from corresponding carboxylic acids

### Illustrative example: Ethyl 3-[5-(2-fluorophenyl)-1,2,4-oxadiazol-3-yl]benzoate, compound 63

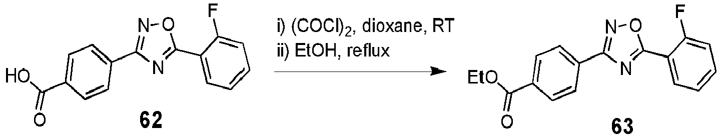

To a suspension of compound 62 (142 mg, 0.5 mmol) and DMF (3 drops) in DCM (4 mL) oxalyl chloride (127 mg, 1 mmol) was added dropwise and the resulting mixture was stirred at room temperature for 2 hours. The mixture was concentrated *in vacuo,* treated with ethanol (5 mL) and stirred under reflux for 3 hours. After removing of solvents *in vacuo,* the residue was dissolved in DCM (5 mL). The solution was filtered and concentrated *in vacuo* to afford the title compounds **63** (148 mg, 95%) as a white solid.

### Method G5: O-deprotection of phenyl alkyl ethers with BBr₃

### Illustrative example: 5-(4-Chlorophenyl)-3-(4-hydroxyphenyl)-1,2,4-oxadiazole, compound 70

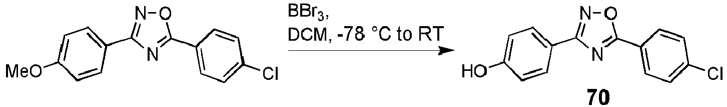

A solution of 5-(4-chlorophenyl)-3-(4-methoxyphenyl)-1,2,4-oxadiazole (200 mg, 0.7 mmol), which was prepared according to Method C1 with 81% yield, in dichloromethane (6 ml) was cooled down to -78 °C, treated with boron tribromide (0.16 ml, 1.8 mmol), stirred at -78 °C for 3 h and then overnight at room temperature. The mixture was cooled down to -78 °C and quenched with methanol (5 ml). After stirring for 3 h at room temperature solvents were evaporated under reduced pressure, the residue was co-evaporated four times with methanol (10 ml). The crude product was purified by recrystallization from MeOH to afford the title compound **70** (135 mg, 71 %) as a white powder.

### Method G6: O-deprotection of benzyl alkyl ethers

### Illustrative example: 5-(4-Hydroxyphenyl]-3-[4-(trifluoromethyl)phenyl]-1,2,4-oxadiazole, compound 72

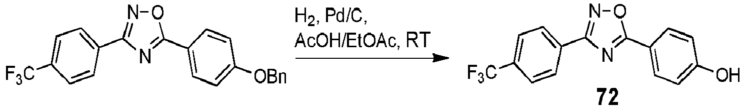

To a stirred suspension of palladium on activated carbon (10 wt %, 40 mg) in acetic acid (2 mL) under a hydrogen atmosphere a solution of 5-(4-benzyloxyphenyl]-3-[4-(trifluoromethyl)phenyl]-1,2,4-oxadiazole (386 mg, 1 mmol), which was prepared according to Method C1 with 53% yield, in ethyl acetate (20 mL) and acetic acid (3 mL) was added. After stirring for 48 hours under the hydrogen atmosphere at room temperature, the reaction mixture was filtered through Celite and concentrated under reduced pressure. A resulting precipitate was purified by column chromatography on silica gel (CH₂Cl₂/MeOH = 100/1) to afford the title compound **72** (150 mg, 49%) as a white solid.

### Method G7: O-deprotection of phenyl alkyl ethers with Me₃Sil/BCl₃

### Illustrative example: 3-{5-[2-Hydroxy-4-(trifluoromethyl)phenyl]-1,3-oxazol-2-yl}benzoic acid, compound 69

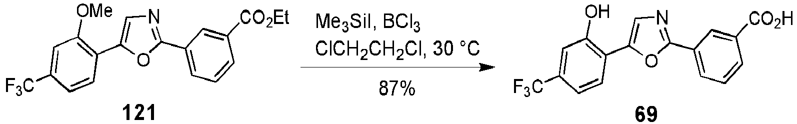

A solution of compound 121 (130 mg, 0.3 mmol) in 1,2-dichloroethane (3 ml) was treated with trimethylsilyl iodide (0.21 mL, 1.5 mmol) and a solution of boron trichloride in n-hexane (1M, 0.6 mL, 0.6 mmol), stirred at RT for 1 hour and then overnight at 30 °C. After cooling down, phosphate buffer (0.3M, pH 6, 20 mL) was added and the product was extracted with ethyl acetate (2*30 mL). The combined organic fractions were washed with brine (5 mL), dried over Na₂SO₄ and concentrated *in vacuo.* The crude residue was dissolved in aqueous NaOH solution (0.2M, 12 mL), aqueous phase washed with DCM (5 mL), filtered and acidified to pH 5-6 with aqueous HCl (1M). Aqueous solution was extracted with ethyl acetate (2*30 mL). The combined organic fractions were washed with brine (5 mL), dried over Na₂SO₄ and concentrated *in vacuo* to afford the title compound 69 (100 mg, 87 %) as a white powder.

If the final product is not carboxylic acid, the step with the dissolution in NaOH was skipped and the crude mixture was purified by column chromatography on silica gel or recrystallized from appropriate solvent.

### Method G8: synthesis of ureas from anilines

### Illustrative example: 1-Phenyl-3-(4-{3-[3-(trifluoromethyl)phenyl]-1,2,4-oxadiazol-5-yl}phenyl)urea, compound 94

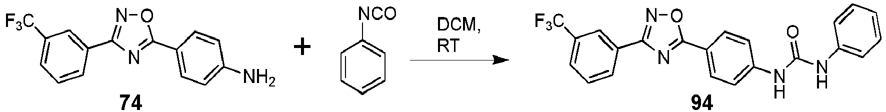

A heterogeneous mixture of compound 74 (152 mg, 0.5 mmol), phenyl isocyanate (62 mg, 0.52 mmol) and DCM (3 mL) was stirred at room temperature for 6 days. During the course of reaction 4 additional portions of phenyl isocyanate (each portion 30 mg, 0.25 mmol) were added at the end day 1, 2, 4, 5. After consumption of compound 74 on the day 6, DCM (3 mL) was added and the mixture was reflux for 3 minutes, cooled in the ice bath for 1 hour. The resulting precipitate was collected by filtration, washed with cold DCM (2x1 mL) and air dried to afford the title compound 94 (195 mg, 92%) as a white solid.

In case of imidazole, pyrazoles and triazoles, after consumption of starting material the crude reaction mixture was concentrated *in vacuo,* treated with triethylamine (1 eq.) and MeOH and incubated at 40 °C overnight. Following aqueous work-out, the crude product was purified by column chromatography on silica gel.

### Method G9: hydrolysis of esters

### Illustrative example: 4-{5-[4-(Methylsulfanyl)phenyl]-1,2,4-oxadiazol-3-yl}benzoic acid, compound 96

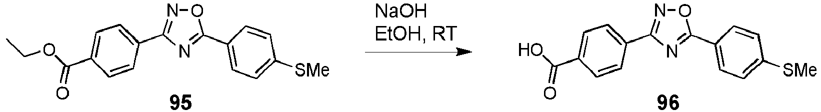

To a suspension of compound 95 (240 mg, 0.7 mmol) in ethanol (5 mL) aqueous solution of sodium hydroxide (1M, 2 mL, 2 mmol) was added and the mixture was stirred at room temperature for 48 hours. The reaction mixture was diluted with aqueous solution of sodium hydroxide (40 mM, 50 mL) and acidified with aqueous 1M HCl solution to pH 4. The resulting precipitated was collected by filtration, washed with cold water (2x3 mL) and air dried to afford the title compound **96** (195 mg, 88%) as a while solid.

### Method G10: CuI-catalyzed coupling reaction

### Illustrative example: 1-Methyl-4-{3-[4-(2,3-dihydro-1,4-benzodioxin-6-yl)-1,3-oxazol-2-yl]phenyl}piperazine, compound 114

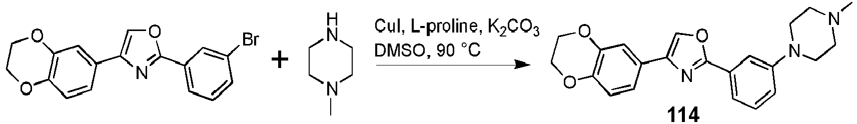

The title compound was prepared according to the published protocol (J. Org. Chem., 2005, 70(13), 5164-5173). A heterogeneous mixture of 2-(3-bromophenyl)-4-(2,3-dihydro-1,4-benzodioxin-6-yl)oxazole (210 mg, 0.6 mmol), which was prepared according to published protocol (WO2018206778A1), CuI (30 mg, 0.16 mmol), L-proline (25 mg, 0.2 mmol), *N*-methyl piperazine (228 mg, 2.3 mmol) and K₂CO₃ (210 mg, 1.5 mmol) in DMSO (1 mL) was stirred at 90 °C for 11 hours. During the course of reaction additional portion of CuI (10 mg, 0.05 mmol) and *N-*methyl piperazine (100 mg, 1.0 mmol) was added after 7 hours. The reaction mixture was quenched by addition of water (80 mL) and extracted with ethyl acetate (2*35 mL). The combined organic fractions were washed with water (20 mL), brine (15 mL), dried over Na₂SO₄ and concentrated *in vacuo.* The crude product was purified by column chromatography on silica gel (CHCl₃/MeOH = 95/5) to afford the title compound **114** (135 mg, 61%) as a light red solid.

### Method G11: Synthesis of amides from corresponding carboxylic acids

### Illustrative example: N,N-diethyl-3-{5-[2-methoxy-4-(trifluoromethyl)phenyl]-1,3-oxazol-2-yl}benzamide, compound 123

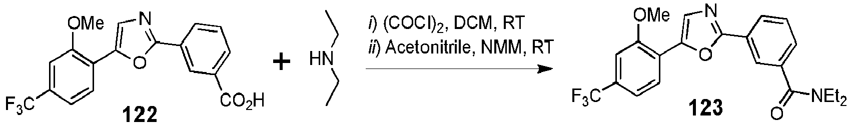

To a suspension of compound **122** (110 mg, 0.3 mmol) and DMF (2 drops) in DCM (3 mL) oxalyl chloride (76 mg, 0.6 mmol) was added dropwise and the resulting mixture was stirred at room temperature for 4 hours. The mixture was concentrated *in vacuo,* the residue was treated with a solution of *N-*methylmorpholine (101 mg, 1.0 mmol) and diethylamine (44 mg, 0.6 mmol) in acetonitrile (3 mL) and stirred overnight at room temperature. The reaction mixture was quenched by addition of water (10 mL) and extracted with ethyl acetate (2*20 mL). The combined organic fractions were washed with water (20 mL), brine (15 mL), dried over Na₂SO₄ and concentrated *in vacuo.* The crude product was purified by column chromatography on silica gel (EtOAc/n-hexane = 1/2) to afford the title compound **123** (110 mg, 88%) as a white solid.

### Method G12: Synthesis of amides

### Illustrative example: (2S)-2-Amino-N-{3-[3-(4-ethylphenyl)-1H-pyrazol-5-yl]phenyl}-3-hydroxypropanamide hydrochloride, compound 129

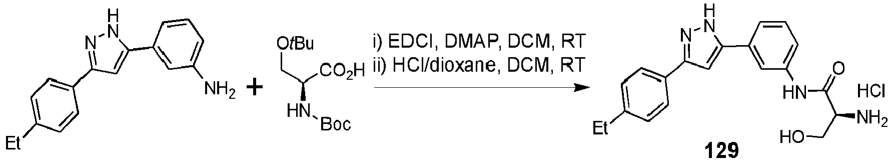

To a mixture of 5-(3-aminophenyl)-3-(4-ethylphenyl)-1*H*-pyrazole (293 mg, 1 mmol), Boc-Ser(*t*Bu)-OH DCHA (974 mg, 2.2 mmol) in DCM (10 mL) EDCI hydrochloride (888 mg, 4.6 mmol) and DMAP (20 mg) were added at room temperature. After 2 hours stirring at room temperature, the reaction mixture was concentrated to a half volume and directly purified by column chromatography on silica gel (EtOAc/n-hexane = 1/3) to afford an intermediate with the protection groups. The intermediate product was dissolved in DCM (10 mL) and treated with HCl in dioxane (4N, 2.4 mL, 9.6 mmol). After 6 days of stirring at room temperature, the reaction mixture was quenched by addition of Et₂O (10 mL). The resulting precipitated was collected by filtration, washed with Et₂O (2x8 mL) and dried. Solid material was dissolved in water (3 mL), the aqueous solution was filtered and the filtrate was lyophilized to afford the title compound 129 (280 mg, 72%) as a while solid.

Compound **131**. Deprotection conditions: 4N HCl in dioxane (25 eq.), 1,2-ethanedithiol (1.5 eq.), water (4 eq.), THF, 1 hour, RT.

Compounds **133**. Deprotection conditions. Step 1: 1-Dodecanethiol (10 eq.), DBU (0.1 eq.), THF, 10 hours, RT. Step 2: 4N HCl in dioxane (15 eq.), DCM, 4 hours, RT.

Compounds **140** and **141.** Deprotection conditions: 4N HCl in dioxane (8 eq.), DCM, 3 hours, RT.

### Table 8:

### Compound number

### Synthetic scheme, method and yield

### Analytical and spectral properties (¹H-NMR, ESI-MS, HPLC retention time)

| |
|---|
| **Compound** 3: |
| 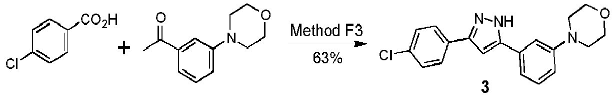 |
| ¹H NMR (400 MHz, DMSO-d₆ + 1% TFA) *δ* = 8.24 (s, 1H), 7.91-7.82 (m, 3H), 7.73 (dd, *J* = 8.1, 1.5 Hz, 1H), 7.58 (t, *J* = 8.0 Hz, 1H), 7.53 (d, *J* = 8.6 Hz, 2H), 7.37 (s, 1H), 4.08 (m, 4H), 3.61 (m, 4H). ESI-MS (m/z) [M+H]⁺ 340.2 (100%), 342.2 (35%). HPLC retention time 23.8 min, gradient A. |
| **Compound 30**: |
| 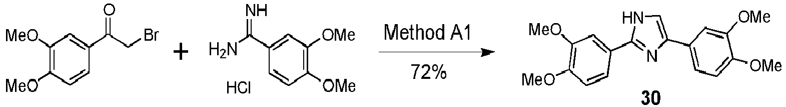 |
| ¹H NMR (400 MHz, DMSO-d6, 298K) *δ* = 12.38 (bs, 1H), 7.63-7.52 (m, 3H), 7.40 (s, 1H), 7.37 (d, *J* = 8.4 Hz, 1H), 7.04 (d, *J* = 8.4 Hz, 1H), 6.96 (d, *J* = 8.3 Hz, 1H), 3.85 (s, 3H), 3.83 (s, 3H), 3.80 (s, 3H), 3.77 (s, 3H). ESI-MS (m/z) [M+H]⁺ 341.3 (100%). HPLC retention time 16.1 min, gradient A. |
| 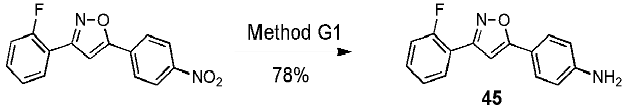 **Compound 45**: |
| ¹H NMR (400 MHz, CDCl₃, 298K) *δ* = 8.37 (d, *J* = 10.0 Hz, 2H), 8.09-8.00 (m, 3H), 7.52-7.45 (m, 1H), 7.29 (td, *J* = 7.6, 1.2 Hz, 1H), 7.23 (dd, *J* = 11.0, 8.4 Hz, 1H), 7.17 (d, *J* = 3.4 Hz, 1H). ESI-MS (m/z) [M+H]⁺ 255.2 (100%). HPLC retention time 20.2 min, gradient A. |
| **Compound 46:** |
| 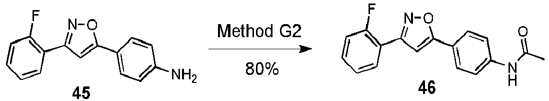 |
| ¹H NMR (400 MHz, DMSO-d₆) *δ* = 10.23 (s, 1H), 7.93 (td, *J* = 7.6, 1.8 Hz, 1H), 7.89 (d, *J* = 8.8 Hz, 2H), 7.77 (d, *J* = 8.8 Hz, 2H), 7.63-7.55 (m, 1H), 7.43 (dd, *J* = 11.2, 8.3 Hz, 1H), 7.38 (td, *J* = 7.6, 1.1 Hz, 1H), 7.31 (d, *J* = 2.7 Hz, 1H), 2.09 (s, 3H). ESI-MS (m/z) [M+H]⁺ 297.3 (100%). HPLC retention time 24.8 min, gradient A. |
| **Compound 47:** |
| 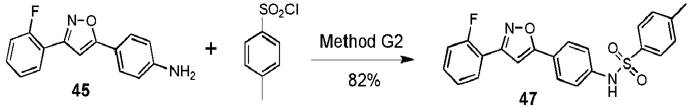 |
| ¹H NMR (400 MHz, DMSO-d₆) *δ* = 10.66 (s, 1H), 7.91 (t, *J* = 7.6 Hz, 1H), 7.82 (d, *J* = 8.6 Hz, 2H), 7.71 (d, *J* = 8.2 Hz, 2H), 7.62-7.54 (m, 1H), 7.46-7.30 (m, 4H), 7.29 (d, *J* = 2.8 Hz, 1H), 7.26 (d, *J =* 8.6 Hz, 2H), 2.33 (s, 3H). ESI-MS (m/z) [M+H]⁺ 409.3 (100%). HPLC retention time 26.3 min, gradient A. |
| **Compound 51:** |
| 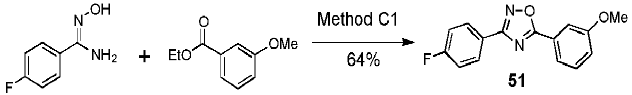 |
| ¹H NMR (400 MHz, DMSO-d₆) *δ* = 8.15-8. m, 2H), 7.76-7.71 (m, 1H), 7.62 (dd, *J* = 2.6, 1.5 Hz, 1H), 7.56 (t, *J* = 8.1 Hz, 1H), 7.41 (t, *J* = 8.9 Hz, 2H), 7.28 (ddd, *J* = 8.4, 2.6, 0.9 Hz, 1H), 3.87 (s, 3H). ESI-MS (m/z) [M+H]⁺ 271.4 (100%). HPLC retention time 29.1 min, gradient A. |
| **Compound 53:** |
| 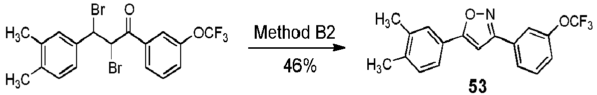 |
| ¹H NMR (400 MHz, DMSO-d₆) *δ* = 7.96 (d, *J* = 7.8 Hz, 1H), 7.86 (s, 1H), 7.73-7.66 (m, 2H), 7.65-7.59 (m, 2H), 7.53 (d, *J* = 8.3 Hz, 1H), 7.32 (d, *J* = 7.9 Hz, 1H), 2.31 (s, 3H), 2.28 (s, 3H). ESI-MS (m/z) [M+H]⁺ 334.2 (100%). HPLC retention time 24.8 min, gradient B. |
| **Compound 54:** |
| 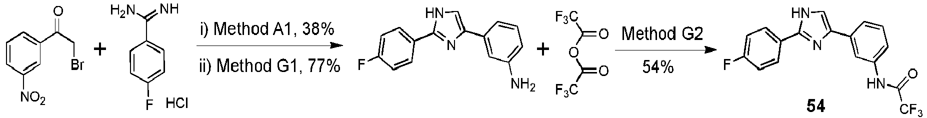 |
| ¹H NMR (400 MHz, DMSO-d₆ + 1% conc. DCI) *δ* = 11.45 (s, 1H), 8.25-8.13 (m, 3H), 7.98 (s, 1H), 7.79 (d, *J* = 7.7 Hz, 1H), 7.60 (d, *J* = 7.8 Hz, 1H), 7.49 (t, *J* = 7.8 Hz, 1H), 7.43 (t, *J* = 8.7 Hz, 2H). ESI-MS (m/z) [M+H]⁺ 350.2 (100%). HPLC retention time 17.1 min, gradient A. |
| **Compound 55:** |
| 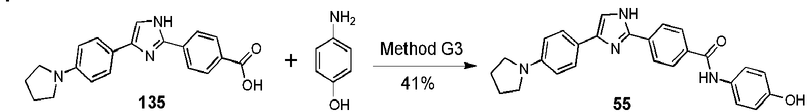 |
| ¹H NMR (400 MHz, DMSO-d₆ + 1% conc. DCI) *δ* = 10.29 (s, 1H), 8.41 (d, *J* = 8.4 Hz, 2H), 8.18 (d, *J* = 8.4 Hz, 2H), 8.07 (s, 1H), 7.84 (d, *J* = 8.6 Hz, 2H), 7.57 (d, *J* = 8.8 Hz, 2H), 6.77 (d, *J* = 8.8 Hz, 2H), 6.66 (d, *J* = 8.8 Hz, 2H), 3.28 (m, 4H), 1.96 (m, 4H). ESI-MS (m/z) [M+H]⁺ 425.4 (100%). HPLC retention time 18.7 min, gradient A. |
| **Compound 63:** |
| 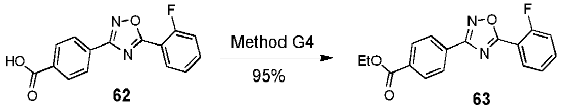 |
| ¹H NMR (400 MHz, DMSO-d₆) *δ* = 8.59 (t, *J* = 1.7 Hz, 1H), 8.33 (dt, J = 7.8, 1.4 Hz, 1H), 8.23 (td, J = 7.6, 1.7 Hz, 1H), 8.17 (dt, J = 7.8, 1.4 Hz, 1H), 7.84-7.72 (m, 2H), 7.58-7.46 (m, 2H), 4.38 (q, *J =* 7.1 Hz, 2H), 1.63 (t, *J* = 7.1 Hz, 3H). ESI-MS (m/z) [M+H]⁺ 313.3 (100%). HPLC retention time 29.1 min, gradient A. |
| **Compound 64:** |
| 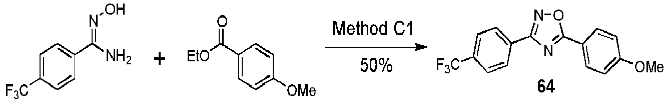 |
| ¹H NMR (400 MHz, DMSO-d₆) *δ* = 8.28 (d, *J* = 8.1 Hz, 2H), 8.14 (d, *J* = 8.9 Hz, 2H), 7.96 (d, *J* = 8.3 Hz, 2H), 7.20 (d, *J* = 8.9 Hz, 2H), 3.89 (s, 3H). ESI-MS (m/z) [M+H]⁺ 321.2 (100%). HPLC retention time 23.9 min, gradient B. |
| **Compound 65:** |
| 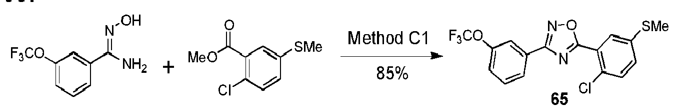 |
| ¹H NMR (400 MHz, DMSO-d₆) *δ* = 8.09 (dt, J = 7.9, 1.3 Hz, 1H), 7.95-7.90 (m, 2H), 7.73 (t, *J* = 7.9 Hz, 1H), 7.66-7.60 (m, 2H), 7.55 (dd, *J* = 8.5, 2.4 Hz, 1H), 2.56 (s, 3H). ESI-MS (m/z) [M+H]⁺ 387.3 (100%), 389.2 (45%). HPLC retention time 26.4 min, gradient B. |
| **Compound 66:** |
| 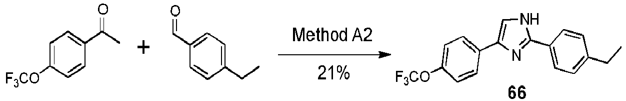 |
| ¹H NMR (400 MHz, DMSO-d₆ + 1% conc. DCI) *δ* = 8.34 (s, 1H), 8.30 (d, *J* = 8.3 Hz, 2H), 8.23 (d, *J* = 8.8 Hz, 2H), 7.54 (d, *J* = 8.2 Hz, 2H), 7.49 (d, *J* = 8.4 Hz, 2H), 2.70 (q, *J* = 7.6 Hz, 2H), 1.22 (t, *J* = 7.6 Hz, 3H). ESI-MS (m/z) [M+H]⁺ 333.2 (100%). HPLC retention time 20.5 min, gradient A. |
| **Compound 68:** |
| 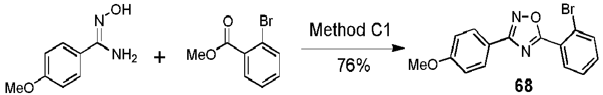 |
| ¹H NMR (400 MHz, DMSO-d₆) *δ* = 8.08 (dd, *J* = 7.1, 2.3 Hz, 1H), 8.03 (d, *J* = 8.8 Hz, 2H), 7.92 (dd, *J* = 7.4, 1.7 Hz, 1H), 7.69-7.58 (m, 2H), 7.14 (d, *J* = 8.8 Hz, 2H), 3.85 (s, 3H). ESI-MS (m/z) [M+H]⁺ 331.2 (97%), 333.2 (100%). HPLC retention time 29.1 min, gradient A. |
| **Compound 69:** |
| 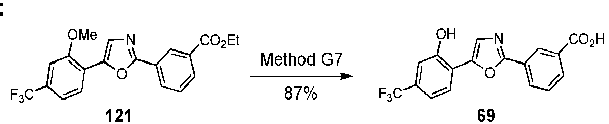 |
| ¹H NMR (400 MHz, DMSO-d₆) *δ* = 11.29 (s, 1H), 8.62 (t, *J* = 1.7 Hz, 1H), 8.36 (dt, J = 7.8, 1.4 Hz, 1H), 8.10 (dt, *J* = 7.7, 1.3 Hz, 1H), 8.06 (d, *J* = 8.1 Hz, 1H), 7.85 (s, 1H), 7.72 (t, *J* = 7.8 Hz, 1H), 7.32 (d, *J* = 8.3 Hz, 1H), 7.28 (s, 1H). ESI-MS (m/z) [M+H]⁺ 350.2 (100%). HPLC retention time 22.6 min, gradient A. |
| **Compound 70:** |
| 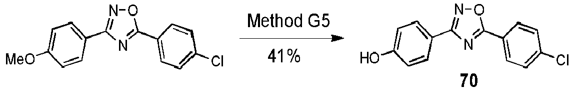 |
| ¹H NMR (400 MHz, DMSO-d₆) *δ* = 11.18 (s, 1H), 8.17 (d, *J* = 8.6 Hz, 2H), 7.92 (d, *J* = 8.7 Hz, 2H), 7.73 (d, *J =* 8.6 Hz, 2H), 6.95 (d, *J* = 8.7 Hz, 2H). ESI-MS (m/z) [M+H]⁺ 273.0 (100%), 275.2 (33%). HPLC retention time 26.0 min, gradient A. |
| **Compound 72:** |
| 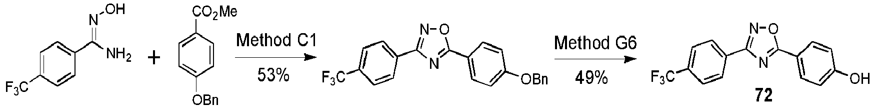 |
| ¹H NMR (400 MHz, DMSO-d₆) *δ* = 10.60, (s, 1H), 8.25 (d, *J* = 8.1 Hz, 2H), 8.02 (d, *J* = 8.7 Hz, 2H), 7.93 (d, *J* = 8.3 Hz, 2H), 7.00 (d, *J* = 8.7 Hz, 2H). ESI-MS (m/z) [M+H]⁺ 307.1 (100%). HPLC retention time 26.7 min, gradient A. |
| **Compound 73:** |
| 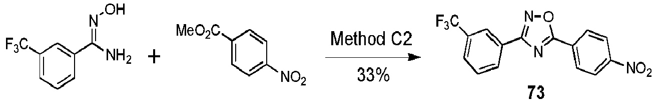 |
| ¹H NMR (400 MHz, DMSO-d₆) *δ* = 8.46 (s, 4H), 8.40 (d, *J* = 7.9 Hz, 1H), 8.32 (s, 1H), 8.03 (d, *J* = 7.9 Hz, 1H), 7.87 (t, *J* = 7.9 Hz, 1H). HPLC retention time 28.9 min, gradient A. |
| **Compound 74:** |
| 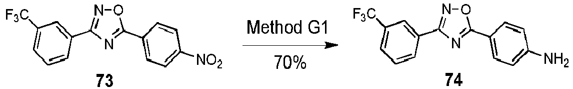 |
| ¹H NMR (400 MHz, DMSO-d₆) *δ* = 8.34 (d, *J* = 7.9 Hz, 1H), 8.28 (s, 1H), 7.98 (d, *J* = 7.9 Hz, 1H), 7.89-7.80 (m, 3H), 6.71 (d, *J* = 8.7 Hz, 2H), 6.24 (s, 2H). ESI-MS (m/z) [M+H]⁺ 306.3 (100%). HPLC retention time 26.9 min, gradient A. |
| **Compound 75:** |
| 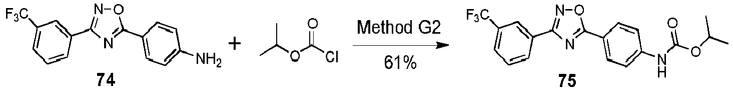 |
| ¹H NMR (400 MHz, CDCl₃) *δ* = 8.45 (s, 1H), 8.36 (d, *J* = 7.7 Hz, 1H), 8.17 (d, *J* = 8.8 Hz, 2H), 7.78 (d, *J* = 7.9 Hz, 1H), 7.64 (t, *J* = 7.9 Hz, 1H), 7.60 (d, *J* = 8.8 Hz, 2H), 6.79 (s, 1H), 5.06 (m, *J* = 6.2 Hz, 1H), 1.33 (d, *J* = 6.2 Hz, 6H). ESI-MS (m/z) [M+H]⁺ 392.3 (100%). HPLC retention time 30.3 min, gradient A. |
| **Compound 76:** |
| 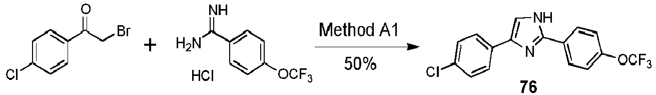 |
| ¹H NMR (400 MHz, DMSO-d₆ + 1% conc. DCI) *δ* = 8.17 (d, *J* = 8.6 Hz, 2H), 8.08 (s, 1H), 8.06 (d, *J* = 8.8 Hz, 2H), 7.64 (d, *J* = 8.6 Hz, 2H), 7.46 (d, *J* = 8.1 Hz, 2H). ESI-MS (m/z) [M+H]⁺ 339.0 (100%), 341.1 (37%). HPLC retention time 19.9 min, gradient A. |
| **Compound 78:** |
| 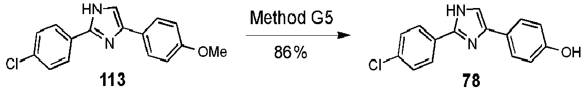 |
| ¹H NMR (400 MHz, DMSO-d₆) *δ* = 8.21 (d, *J* = 8.7 Hz, 2H), 8.12 (s, 1H), 7.78 (d, *J* = 8.6 Hz, 2H), |
| 7.74 (d, *J* = 8.7 Hz, 2H), 6.92 (d, *J* = 8.7 Hz, 2H). ESI-MS (m/z) [M+H]⁺ 271.2 (100%), 273.2 (32%). HPLC retention time 15.3 min, gradient A. |
| **Compound 79:** |
| 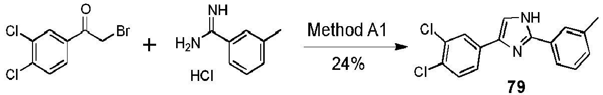 |
| ¹H NMR (400 MHz, DMSO-d₆ + 1% conc. DCI) *δ* = 8.33 (d, *J* = 2.1 Hz, 1H), 8.26 (s, 1H), 8.07 (s, 1H), 8.05-7.97 (m, 2H), 7.73 (d, *J* = 8.5 Hz, 1H), 7.47 (t, *J* = 7.7 Hz, 1H), 7.39 (d, *J* = 7.7 Hz, 1H), 2.39 (s, 3H). ESI-MS (m/z) [M+H]⁺ 303.2 (100%), 305.2 (65%). HPLC retention time 7.4 min, gradient B. |
| **Compound 81:** |
| 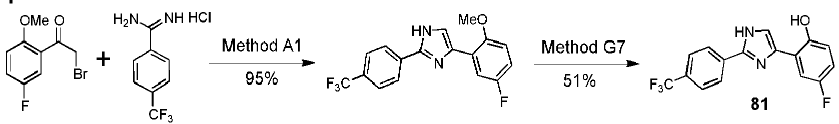 |
| ¹H NMR (400 MHz, DMSO-d₆ + 1% conc. DCI) *δ* = 8.53 (d, *J* = 8.2 Hz, 2H), 8.07 (s, 1H), 8.06-7.96 (m, 2H), 7.16-7.06 (m, 2H). ESI-MS (m/z) [M+H]⁺ 323.0 (100%). HPLC retention time 18.8 min, gradient A. |
| **Compound 83:** |
| 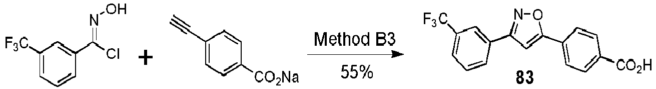 |
| ¹H NMR (400 MHz, DMSO-d₆) δ = 13.24 (bs, 1H), 8.28-8.22 (m, 2H), 8.12 (d, *J* = 8.6 Hz, 2H), 8.03 (d, *J* = 8.6 Hz, 2H), 7.95 (s, 1H), 7.92 (d, *J* = 7.9 Hz, 1H), 7.81 (t, *J* = 7.9 Hz, 1H). ESI-MS (m/z) [M+H]⁺ 334.2 (100%). HPLC retention time 26.1 min, gradient A. |
| **Compound 84:** |
| 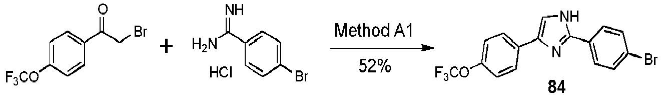 |
| ¹H NMR (400 MHz, DMSO-d₆ + 1% conc. DCI) *δ* = 8.10 (d, *J* = 8.7 Hz, 2H), 8.08-8.03 (m, 3H), 7.75 (d, *J* = 8.6 Hz, 2H), 7.43 (d, *J* = 8.0 Hz, 2H). ESI-MS (m/z) [M+H]⁺ 383.0 (100%), 385.0 (99%). HPLC retention time 20.2 min, gradient A. |
| **Compound 85:** |
| 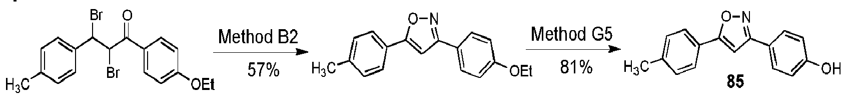 |
| ¹H NMR (400 MHz, DMSO-d₆) *δ* = 9.94 (s, 1H), 7.78 (d, *J* = 8.2 Hz, 2H), 7.74 (d, *J* = 8.6 Hz, 2H), 7.39 (s, 1H), 7.36 (d, *J* = 8.2 Hz, 2H), 6.91 (d, *J* = 8.6 Hz, 2H), 2.37 (s, 3H). ESI-MS (m/z) [M+H]⁺ 252.2 (100%). HPLC retention time 24.3 min, gradient A. |
| **Compound 87:** |
| 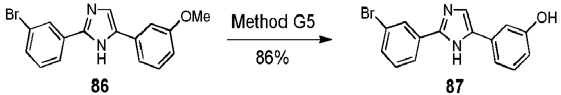 |
| ¹H NMR (400 MHz, DMSO-d₆ + 1% conc. DCI) *δ* = 9.82 (bs, 1H), 8.39 (s, 1H), 8,27 (s, 1H), 8.11 (d, *J* = 7.8 Hz, 1H), 7.86 (d, *J* = 7.9 Hz, 1H), 7.63 (t, *J* = 8.0 Hz, 1H), 7.41-7.24 (m, 3H), 6.90 (m, 1H). ESI-MS (m/z) [M+H]⁺ 315.2 (92%), 317.1 (100%). HPLC retention time 15.6 min, gradient A. |
| **Compound 88:** |
| 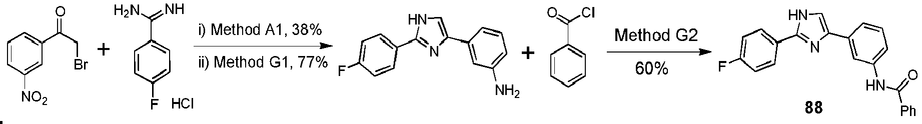 |
| ¹H NMR (400 MHz, DMSO-d₆ + 1% conc. DCI) *δ* = 10.49 (s, 1H), 8.35 (s, 1H), 8.32-8.25 (m, 2H), 8.07-8.00 (m, 3H), 7.77-7.70 (m, 2H), 7.64-7.44 (m, 6H). ESI-MS (m/z) [M+H]⁺ 358.2 (100%). HPLC retention time 5.8 min, gradient B. |
| **Compound 89:** |
| 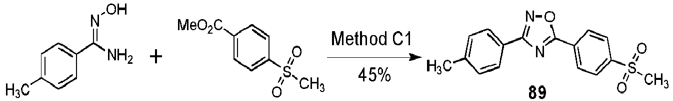 |
| ¹H NMR (400 MHz, CDCl₃) *δ* = 8.41 (d, *J* = 8.5 Hz, 2H), 8.13 (d, *J* = 8.5 Hz, 2H), 8.05 (d, *J* = 8.2 Hz, 2H), 7.32 (d, *J* = 8.0 Hz, 2H), 3.12 (s, 3H), 2.43 (s, 3H). ESI-MS (m/z) [M+H]⁺ 315.0 (100%). HPLC retention time 14.5 min, gradient B. |
| **Compound 92:** |
| 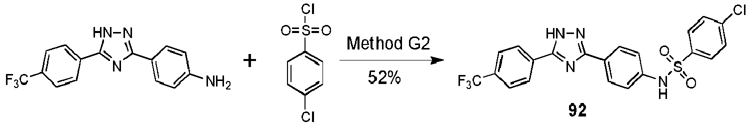 |
| ¹H NMR (400 MHz, DMSO-d₆ + 1% conc. DCI) *δ* = 10.81 (s, 1H), 8.27 (d, *J* = 8.2 Hz, 2H), 7.99 (d, *J* = 8.7 Hz, 2H), 7.85 (d, *J* = 8.3 Hz, 2H), 7.82 (d, *J* = 8.6 Hz, 2H), 7.63 (d, *J* = 8.6 Hz, 2H), 7.29 (d, *J* = 8.7 Hz, 2H). ESI-MS (m/z) [M+H]⁺ 479.2 (100%), 481.2 (39%). HPLC retention time 13.8 min, gradient B. |
| **Compound 94:** |
| 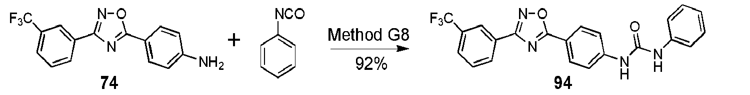 |
| ¹H NMR (400 MHz, DMSO-d₆) *δ* = 9.27 (s, 1H), 8.88 (s, 1H), 8.38 (d, *J* = 7.8 Hz, 1H), 8,32 (s, 1H), 8.14 (d, *J* = 8.8 Hz, 2H), 8.01 (d, *J* = 7.8 Hz, 1H), 7.86 (t, *J* = 7.9 Hz, 1H), 7.74 (d, *J* = 8.8 Hz, 2H), 7.48 (d, *J* = 7.6 Hz, 2H), 7.31 (t, *J* = 7.6 Hz, 2H), 7.01 (t, *J* = 7.4 Hz, 1H). ESI-MS (m/z) [M+H]⁺ 425.3 (100%). HPLC retention time 20.7 min, gradient B. |
| **Compound 95:** |
| 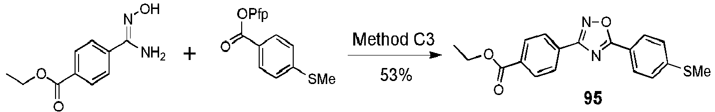 |
| NMR (400 MHz, DMSO-d₆) *δ* = 8.17 (d, *J* = 8.7 Hz, 2H), 8.11 (d, *J =* 8.7 Hz, 2H), 8.05 (d, *J* = 8.6 Hz, 2H), 7.47 (d, *J* = 8.6 Hz, 2H), 4.34 (q, *J* = 7.1 Hz, 2H), 2.55 (s, 3H), 1.34 (t, *J* = 7.1 Hz, 3H). ESI-MS (m/z) [M+H]⁺ 341.3 (100%). HPLC retention time 28.7 min, gradient B. |
| **Compound 96:** |
| 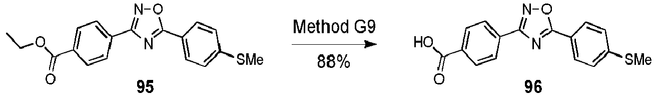 |
| ¹H NMR (400 MHz, DMSO-d₆) *δ* = 8.17 (d, *J* = 8.3 Hz, 2H), 8.12 (d, *J* = 8.3 Hz, 2H), 8.06 (d, *J* = 8.4 Hz, 2H), 7.48 (d, *J* = 8.4 Hz, 2H), 2.56 (s, 3H). ESI-MS (m/z) [M+H]⁺ 313.3 (100%). HPLC retention time 28.1 min, gradient A. |
| **Compound 98:** |
| 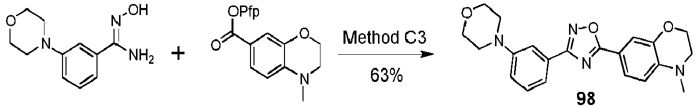 |
| ¹H NMR (400 MHz, DMSO-d₆) *δ* = 7.62 (dd, *J* = 8.5, 2.0 Hz, 1H), 7.55 (bs, 1H), 7.49 (d, *J* = 7.6 Hz, 1H), 7.41 (t, *J* = 7.8 Hz, 1H), 7.38 (d, *J =* 2.0 Hz, 1H), 7.17 (dd, *J* = 7.8, 2.0 Hz, 1H), 6.83 (d, *J =* 8.7 Hz, 1H), 4.25 (m, 2H), 3.77 (m, 4H), 3.42 (m, 2H), 3.19 (m, 4H), 2.98 (s, 3H). ESI-MS (m/z) [M+H]⁺ 378.2 (100%). HPLC retention time 28.4 min, gradient A. |
| **Compound 99:** |
| 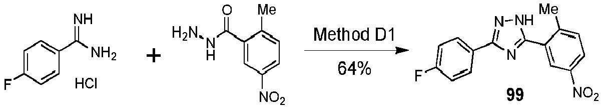 |
| ¹H NMR (400 MHz, 308K, DMSO-d₆ + 1% conc. DCI) *δ* = 8.73 (d, *J* = 2.6 Hz, 1H), 8.18-8.10 (m, 3H), 7.59 (d, *J* = 8.5 Hz, 1H), 7.36 (t, *J* = 8.9 Hz, 2H), 2.74 (s, 3H). ESI-MS (m/z) [M+H]⁺ 269.3 (100%). HPLC retention time 13.4 min, gradient A. |
| **Compound 100:** |
| 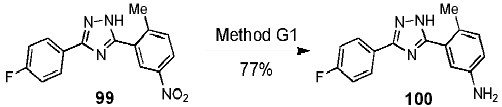 |
| ¹H NMR (400 MHz, CDCl₃) *δ* = 11.21 (bs, 1H), 8.12 (m, 2H), 7.17-7.06 (m, 4H), 6.72 (dd, *J* = 8.1, 2.5 Hz, 1H), 3.69 (bs, 2H), 2.45 (s, 3H). ESI-MS (m/z) [M+H]⁺ 269.3 (100%). HPLC retention time 13.5 min, gradient A. |
| **Compound 101:** |
| 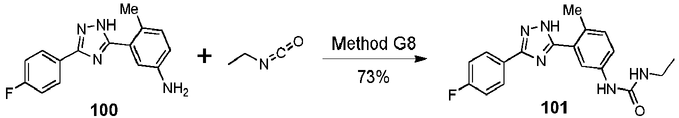 |
| ¹H NMR (400 MHz, DMSO-d₆ + 1% conc. DCI) *δ* = 8.79 (bs, 1H), 8.13 (m, 2H), 7.83 (d, *J* = 2.0 Hz, 1H), 7.43-7.33 (m, 3H), 7.20 (d, *J* = 8.3 Hz, 1H), 3.10 (q, *J* = 7.2 Hz, 2H), 2.44 (s, 3H), 1.04 (t, *J* = 7.2 Hz, 3H). ESI-MS (m/z) [M+H]⁺ 340.2 (100%). HPLC retention time 19.4 min, gradient A. |
| **Compound 102:** |
| 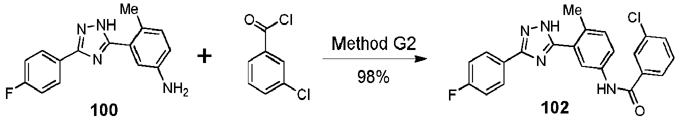 |
| ¹H NMR (400 MHz, DMSO-d₆ + 1% conc. DCI) *δ* = 10.51 (s, 1H), 8.27 (t, *J* = 2.3 Hz, 1H), 8.19-8.11 (m, 2H), 8.06 (t, *J* = 1.8 Hz, 1H), 7.96 (d, *J* = 7.8 Hz, 1H), 7.83 (dt, *J* = 8.3, 2.3 Hz, 1H), 7.67 (d, *J* = 8.7 Hz, 1H), 7.57 (t, *J* = 7.9 Hz, 1H), 7.39 (t, *J* = 8.9 Hz, 2H), 7.35 (d, *J* = 8.4 Hz, 1H), 2.55 (s, 3H). ESI-MS (m/z) [M+H]⁺ 407.3 (100%), 409.3 (38%). HPLC retention time 12.3 min, gradient B. |
| **Compound 106:** |
| 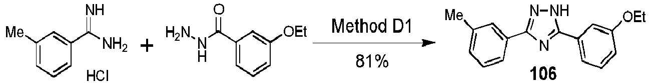 |
| ¹H NMR (400 MHz, DMSO-d₆ + 1% conc. DCI) *δ* = 7.95 (s, 1H), 7.91 (d, *J* = 7.7 Hz, 1H), 7.71-7.64 (m, 2H), 7.47-7.39 (m, 2H), 7.31 (d, *J* = 7.6 Hz, 1H), 7.05 (dd, *J* = 8.1, 2.3 Hz, 1H), 4.12 (q, *J* = 7.0 Hz, 2H), 2.40 (s, 3H), 1.36 (t, *J* = 7.0 Hz, 3H). ESI-MS (m/z) [M+H]⁺ 280.3 (100%). HPLC retention time 11.5 min, gradient B. |
| **Compound 107:** |
| 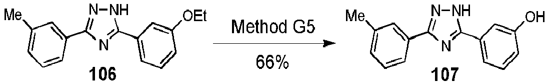 |
| ¹H NMR (400 MHz, DMSO-d₆ + 1% conc. DCI) *δ* = 7.92 (s, 1H), 7.88 (d, *J* = 7.7 Hz, 1H), 7.55-7.50 (m, 2H), 7.42 (t, *J* = 7.6 Hz, 1H), 7.35-7.28 (m, 2H), 6.89 (dd, *J* = 7.4, 2.3 Hz, 1H), 2.40 (s, 3H). ESI-MS (m/z) [M+H]⁺ 252.2 (100%). HPLC retention time 18.2 min, gradient A. |
| **Compound 108:** |
| 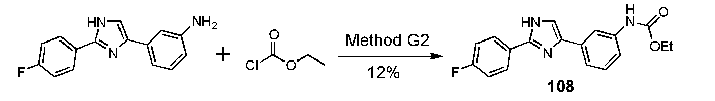 |
| ¹H NMR (400 MHz, acetone-d₆ + 1% acetic acid-d₄) *δ* = 8.66 (bs, 1H), 8.16-8.06 (m, 3H), 7.57 (s, 1H), 7.53 (d, *J* = 7.7 Hz, 1H), 7.45 (d, *J* = 7.9 Hz, 1H), 7.29 (t, *J* = 7.9 Hz, 1H), 7.23 (t, *J* = 8.9 Hz, 2H), 4.17 (q, *J* = 7.1 Hz, 2H), 1.27 (t, *J* = 7.1 Hz, 3H). ESI-MS (m/z) [M+H]⁺ 326.3 (100%). HPLC retention time 17.0 min, gradient A. |
| **Compound 110:** |
| 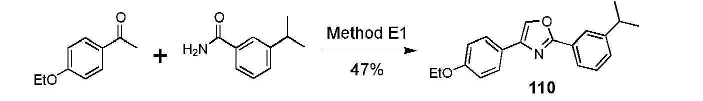 |
| ¹H NMR (400 MHz, CDCl₃) *δ* = 8.00 (t, *J* = 1.8 Hz, 1H), 7.92 (dt, *J* = 7.7, 1.4 Hz, 1H), 7.87 (s, 1H), 7.75 (d, *J* = 8.8, 2H), 7.40 (t, *J* = 7.6 Hz, 1H), 7.33 (dt, *J* = 7.7, 1.4 Hz, 1H), 6.96 (d, *J* = 8.8 Hz, 2H), 4.08 (q, *J* = 7.0 Hz, 2H), 3.01 (m, *J* = 7.0 Hz, 1H), 1.44 (t, *J* = 7.0 Hz, 3H), 1.32 (d, *J* = 7.1 Hz, 6H). ESI-MS (m/z) [M+H]⁺ 308.3 (100%). HPLC retention time 25.3 min, gradient B. |
| **Compound 111:** |
| 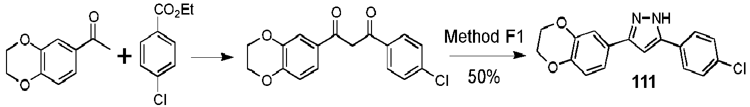 |
| ¹H NMR (400 MHz, DMSO-d₆ + 1% conc. DCI) *δ* = 7.86 (d, *J* = 8.6 Hz, 2H), 7.50 (d, *J* = 8.6 Hz, 2H), 7.36 (d, *J* = 2.0 Hz, 1H), 7.31 (dd, *J* = 8.4, 2.0 Hz, 1H), 7.14 (s, 1H), 6.93 (d, *J* = 8.4 Hz, 1H), 4.27 (s, 4H). ESI-MS (m/z) [M+H]⁺ 313.2 (100%), 315.2 (36%). HPLC retention time 14.1 min, gradient B. |
| **Compound 113:** |
| 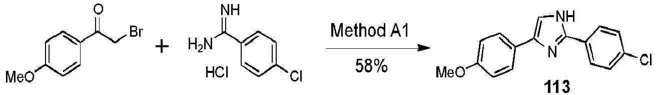 |
| ¹H NMR (400 MHz, DMSO-d₆ + 1% conc. DCI) *δ* = 8.35 (d, *J* = 8.8 Hz, 2H), 8.18 (s, 1H), 7.99 (d, *J* = 8.9 Hz, 2H), 7.74 (d, *J* = 8.0 Hz, 2H), 7.09 (d, *J* = 7.9 Hz, 2H), 3.82 (s, 1H). ESI-MS (m/z) [M+H]⁺ 285.2 (100%), 287.2 (34%). HPLC retention time 5.2 min, gradient B. |
| **Compound 114:** |
| 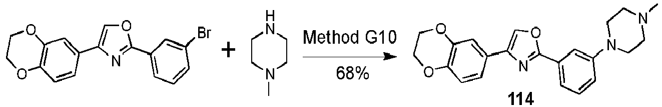 |
| ¹H NMR (400 MHz, DMSO-d₆) *δ* = 8.57 (s, 1H), 7.52 (m, 1H), 7.43 (d, *J* = 7.8 Hz, 1H), 7.40-7.31 (m, 3H), 7.11 (dd, *J* = 8.2, 2.3 Hz, 1H), 6.93 (d, *J* = 8.2 Hz, 1H), 4.28 (s, 4H), 3.22 (m, 4H), 2.48 (m, 4H), 2.23 (s, 3H). ESI-MS (m/z) [M+H]⁺ 378.3 (100%). HPLC retention time 19.3 min, gradient A. |
| Compound 115: |
| 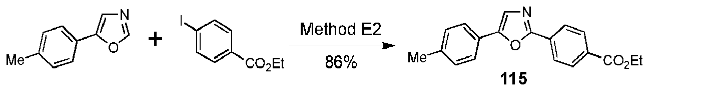 |
| ¹H NMR (400 MHz, DMSO-d₆) *δ* = 8.2 (d, *J =* 8.5 Hz, 2H), 8.11 (d, *J =* 8.5 Hz, 2H), 7.86 (s, 1H), 7.76 (d, *J* = 8.2 Hz, 2H), 7.33 (d, *J* = 8.2 Hz, 2H), 4.35 (q, *J* = 7.2 Hz, 2H), 2.36 (s, 3H), 1.35 (t, *J* = 7.2 Hz, 3H). ESI-MS (m/z) [M+H]⁺ 308.3 (100%). HPLC retention time 25.7 min, gradient B. |
| Compound 116: |
| 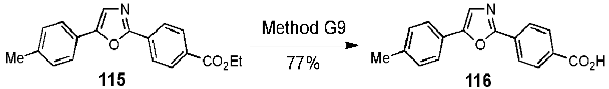 |
| ¹H NMR (400 MHz, DMSO-d₆) *δ* = 8.18 (d, *J* = 8.1 Hz, 2H), 8.09 (d, *J* = 8.1 Hz, 2H), 7.84 (s, 1H), 7.75 (d, *J =* 7.8 Hz, 2H), 7.32 (d, *J =* 7.8 Hz, 2H), 2.35 (s, 3H). ESI-MS (m/z) [M+H]⁺ 280.3 (100%). HPLC retention time 25.8 min, gradient A. |
| Compound 117: |
| 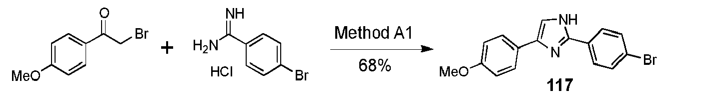 |
| ¹H NMR (400 MHz, DMSO-d₆ + 1% conc. DCI) *δ* = 8.28 (d, *J* = 8.6 Hz, 2H), 8.19 (s, 1H), 7.99 (d, *J* = 8.8 Hz, 2H), 7.88 (d, *J* = 8.6 Hz, 2H), 7.09 (d, *J* = 8.9 Hz, 2H), 3.82 (s, 1H). ESI-MS (m/z) [M+H]⁺ 329.2 (100%), 331.1 (1005). HPLC retention time 5.5 min, gradient B. |
| Compound 118: |
| 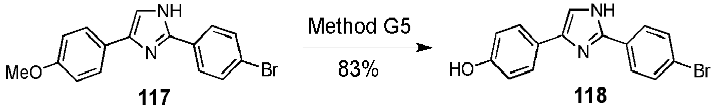 |
| ¹H NMR (400 MHz, DMSO-d₆) *δ* = 9.95 (bs, 1H), 8.11 (s, 1H), 8.03 (d, *J* = 8.7 Hz, 2H), 7.92 (d, *J* = 8.7 Hz, 2H), 7.72 (d, *J* = 8.6 Hz, 2H), 6.93 (d, *J* = 8.6 Hz, 2H). ESI-MS (m/z) [M+H]⁺ 315.1 (100%), 317.2 (98%). HPLC retention time 15.6 min, gradient A. |
| Compound 119: |
| 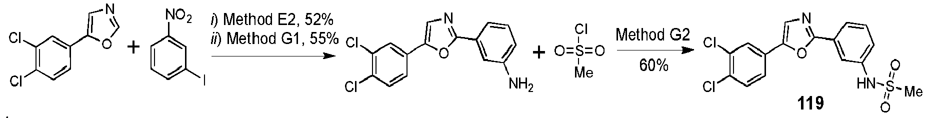 |
| ¹H NMR (400 MHz, DMSO-d₆) *δ* = 10.01 (s, 1H), 8.12 (d, *J* = 1.8 Hz, 1H), 8.01 (s, 1H), 7.95 (t, *J* = 1.9 Hz, 1H), 7.87 (d, *J* = 7.8 Hz, 1H), 7.84-7.76 (m, 2H), 7.53 (t, *J* = 8.0 Hz, 1H), 7.39 (m, 1H), 3.06 (s, 3H). ESI-MS (m/z) [M+H]⁺ 383.1 (100%), 385.2 (72%). HPLC retention time 15.8 min, gradient B. |
| Compound 120: |
| 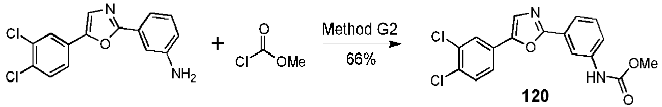 |
| ¹H NMR (400 MHz, DMSO-d₆) *δ* = 9.89 (s, 1H), 8.25 (t, *J =* 1.8 Hz, 1H), 8.10 (m, 1H), 7.99 (s, 1H), 7.80-7.73 (m, 3H), 7.62 (ddd, *J* = 8.1, 2.1, 1,0 Hz, 1H), 7.46 (t, *J* = 8.0 Hz, 1H), 3.70 (s, 3H). ESI-MS (m/z) [M+H]⁺ 363.2 (100%), 365.2 (65%). HPLC retention time 21.5 min, gradient B. |
| **Compound 121:** |
| 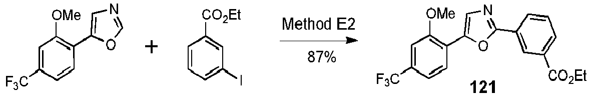 |
| ¹H NMR (400 MHz, DMSO-d₆) *δ* = 8.59 (s, 1H), 8.35 (d, *J* = 7.8 Hz, 1H), 8.13-8.03 (m, 2H), 7.82 (s, 1H), 7.71 (t, *J =* 7.8 Hz, 1H), 7.48-7.40 (m, 2H), 4.39 (q, *J =* 7.1 Hz, 2H), 4.07 (s, 3H), 1.38 (d, *J* = 7.1 Hz, 3H). ESI-MS (m/z) [M+H]⁺ 392.3 (100%). HPLC retention time 25.2 min, gradient B. |
| Compound 122: |
| 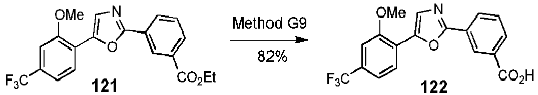 |
| ¹H NMR (400 MHz, DMSO-d₆) *δ* = 13.33 (bs, 1H), 8.57 (t, *J =* 1.5 Hz, 1H), 8.30 (dt, *J* = 8.0, 1.4 Hz, 1H), 8.10-8.01 (m, 2H), 7.80 (s, 1H), 7.67 (t, *J* = 7.8 Hz, 1H), 7.45-7.38 (m, 2H), 4.04 (s, 3H). ESI-MS (m/z) [M+H]⁺ 364.2 (100%). HPLC retention time 26.2 min, gradient A. |
| **Compound 123:** |
| 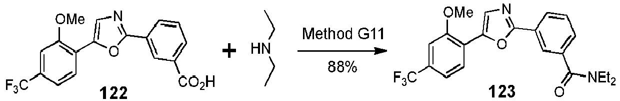 |
| ¹H NMR (400 MHz, DMSO-d₆) *δ* = 8.21-8.13 (m, 2H), 8.06 (t, *J* = 1.6 Hz, 1H), 7.85 (s, 1H), 7.64 (t, *J* = 7.7 Hz, 1H), 7.53 (dt, *J* = 7.7, 1.4 Hz, 1H), 7.47-7.42 (m, 2H), 4.07 (s, 3H), 3.48 (bs, 2H), 3.23 (bs, 2H), 1.29-1.00 (m, 6H). ESI-MS (m/z) [M+H]⁺ 419.4 (100%). HPLC retention time 20.8 min, gradient B. |
| **Compound 124:** |
| 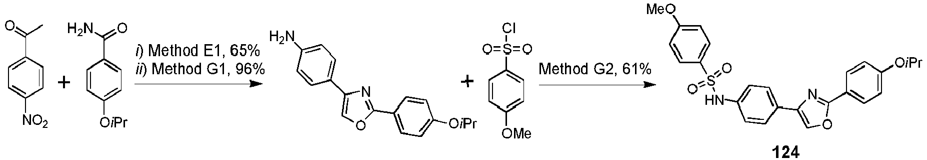 |
| ¹H NMR (400 MHz, DMSO-d₆) *δ* = 10.25 (s, 1H), 8.50 (s, 1H), 7.92 (d, *J* = 8.9 Hz, 2H), 7.74-7.65 (m, 4H), 7.16 (d, *J* = 8.6 Hz, 2H), 7.10-7.02 (m, 4H), 4.71 (m, *J* = 6.0 Hz, 1H), 3.78 (s, 3H), 1.29 (d, *J* = 6.0 Hz, 6H). ESI-MS (m/z) [M+H]⁺ 465.4 (100%). HPLC retention time 17.5 min, gradient B. |
| **Compound 125:** |
| 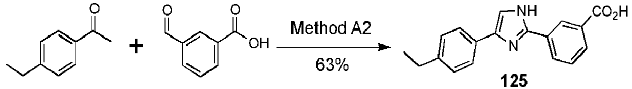 |
| ¹H NMR (400 MHz, DMSO-d₆ + 1% TFA-d₆) *δ* = 8.76 (s, 1H), 8.38 (d, *J* = 7.8 Hz, 1H), 8.27 (s, 1H), 8.19 (d, *J* = 7.7 Hz, 1H), 7.90-7.76 (m, 3H), 7.39 (d, *J* = 8.1 Hz, 2H), 2.67 (q, *J* = 7.5 Hz, 2H), 1.21 (t, *J* = 7.6 Hz, 3H). ESI-MS (m/z) [M+H]⁺ 293.2 (100%). HPLC retention time 17.0 min, gradient A. |
| **Compound 126:** |
| 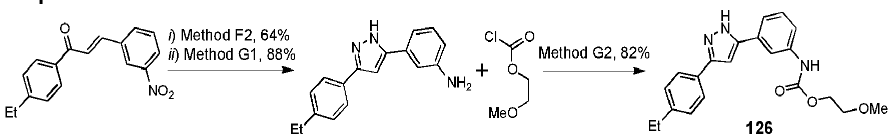 |
| ¹H NMR (400 MHz, DMSO-d₆ + 1% conc. DCI) *δ* = 9.85 (s, 1H), 8.06 (s, 1H), 7.75 (d, *J* = 8.2 Hz, 2H), 7.52-7.43 (m, 2H), 7.35 (t, *J* = 7.9 Hz, 1H), 7.27 (d, *J* = 8.2 Hz, 2H), 7.01 (s, 1H), 4.24 (m, 2H), 3.57 (m, 2H), 3.28 (s, 3H), 2.60 (q, *J* = 7.5 Hz, 2H), 1.17 (t, *J* = 7.5 Hz, 3H). ESI-MS (m/z) [M+H]⁺ |
| 366.4 (100%). HPLC retention time 24.1 min, gradient A. |
| **Compound 127:** |
| 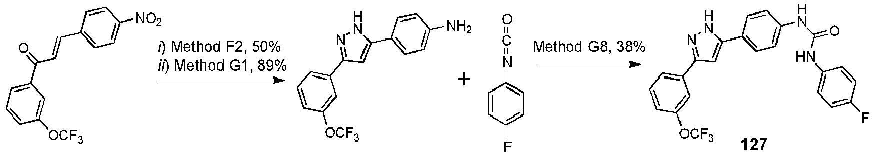 |
| ¹H NMR (400 MHz, DMSO-d₆ + 1% conc. DCI) *δ* = 9.01 (s, 1H), 8.95 (s, 1H), 7.89 (d, *J* = 7.9 Hz, 1H), 7.82 (s, 1H), 7.77 (d, *J* = 8.7 Hz, 2H), 7.62-7.53 (m, 3H), 7.53-7.47 (m, 2H), 7.30 (d, *J* = 8.2 Hz, 1H), 7.23 (s, 1H), 7.12 (t, *J* = 8.9 Hz, 2H). ESI-MS (m/z) [M+H]⁺ 457.3 (100%). HPLC retention time 14.2 min, gradient B. |
| **Compound 128:** |
| 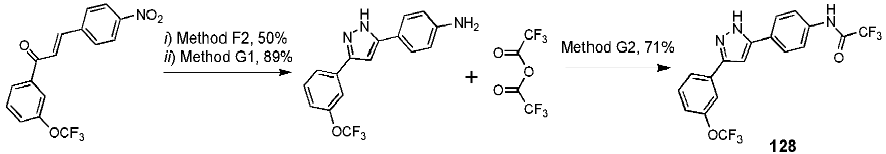 |
| ¹H NMR (400 MHz, DMSO-d₆ + 1% conc. DCI) *δ* = 11.39 (s, 1H), 7.91-7.85 (m, 3H), 7.83-7.76 (m, 3H), 7.58 (t, *J* = 8.0 Hz, 1H), 7.34-7.28 (m, 2H). ESI-MS (m/z) [M+H]⁺ 416.3 (100%). HPLC retention time 13.6 min, gradient B. |
| **Compound 129:** |
| 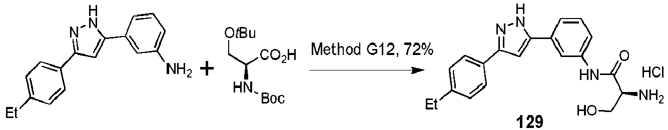 |
| ¹H NMR (400 MHz, DMSO-d₆) *δ* = 10.96 (s, 1H), 8.40 (bd, *J* = 4.6 Hz, 3H), 8.15 (s, 1H), 7.76 (d, *J* = 8.2 Hz, 2H), 7.66 (d, *J* = 8.0 Hz, 1H), 7.58 (d, *J* = 7.8 Hz, 1H), 7.41 (t, *J* = 8.0 Hz, 1H), 7.30 (d, *J* = 8.2 Hz, 2H), 7.04 (s, 1H), 4.11 (m, 1H), 3.92 (m, 2H), 2.63 (q, *J =* 7.5 Hz, 2H), 1.19 (t, *J* = 7.5 Hz, 3H). ESI-MS (m/z) [M+H]⁺ 351.3 (100%). HPLC retention time 17.3 min, gradient A. |
| **Compound 130:** |
| 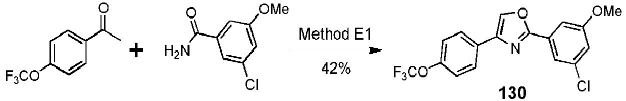 |
| ¹H NMR (400 MHz, DMSO-d₆) *δ* = 8.74 (s, 1H), 7.93 (d, *J* = 8.9 Hz, 2H), 7.52 (t, *J* = 1.7 Hz, 1H), 7.43-7.38 (m, 3H), 7.15 (t, *J* = 2.1 Hz, 1H), 3.84 (s, 3H). ESI-MS (m/z) [M+H]⁺ 370.2 (100%), 372.1 (35%). HPLC retention time 26.5 min, gradient B. |
| **Compound 131:** |
| 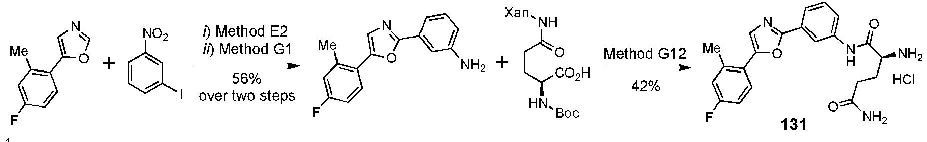 |
| ¹H NMR (400 MHz, DMSO-d₆) *δ* = 11.18 (s, 1H), 8.56-8.44 (m, 4H), 7.87-7.78 (m, 3H), 7.62 (s, 1H), 7.55 (t, *J* = 8.0 Hz, 1H), 7.51 (bs, 1H), 7.31-7.18 (m, 2H), 6.94 (bs, 1H), 4.11 (m, 1H), 2.53 (s, 3H), 2.28 (t, *J* = 7.5 Hz, 2H), 2.10 (m, 2H). ESI-MS (m/z) [M+H]⁺ 397.4 (100%). HPLC retention time 18.2 min, gradient A. |
| **Compound 132:** |
| 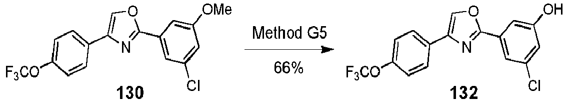 |
| ¹H NMR (400 MHz, DMSO-d₆) *δ* = 10.41 (s, 1H), 8.79 (s, 1H), 7.97 (d, *J* = 8.7 Hz, 2H), 7.50-7.43 (m, 3H), 7.40 (t, *J* = 1.7 Hz, 1H), 6.98 (t, *J* = 2.1 Hz, 1H). ESI-MS (m/z) [M+H]⁺ 356.2 (100%), 358.1 (34%). HPLC retention time 28.3 min, gradient A. |
| **Compound 133:** |
| 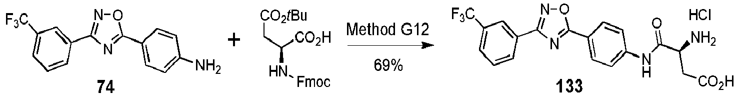 |
| ¹H NMR (400 MHz, DMSO-d₆) *δ* = 11.40 (bs, 1H), 8.37 (d, *J* = 7.8 Hz, 1H), 8.31 (s, 1H), 8.21 (d, *J* = 8.9 Hz, 2H), 8.01 (m, 1H), 7.94 (d, *J* = 8.9 Hz, 2H), 7.86 (t, *J* =7.9 Hz, 1H), 4.36 (dd, *J* = 7.0, 5.6 Hz, 1H), 3.02 (m, 2H). ESI-MS (m/z) [M+H]⁺ 421.3 (100%). HPLC retention time 19.6 min, gradient A. |
| **Compound 134:** |
| 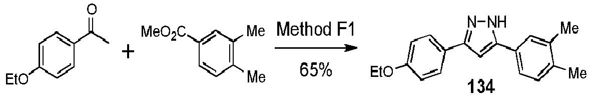 |
| ¹H NMR (400 MHz, DMSO-d₆ + 1 % conc. DCI) *δ* = 7.81 (d, *J* = 8.8 Hz, 2H), 7.68 (s, 1H), 7.60 (d, *J* = 7.7 Hz, 1H), 7.22 (d, *J* = 7.8 Hz, 1H), 7.17 (s, 1H), 7.01 (d, *J* = 8.8 Hz, 2H), 4.06 (q, *J* = 7.0 Hz, 2H), 2.28 (s, 3H), 2.25 (s, 3H), 1.33 (t, *J* = 7.0 Hz, 3H). ESI-MS (m/z) [M+H]⁺ 293.2 (100%). HPLC retention time 15.8 min, gradient B. |
| **Compound 135:** |
| 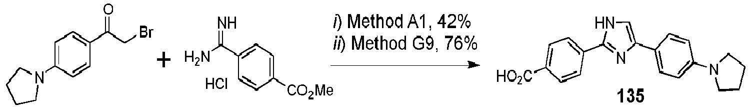 |
| ¹H NMR (400 MHz, DMSO-d₆ + 1% TFA) *δ* = 8.15 (d, *J* = 8.3 Hz, 2H), 8.10 (d, *J* = 8.3 Hz, 2H), 7.84 (s, 1H), 7.68 (d, *J* = 8.6 Hz, 2H), 6.60 (d, *J* = 8.6 Hz, 2H), 3.26 (m, 4H), 1.95 (m, 4H). ESI-MS (m/z) [M+H]⁺ 334.2 (100%). HPLC retention time 17.6 min, gradient A. |
| **Compound 136:** |
| 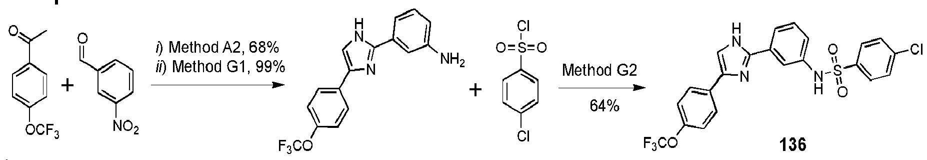 |
| ¹H NMR (400 MHz, DMSO-d₆ + 1% TFA) *δ* = 10.78 (s, 0.5H), 8.31 (s, 1H), 8.03 (d, *J* = 8.8 Hz, 2H), 7.88 (s, 1H), 7.82 (d, *J* = 8.7 Hz, 2H), 7.77 (d, *J* = 7.9 Hz, 1H), 7.63 (d, *J* = 8.7 Hz, 2H), 7.60-7.50 (m, 3H), 7.27 (d, *J* = 8.1 Hz, 1H). ESI-MS (m/z) [M+H]⁺ 494.1 (100%), 496.1 (42%). HPLC retention time 21.3 min, gradient A. |
| **Compound 137:** |
| 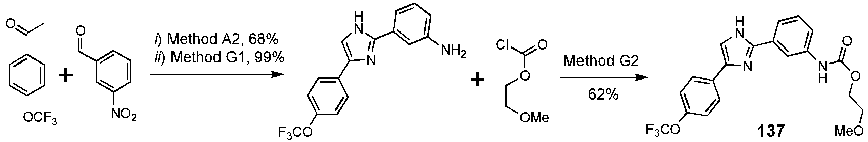 |
| ¹H NMR (400 MHz, DMSO-d₆ + 1% TFA) *δ* = 10.16 (s, 0.8H), 8.33-8.27 (m, 2H), 8.03 (d, *J* = 8.7 Hz, 2H), 7.70-7.53 (m, 5H), 4.26 (m, 2H), 3.59 (m, 2H), 3.29 (s, 3H). ESI-MS (m/z) [M+H]⁺ 422.3 (100%). HPLC retention time 19.1 min, gradient A. |
| **Compound 138:** |
| 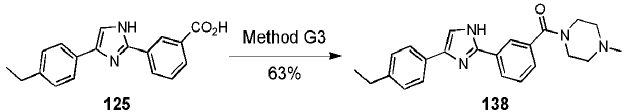 |
| ¹H NMR (400 MHz, DMSO-d₆ + 1% TFA) *δ* = 8.23-8.17 (m, 2H), 7.93 (s, 1H), 7.82 (d, *J* = 8.1 Hz, 2H), 7.66 (t, *J* = 7.8 Hz, 1H), 7.58 (d, *J* = 7.9 Hz, 1H), 7.29 (d, *J* = 8.1 Hz, 2H), 3.78 (bs, 4H), 3.32 (bs, 4H), 2.85 (s, 3H), 2.63 (q, *J* = 7.6 Hz, 2H), 1.20 (t, *J* = 7.6 Hz, 3H). ESI-MS (m/z) [M+H]⁺ 375.4 (100%). HPLC retention time 14.4 min, gradient A. |
| **Compound 139:** |
| 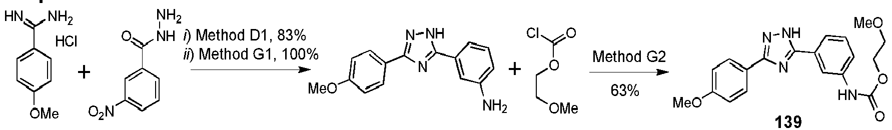 |
| ¹H NMR (400 MHz, DMSO-d₆ + 1% TFA) *δ* = 8.30 (s, 1H), 8.25 (d, *J* = 8.9 Hz, 2H), 7.92 (d, *J* = 7.7 Hz, 1H), 7.59 (d, *J* = 8.0 Hz, 1H), 7.47 (t, *J* = 8.0 Hz, 1H), 7.15 (d, *J* = 9.0 Hz, 2H), 4.22 (m, 2H), 3.84 (s, 3H), 3.56 (m, 2H), 3.27 (s, 3H). ESI-MS (m/z) [M+H]⁺ 369.3 (100%). HPLC retention time 19.0 min, gradient A. |
| **Compound 140:** |
| 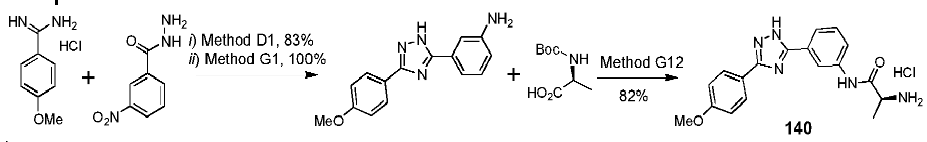 |
| ¹H NMR (400 MHz, DMSO-d₆) *δ* = 10.99 (s, 1H), 8.48-8.39 (m, 3H), 8.06 (d, *J* = 8.9 Hz, 2H), 7.85 (d, *J* = 8.0 Hz, 1H), 7.77 (m, 1H), 7.48 (t, *J* = 8.0 Hz, 1H), 7.11 (d, *J* = 9.0 Hz, 2H), 4.12 (m, 1H), 1.52 (d, *J* = 7.0 Hz, 3H). ESI-MS (m/z) [M+H]⁺ 338.3 (100%). HPLC retention time 14.3 min, gradient A. |
| **Compound 141:** |
| 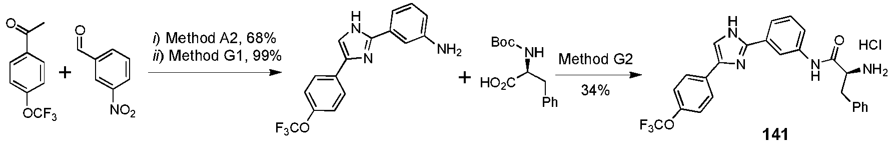 |
| ¹H NMR (400 MHz, DMSO-d₆) *δ* = 11.47 (s, 1H), 8.61 (bs, 2H), 8.38 (s, 1H), 8.32 (s, 1H), 8.19 (d, *J* = 8.8 Hz, 2H), 8.03 (d, *J* = 7.9 Hz, 1H), 7.82 (d, *J* = 8.1 Hz, 1H), 7.60 (t, *J* = 8.0 Hz, 1H), 7.53 (d, *J* = 8.2 Hz, 2H), 7.39 (d, *J* = 7.1 Hz, 2H), 7.31-7.23 (m, 3H), 4.44 (m, 1H), 3.24 (m. 1H). ESI-MS (m/z) [M+H]⁺ 467.3 (100%). HPLC retention time 17.2 min, gradient A. |
| **Compound 142:** |
| 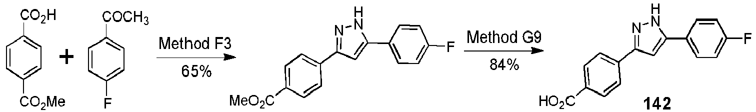 |
| ¹H NMR (400 MHz, DMSO-d₆ + 1% conc. DCI) *δ* = 8.04-7.95 (m, 4H), 7.94-7.87 (m, 2H), 7.34-7.25 (m, 3H). ESI-MS (m/z) [M+H]⁺ 283.2 (100%). HPLC retention time 20.6 min, gradient A. |
| **Compound 143:** |
| 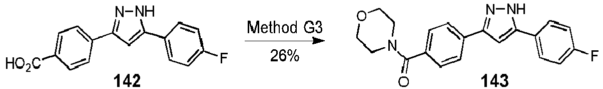 |
| ¹H NMR (400 MHz, DMSO-d₆ + 1% conc. DCI) *δ* = 7.98-7.88 (m, 4H), 7.48 (d, *J* = 8.0 Hz, 2H), 7.34-7.26 (m, 3H), 3.81-3.23 (m, 8H). ESI-MS (m/z) [M+H]⁺ 352.3 (100%). HPLC retention time 20.7 min, gradient A. |
| **Compound 144:** |
| 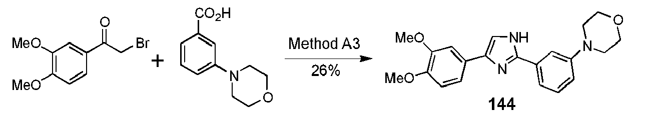 |
| ¹H NMR (400 MHz, DMSO-d₆ + 1% TFA) *δ* = 8.20 (s, 1H), 7.61 (s, 1H), 7.55-7.44 (m, 4H), 7.21 (m, 1H), 7.12 (d, *J* = 9.0 Hz, 1H), 3.86 (s, 3H), 3.82 (s, 3H), 3.78 (m, 4H), 3.24 (m, 4H). ESI-MS (m/z) [M+H]⁺ 366.3 (100%). HPLC retention time 16.1 min, gradient A. |

## Claims

1. Use of a compound of the formula (I) as an active ingredient for treatment or protection of plant diseases caused fungi, oomycetes or bacteria 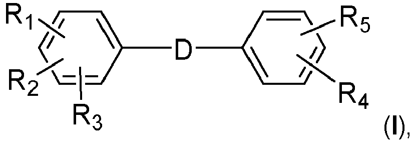 wherein
D represents 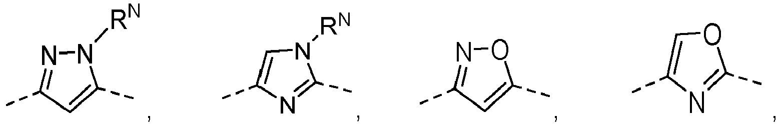 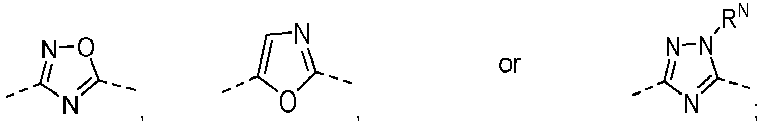
**R₁, R₂, R₃, R₄, R₅** are independently selected from hydrogen, halogen, hydroxy, C₁₋₄ alkoxy, C₁₋₄ alkyl, C₁₋₄ alkylene-OH, C₁₋₄ alkylene-OCH₃, -N**R^{E1}R^{E2}**, -OCF₃, -OCF₂CF₃, -NO₂, -CF₃, -CF₂CF₃, C₁₋₄ alkylthio, -C(=O)CH₃, -C(=O)CF₃, -COO**R^{E3}**, -C(=O)**NR^{E4}R^{E5}**, -NHC(=O)**R^{E6}**, -NHS(=O)₂**R^{E7}** wherein at least one of **R₁-R₅** is different from -H; or
**R₁**, and **R₂, R₂** and **R₃, R₄** and **R₅** together can non-directionally form a structure -**T**-(C**R^{E8}R^{E9}**)ₙ-V- as well as corresponding structures in which one or two double bond(s) is/are present, if they are attached to adjacent carbon atoms; and **T** is independently selected from C**R^{E10}R^{E11}**, N**R^{E1}** and O; and **V** is independently selected from C**R^{E8}R^{E9}**, N**R^{E1}** and O;
**R^{N}** is independently selected from hydrogen, C₁₋₄ alkyl, C₂₋₄ alkylene-OH, C₂₋₄ alkenyl, -CH₂-O**R^{E1}**; -CH₂CH₂-O**R^{E1}**;
**R^{E1}, R^{E2}** are independently selected from -H or C₁₋₄ alkyl; or -N**R^{E1}R^{E2}** forms a cyclic amine;
**R^{E3}** is selected from -H, C₁₋₄ alkyl, or -CH₂CH₂-O**R^{E1}**;
**R^{E4}, R^{E5}** are independently selected from -H, C₁₋₄ alkyl, 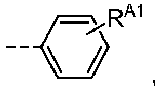 or 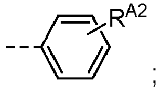 or -N**R^{E4}R^{E5}** forms a cyclic amine;
**R^{E6}** is selected from -H, C₁₋₄ alkyl, -CF₃, -CF₂CF₃, 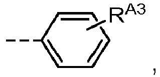 C₁₋₄ alkoxy, -OCH₂CH₂-O**R^{E1}**, -NHCH₂CH₂-O**R^{E1}**, -CH(NH₂)**R^{E12}**, -N**R^{E4}R^{E5}** or -N**R^{E4}R^{E5}** forms a cyclic amine,
**R^{E7}** is selected from -H, C₁₋₄ alkyl, -CF₃, -CF₂CF₃, 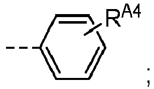
**R^{E8}, R^{E9}, R^{E10}, R^{E11}** are independently -H, -F, or C₁₋₄ alkyl;
wherein one or more hydrogens of the C₁₋₄ alkyl, C₁₋₄ alkoxy, and C₁₋₄ alkoxy are optionally substituted by halogen,
**n** is 1 or 2,
**R^{E12}** is selected from the group consisting of: -H, -CH₃, -CH₂OH, -CH₂SH, -CH(OH)CH₃, -CH(CH₃)₂, -CH₂CH(CH₃)₂, -CH(CH₃)CH₂CH₃, -CH₂CO₂H₇ -CH₂CONH₂, -CH₂CH₂CO₂H, -CH₂CH₂CONH₂, -CH₂CH₂CH₂NH-C(=NH)(NH₂), -CH₂CH₂SCH₃, -CH₂CH₂CH₂CH₂NH₂, 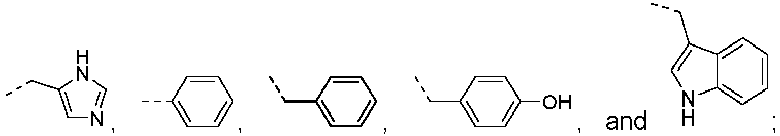
**R^{A1}, R^{A2}, R^{A3}**, and **R^{A4}**, represent independently of each other -H, -OH, -F, -Br, -Cl, -I, -CF₃, -OCF₃, C₁₋₄ alkyl, or C₁₋₄ alkoxy;
with the proviso that when the ring **D** is 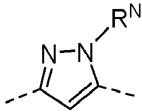 and one of **R₁** - **R₅** is COO**R^{E3}**, COO**R^{E3}** is not substituted at the ortho-position of the phenyl ring of the formula (I),
or tautomers, *N*-oxide, hydrates, solvates, metallic complexes, or acid salt forms thereof.

2. The use of a compound according to claim 1, wherein
**R₁** - **R₅** represent independently of each other -H, -F, -Br, -Cl, -I, -OH, -CF₃, -CH₃, -CH₂CH₃, -CH(CH₃)₂, -OCH₃, -OCH₂CH₃, -OCH(CH₃)₂, -N**R^{E1}R^{E2}**, -NO₂, -SCH₃, -SCH₂CH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃, -NHC(=O)NHCH₂CH₃, -NHC(=O)NHCH₂CH₂OCH₃, -NHC(=O)NHPh, -NHC(=O)NH(4-F-Ph), -NHC(=O)NH(4-MeO-Ph), -NHC(=O)NHCH(CH₃)₂, -NHC(=O)CH(NH₂)CH₂Ph, -NHC(=O)CH(NH₂)CH₃, -NHC(=O)CH(NH₂)CH₂OH, -NHC(=O)CH(NH₂)CH₂CO₂H, -NHC(=O)CH(NH₂)CH₂CH₂CONH₂, -NHC(=O)CH(NH₂)CH₂CH₂CH₂CH₂NH₂, -NHSO₂CH₃, -NHSO₂Ph, -NHSO₂(4-Cl-Ph), -NHSO₂(4-MeO-Ph), 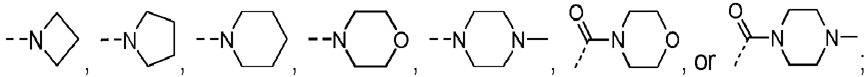
wherein at least one of **R₁-R₅** is different from -H; or
**R₁**, and **R₂, R₂** and **R₃, R₄** and **R₅** form together can non-directionally form a structure -T-(C**R^{E8}R^{E9}**)ₙ-V- as well as corresponding structures in which one or two double bond(s) is/are present, if they are attached to adjacent carbon atoms and **T** is selected from C**R^{E10}R^{E11}**, N**R^{E1}** and O and **V** is selected from C**R^{E8}R^{E9}**, N**R^{E1}** and O;
**R^{E8}, R^{E9}, R^{E10}, R^{E11}** are independently of each other -H or -F;
**n** is 1 or 2,
**R^{N}** is independently selected from hydrogen, C₁₋₄ alkyl, C₂₋₄ alkylene-OH, -C₂₋₄ alkenyl, -CH₂-O**R^{E1}**, -CH₂CH₂-O**R^{E1}**, and
**R^{E1}** and **R^{E2}** have the meanings as defined in Claim 1.

3. The use of the compound according to Claim 1 or 2, wherein the compound is defined by any one of the formulae (**IIa**) - (**IIg**): 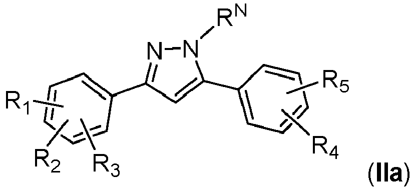 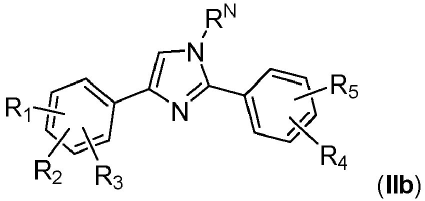 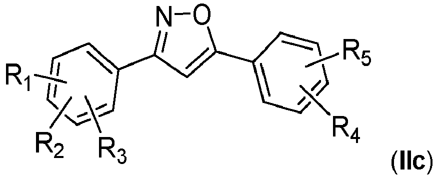 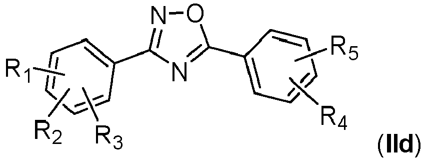 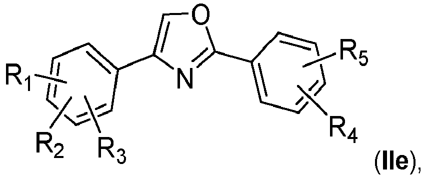 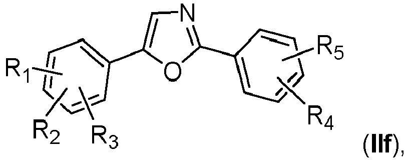 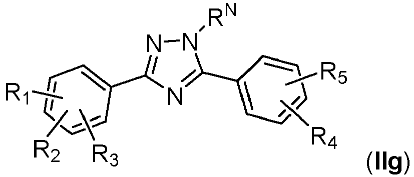 wherein **R₁**-**R₅** and **R^{N}** have the same meanings as defined in Claim 1.

4. The use of the compound according to any one of the Claims 1 - 3, wherein
**R₁**and **R₂** or **R₂** and **R₃** form together the moiety 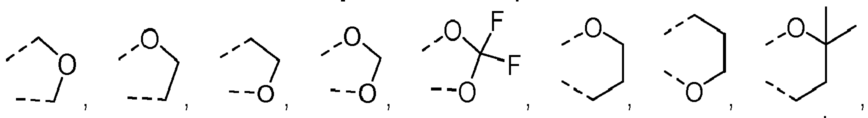 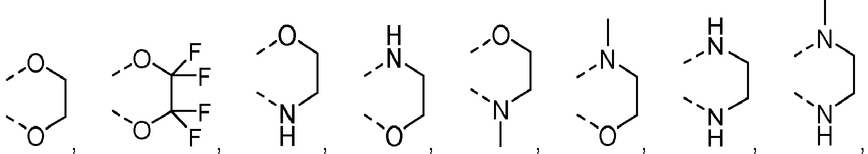 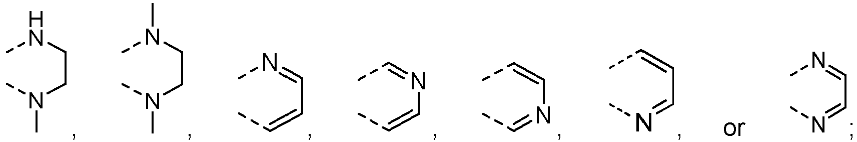
**R₄** and **R₅** forms together the moiety

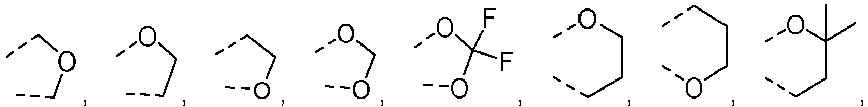

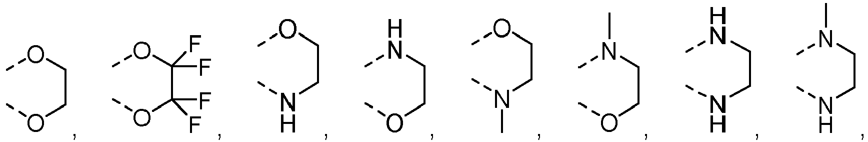

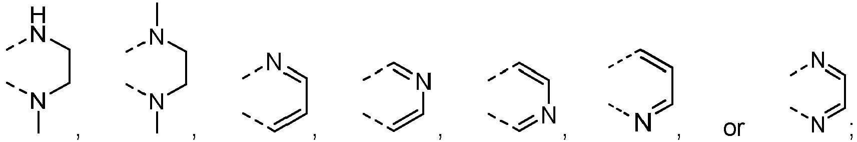

5. The use of the compound according to any one of the Claims 1 - 4, wherein
**R₁**- **R₅** represent independently of each other -H, -F, -Br, -Cl, -I, -OH, -CF₃, -CH₃, -CH₂CH₃, -CH(CH₃)₂, -OCH₃, -OCH₂CH₃, -OCH(CH₃)₂, -NH₂, -NH(CH₃), -N(CH₃)₂, -NO₂, -SCH₃, -SCH₂CH₃, -OCF₃, -COCH₃, -COCF₃, -COOH, -COOCH₃, -COOCH₂CH₃, -CONH₂, -CONHCH₃, -CON(CH₂CH₃)₂, -NHCOCH₃, -NHCOCF₃, -NHCOPh, -NHCO(4-Cl-Ph), -NHC(=O)OCH₃, -NHC(=O)OCH₂CH₃, -NHC(=O)OCH(CH₃)₂, -NHC(=O)OCH₂CH₂OCH₃, -NHC(=O)NHCH₂CH₃, -NHC(=O)NHCH₂CH₂OCH₃, -NHC(=O)NHPh, -NHC(=O)NH(4-F-Ph), -NHC(=O)NH(4-MeO-Ph), -NHC(=O)NHCH(CH₃)₂, -NHC(=O)CH(NH₂)CH₂Ph, -NHC(=O)CH(NH₂)CH₃, -NHC(=O)CH(NH₂)CH₂OH, -NHC(=O)CH(NH₂)CH₂CO₂H, -NHC(=O)CH(NH₂)CH₂CH₂CONH₂, -NHC(=O)CH(NH₂)CH₂CH₂CH₂CH₂NH₂, -NHSO₂CH₃, -NHSO₂Ph, -NHSO₂(4-Cl-Ph), -NHSO₂(4-MeO-Ph), 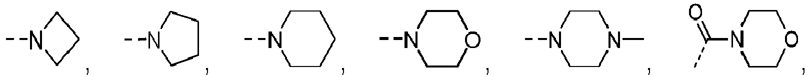 or 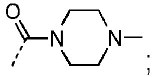
wherein at least one of **R₁** - **R₅** is different from -H; or
**R₁** and **R₂** or **R₂** and **R₃** form together the moiety 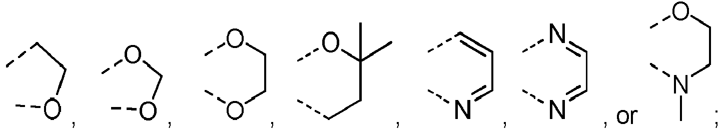 and
**R₄** and **R₅** forms together the moiety 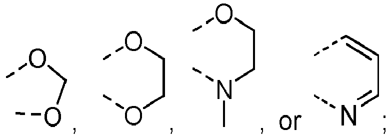 and
**R^{N}** is selected from hydrogen, C₁₋₄ alkyl, C₁₋₄ alkylene-OH, C₁₋₄ alkenyl, or C₁₋₄ alkoxy.

6. The use of the compound according to Claim 1, wherein the compound is selected from the group consisting of compounds:
| | |
|---|---|
| **1** | 4-(1,3-Benzodioxol-5-yl)-2-phenyl-1*H*-imidazole |
| **2** | 4-(3,4-Dihydroxyphenyl)-2-phenyl-1H-imidazole hydrobromide |
| **3** | 4-{3-[3-(4-Chlorophenyl)-1*H*-pyrazol-5-yl]phenyl}morpholine |
| **4** | 3-(3-Bromophenyl)-5-(3,4-dihydroxyphenyl)-1*H*-pyrazole |
| **5** | 3-(3,4-Dihydroxyphenyl)-5-(3,4,5-trihydroxyphenyl)-1*H*-pyrazole |
| **6** | 3-(3,5-Dibromophenyl)-5-(3,4,5-trihydroxyphenyl)-1*H*-pyrazole |
| **7** | 3-(1,3-Benzodioxol-5-yl)-5-(3-fluorophenyl)-1*H*-pyrazole |
| **8** | 3-(1,3-Benzodioxol-5-yl)-5-(3-iodophenyl)-1*H*-pyrazole |
| **9** | 3-(1,3-Benzodioxol-5-yl)-5-(3-methoxyphenyl)-1*H*-pyrazole |
| **10** | 3-(1,3-Benzodioxol-5-yl)-5-(2-bromophenyl)-1*H*-pyrazole |
| **11** | 3-(1,3-Benzodioxol-5-yl)-5-[3-(trifluoromethyl)phenyl]-1*H*-pyrazole |
| **12** | 3-(3,4-Dihydroxyphenyl)-5-[3-(trifluoromethyl)phenyl]-1*H*-pyrazole |
| **13** | 3-(3-Bromophenyl)-5-[4-(dimethylamino)phenyl]-1*H*-pyrazole |
| **14** | 3-(1,3-Benzodioxol-5-yl)-5-[3-(dimethylamino)phenyl]-1*H*-pyrazole |
| **15** | 3-(1,3-Benzodioxol-5-yl)-5-(4-iodophenyl)-1*H*-pyrazole |
| **16** | 3-(3,4-Dihydroxyphenyl)-5-(3-fluorophenyl)-1*H*-pyrazole |
| **17** | 3-(1,3-Benzodioxol-5-yl)-5-phenylisoxazole |
| **18** | 3-(3,4-Dihydroxyphenyl)-5-phenyl-1,2,4-oxadiazole |
| **19** | 5-(3,4-Dimethoxyphenyl)-3-(4-methoxyphenyl)isoxazole |
| **20** | 3-(3,4-Dimethoxyphenyl)-5-(3-fluorophenyl)isoxazole |
| **21** | 3-(3,4-Dihydroxyphenyl)-5-(3-fluorophenyl)isoxazole |
| **22** | 5-(3,4-Dihydroxyphenyl)-3-(4-hydroxyphenyl)isoxazole |
| **23** | 3-(1,3-Benzodioxol-5-yl)-5-(3-fluorophenyl)-1,2,4-oxadiazole |
| **24** | 3,5-Bis(3,4-Dihydroxyphenyl)-1,2,4-oxadiazole hydrobromide |
| **25** | 3-(3,4-Dihydroxyphenyl)-5-(3-fluorophenyl)-1,2,4-oxadiazole |
| **26** | 3,5-Bis(3,4-Dihydroxyphenyl)isoxazole hydrobromide |
| **27** | 3,5-Bis(3,4-Dihydroxyphenyl)-1*H*-pyrazole hydrobromide |
| **28** | 2,4-Bis(3,4-dihydroxyphenyl)-1*H*-imidazolehydrobromide |
| **29** | 3-(3-Bromophenyl)-5-(3,4-dimethoxyphenyl)-1*H*-pyrazole |
| **30** | 2,4-Bis(3,4-dimethoxyphenyl)-1*H*-imidazole |
| **31** | 3-(4-Chlorophenyl)-5-(4-methoxyphenyl)-1*H*-pyrazole |
| **32** | 3-(3,4-Dimethoxyphenyl)-5-(4-hydroxyphenyl)-1*H*-pyrazole |
| **33** | 3-(3,4-Dimethoxyphenyl)-5-(3-hydroxyphenyl)-1*H*-pyrazole |
| **34** | 3-(1,3-Benzodioxol-5-yl)-5-(3-hydroxyphenyl)-1*H*-pyrazole |
| **35** | 3-(1,3-Benzodioxol-5-yl)-5-(3-chlorophenyl)-1*H*-pyrazole |
| **36** | 3-(4-Chlorophenyl)-5-(4-hydroxyphenyl)-1*H*-pyrazole hydrobromide |
| **37** | 2-(1,3-Benzodioxol-5-yl)-4-(3-bromophenyl)-1*H*-imidazole |
| **38** | 3-(1,3-Benzodioxol-5-yl)-5-(4-bromophenyl)-1*H*-pyrazole |
| **39** | 3-(4-Aminophenyl)-5-(3-chlorophenyl)-1*H*-pyrazole |
| **40** | 3-(4-Aminophenyl)-5-(3-bromophenyl)-1*H*-pyrazole |
| **41** | 3-(1,3-Benzodioxol-5-yl)-5-(3-bromophenyl)-1-methyl-1*H*-pyrazole |
| **42** | 3-(3-Bromophenyl)-5-[4-(methylamino)phenyl]-1*H*-pyrazole |
| **43** | 3,5-Bis(3,4-dimethoxyphenyl)isoxazole |
| **44** | 7-[5-(3-Bromophenyl)-1*H*-pyrazol-3-yl]-4-methyl-3,4-dihydro-2*H*-1,4-benzoxazine |
| **45** | 5-(4-Aminophenyl)-3-(2-fluorophenyl)-isoxazole |
| **46** | *N*-{4-[3-(2-Fluorophenyl)-isoxazol-5-yl]phenyl}acetamide |
| **47** | *N*-{4-[3-(2-Fluorophenyl)-isoxazol-5-yl]phenyl}-4-methylbenzenesulfonamide |
| **48** | 5-(4-Chloro-3-methoxyphenyl)-3-[3-(trifluoromethyl)phenyl]-isoxazole |
| **49** | 5-(4-Chloro-3-hydroxyphenyl)-3-[3-(trifluoromethyl)phenyl]-isoxazole |
| **50** | 3-(3-Bromophenyl)-5-(2,3-dihydro-1,4-benzodioxin-6-yl)-1*H*-pyrazole |
| **51** | 3-(4-Fluorophenyl)-5-(3-methoxyphenyl)-1,2,4-oxadiazole |
| **52** | 3-(2-Chlorophenyl)-5-[4-(ethylsulfanyl)phenyl]-isoxazole |
| **53** | 5-(3,4-Dimethylphenyl)-3-[3-(trifluoromethoxy)phenyl]-isoxazole |
| **54** | 2,2,2-Trifluoro-*N*-{3-[2-(4-fluorophenyl)-1*H*-imidazol-5-yl]phenyl}acetamide |
| **55** | *N*-(4-Hydroxyphenyl)-4-{4-[4-(pyrrolidin-1-yl)phenyl]-1*H*-imidazol-2-yl}benzamide |
| **56** | 4-[5-(4-Chlorophenyl)-1*H*-1,2,4-triazol-3-yl]benzoicacidhydrochloride |
| **57** | 1-{4-[5-(3-Fluorophenyl)-1,2-oxazol-3-yl]phenyl}-3-(4-methoxyphenyl)urea |
| **58** | (2S)-2,6-Diamino-*N*-(4-{3-[4-(trifluoromethoxy)phenyl]-1*H*-1,2,4-triazol-5-yl}phenyl)hexanamide dihydrochloride |
| **59** | {4-[5-(4-Chlorophenyl)-1*H*-1,2,4-triazol-3-yl]phenyl}(morpholin-4-yl)methanone hydrochloride |
| **60** | 6-[4-(4-Hydroxyphenyl)-1,3-oxazol-2-yl]quinoline |
| **61** | 2,2,2-Trifluoro-*N*-(4-{5-[4-(trifluoromethoxy)phenyl]-1*H*-1,2,4-triazol-3-yl}phenyl)acetamide |
| **62** | 3-[5-(2-Fluorophenyl)-1,2,4-oxadiazol-3-yl]benzoic acid |
| **63** | Ethyl 3-[5-(2-fluorophenyl)-1,2,4-oxadiazol-3-yl]benzoate |
| **64** | 5-(4-Methoxyphenyl)-3-[4-(trifluoromethyl)phenyl]-1,2,4-oxadiazole |
| **65** | 5-[2-Chloro-5-(methylsulfanyl)phenyl]-3-[3-(trifluoromethoxy)phenyl]-1,2,4-oxadiazole |
| **66** | 2-(4-Ethylphenyl)-4-[4-(trifluoromethoxy)phenyl]-1*H*-imidazole |
| **67** | 5-(3-Chlorophenyl)-3-(4-methoxyphenyl)-1,2,4-oxadiazole |
| **68** | 5-(2-Bromophenyl)-3-(4-methoxyphenyl)-1,2,4-oxadiazole |
| **69** | 3-{5-[2-Hydroxy-4-(trifluoromethyl)phenyl]-1,3-oxazol-2-yl}benzoic acid |
| **70** | 5-(4-Chlorophenyl)-3-(4-hydroxyphenyl)-1,2,4-oxadiazole |
| **71** | 3-(2,3-Dihydro-1,4-benzodioxin-6-yl)-5-[3-(trifluoromethyl)phenyl]-1*H-*pyrazole |
| **72** | 5-(4-Hydroxyphenyl]-3-[4-(trifluoromethyl)phenyl]-1,2,4-oxadiazole |
| **73** | 5-(4-Nitrophenyl)-3-[3-(trifluoromethyl)phenyl]-1,2,4-oxadiazole |
| **74** | 5-(4-Aminophenyl)-3-[3-(trifluoromethyl)phenyl]-1,2,4-oxadiazole |
| **75** | Propan-2-yl (4-{3-[3-(trifluoromethyl)phenyl]-1 ,2,4-oxadiazol-5-yl}phenyl)carbamate |
| **76** | 2-(4-Chlorophenyl)-5-[4-(trifluoromethoxy)phenyl]-1*H*-imidazole |
| **77** | Methyl (4-{5-[4-(trifluoromethoxy)phenyl]-1*H*-1,2,4-triazol-3-yl}phenyl)carbamate |
| **78** | 2-(4-Chlorophenyl)-5-(4-hydroxyphenyl)-1*H*-imidazole |
| **79** | 5-(3,4-Dichlorophenyl)-2-(3-methylphenyl)-1*H*-imidazole |
| **80** | 5-(3-Chlorophenyl)-3-(2,3-dihydro-1,4-benzodioxin-6-yl)-1*H*-pyrazole |
| **81** | 5-(2-Hydroxy-5-fluorophenyl)-2-[4-(trifluoromethyl)phenyl]-1*H*-imidazole |
| **82** | 1-(2-Methoxyethyl)-3-(4-{5-[4-(trifluoromethyl)phenyl]-1*H*-1,2,4-triazol-3-yl}phenyl)urea |
| **83** | 4-{3-[3-(Trifluoromethyl)phenyl]-1,2-oxazol-5-yl}benzoic acid |
| **84** | 2-(4-Bromophenyl)-5-[4-(trifluoromethoxy)phenyl]-1*H*-imidazole |
| **85** | 3-(4-Hydroxyphenyl)-5-(4-methylphenyl)-1,2-oxazole |
| **86** | 2-(3-Bromophenyl)-5-(3-methoxyphenyl)-1*H*-imidazole |
| **87** | 2-(3-Bromophenyl)-5-(3-hydroxyphenyl)-1*H*-imidazole |
| **88** | *N*-{3-[2-(4-fluorophenyl)-1*H*-imidazol-5-yl]phenyllbenzamide |
| **89** | 3-(4-Methylphenyl)-5-[4-(methylsulfonyl)phenyl]-1,2,4-oxadiazole |
| **90** | 5-[4-(Azetidin-1-yl)phenyl]-3-(4-ethylphenyl)-1,2-oxazole |
| **91** | 1-{4-[3-(2-Chlorophenyl)-1,2-oxazol-5-yl]phenyl}piperidine |
| **92** | 4-Chloro-*N*-(4-{5-[4-(trifluoromethyl)phenyl]-1*H*-1,2,4-triazol-3-yl}phenyl)benzenesulfonamide |
| **93** | (2S)-2-Amino-*N*-{4-[5-(3-fluorophenyl)-1,2-oxazol-3-yl]phenyl}-3-methylbutanamide hydrochloride |
| **94** | 1-Phenyl-3-(4-{3-[3-(trifluoromethyl)phenyl]-1,2,4-oxadiazol-5-yl}phenyl) urea |
| **95** | Ethyl 4-{5-[4-(methylsulfanyl)phenyl]-1,2,4-oxadiazol-3-yl}benzoate |
| **96** | 4-{5-[4-(Methylsulfanyl)phenyl]-1,2,4-oxadiazol-3-yl}benzoic acid |
| **97** | 5-(3-Bromophenyl)-3-(2,2-dimethyl-3,4-dihydro-2*H*-chromen-6-yl)-1*H-*pyrazole |
| **98** | 4-Methyl-7-{3-[3-(morpholin-4-yl)phenyl]-1,2,4-oxadiazol-5-yl}-3,4-dihydro-2H-1,4-benzoxazine |
| **99** | 3-(4-Fluorophenyl)-5-(2-methyl-5-nitrophenyl)-1*H*-1,2,4-triazole |
| **100** | 5-(3-Amino-6-methylphenyl)-3-(4-fluorophenyl)-1*H*-1,2,4-triazole |
| **101** | 1-Ethyl-3-{3-[3-(4-fluorophenyl)-1*H*-1,2,4-triazol-5-yl]-4-methylphenyl}urea |
| **102** | 3-Chloro-N-{3-[3-(4-fluorophenyl)-1*H*-1,2,4-triazol-5-yl]-4-methylphenyl}benzamide |
| **103** | (2*S*)-2-Amino-*N*-{3-[3-(4-fluorophenyl)-1*H*-1,2,4-triazol-5-yl]-4-methylphenyl)-3-phenylpropanamide hydrochloride |
| **104** | 3-(1,3-Benzodioxol-5-yl)-5-(3,5-difluorophenyl)-1*H*-pyrazole |
| **105** | 3-[3-(3,4-Dimethoxyphenyl)-1*H*-1,2,4-triazol-5-yl]-*N*,*N*-dimethylaniline |
| **106** | 5-(3-Ethoxyphenyl)-3-(3-methylphenyl)-1*H*-1,2,4-triazole |
| **107** | 5-(3-Hydroxyphenyl)-3-(3-methylphenyl)-1*H*-1,2,4-triazole |
| **108** | Ethyl {3-[2-(4-fluorophenyl)-1*H*-imidazol-5-yl]phenyl}carbamate |
| **109** | 4-{4-[5-(2,3-Dihydro-1-benzofuran-5-yl)-1*H*-imidazol-2-yl]phenyl}morpholine |
| **110** | 4-(4-Ethoxyphenyl)-2-[3-(propan-2-yl)phenyl]-1,3-oxazole |
| **111** | 5-(4-Chlorophenyl)-3-(2,3-dihydro-1,4-benzodioxin-6-yl)-1*H*-pyrazole |
| **112** | 6-[5-(3-Bromophenyl)-1*H*-pyrazol-3-yl]quinoxaline |
| **113** | 2-(4-Chlorophenyl)-5-(4-methoxyphenyl)-1*H*-imidazole |
| **114** | 1-Methyl-4-{3-[4-(2,3-dihydro-1,4-benzodioxin-6-yl)-1,3-oxazol-2-yl]phenyl}piperazine |
| **115** | Ethyl 4-[5-(4-methylphenyl)-1 ,3-oxazol-2-yl]benzoate |
| **116** | 4-[5-(4-Methylphenyl)-1,3-oxazol-2-yl]benzoic acid |
| **117** | 2-(4-Bromophenyl)-5-(4-methoxyphenyl)-1*H*-imidazole |
| 118 | 2-(4-Bromophenyl)-5-(4-hydroxyphenyl)-1*H*-imidazole |
| **119** | *N*-{3-[5-(3,4-Dichlorophenyl)-1,3-oxazol-2-yl]phenyl}methanesulfonamide |
| **120** | Methyl {3-[5-(3,4-dichlorophenyl)-1,3-oxazol-2-yl]phenyl}carbamate |
| **121** | Ethyl 3-{5-[2-methoxy-4-(trifluoromethyl)phenyl]-1,3-oxazol-2-yl}benzoate |
| **122** | 3-{5-[2-Methoxy-4-(trifluoromethyl)phenyl]-1,3-oxazol-2-yl}benzoic acid |
| **123** | *N*,*N*-diethyl-3-{5-[2-methoxy-4-(trifluoromethyl)phenyl]-1,3-oxazol-2-yl}benzamide |
| **124** | 4-Methoxy-*N*-{4-[2-[4-(propan-2-yloxy)phenyl]-1,3-oxazol-4-yl]phenyl}benzenesulfonamide |
| **125** | 3-[4-(4-Ethylphenyl)-1*H*-imidazol-2-yl]benzoic acid |
| **126** | 2-Methoxyethyl {3-[5-(4-ethylphenyl)-1*H*-pyrazol-3-yl]phenyl}carbamate |
| **127** | 1-(4-Fluorophenyl)-3-(4-{5-[3-(trifluoromethoxy)phenyl]-1*H*-pyrazol-3-yl}phenyl)urea |
| **128** | 2,2,2-Trifluoro-*N*-(4-{5-[3-(trifluoromethoxy)phenyl]-1*H*-pyrazol-3-yl}phenyl)acetamide |
| **129** | (2*S*)-2-Amino-*N*-{3-[3-(4-ethylphenyl)-1*H*-pyrazol-5-yl]phenyl}-3-hydroxypropanamide hydrochloride |
| **130** | 2-(3-Chloro-5-methoxyphenyl)-4-[4-(trifluoromethoxy)phenyl]-1,3-oxazole |
| **131** | (2*S*)-2-Amino-*N*¹-{3-[5-(4-fluoro-2-methylphenyl)-1,3-oxazol-2-yl]phenyl}pentanediamide hydrochloride |
| **132** | 2-(3-Chloro-5-hydroxyphenyl)-4-[4-(trifluoromethoxy)phenyl]-1,3-oxazole |
| **133** | (3*S*)-3-Amino-4-oxo-4-[(4-{3-[3-(trifluoromethyl)phenyl]-1,2,4-oxadiazol-5-yl}phenyl)amino]butanoic acid hydrochloride |
| **134** | 3-(3,4-Dimethylphenyl)-5-(4-ethoxyphenyl)-1*H*-pyrazole |
| **135** | 4-{4-[4-(Pyrrolidin-1-yl)phenyl]-1*H*-imidazol-2-yl}benzoic acid |
| **136** | 4-Chloro-N-(3-{4-[4-(trifluoromethoxy)phenyl]-1*H*-imidazol-2-yl}phenyl)benzenesulfonamide |
| **137** | 2-Methoxyethyl (3-{4-[4-(trifluoromethoxy)phenyl]-1*H*-imidazol-2-yl}phenyl)carbamate |
| **138** | (4-Methylpiperazin-1-yl){3-[4-(4-methylphenyl)-1H-imidazol-2-yl]phenyl]methanone |
| **139** | 2-Methoxyethyl {3-[3-(4-methoxyphenyl)-1*H*-1,2,4-triazol-5-yl]phenyl}carbamate |
| **140** | (2*S*)-2-Amino-*N*-{3-[3-(4-methoxyphenyl)-1*H*-1,2,4-triazol-5-yl]phenyl}propenamidehydrochloride |
| **141** | (2S)-2-Amino-*N*-{3-[4-(4-trifluoromethoxyphenyl)-1*H*-imidazol-2-yl]phenyl}-3-phenylpropanamide hydrochloride |
| **142** | 4-[5-(4-Fluorophenyl)-1*H*-pyrazol-3-yl]benzoic acid |
| **143** | {4-[5-(4-Fluorophenyl)-1*H*-pyrazol-3-yl]phenyl}(morpholin-4-yl)methanone |
| **144** | 4-{3-[4-(3,4-Dimethoxyphenyl)-1*H*-imidazol-2-yl]phenyl}morpholine |
or acid salt forms thereof.

7. The use of the compound according to any one of the claims 1 - 6, wherein the plant disease caused by fungi, oomycetes or bacteria is selected from the group consisting of: Blumeria diseases, Podosphaera diseases, Sphaerotheca diseases, Uncinula diseases, Gymnosporangium diseases, Hemileia diseases, Phakopsora diseases, Puccinia diseases, Uromyces diseases, Albugo diseases, Bremia diseases, Peronospora diseases, Phytophthora diseases, Plasmopara disease, Pseudoperonospora diseases, Pythium diseases, Altemaria disease, Cercospor diseases, Cladiosporum diseases, Cochliobolus diseases, Colletotrichum diseases, Cycloconium disease, Diaporthe disease, Elsinoe diseases, Gloeosporium diseases, Glomerella diseases, Guignardia, Leptosophaeria diseases, Magnaporthe diseases, Mycosphaerella diseases, Phaeosphaeria diseases, Pyrenophora diseases, Ramularia diseases, Rhynchosporium diseases, Septoria diseases, Typhula diseases, Venturia diseases, Corticium diseases, Fusarium diseases, Gaeumannomyces diseases, Rhizoctonia diseases, Sarocladium diseases, Sclerotium diseases, Tapesia diseases, Thielaviopsis diseases, Alternaria diseases, Aspergillus diseases, Cladosporium diseases, Claviceps diseases, Fusarium diseases, Gibberella diseases, Monographella diseases, Sphacelotheca diseases, Tilletia diseases, Urocystis diseases, Ustilago diseases, Aspergillus diseases, Botrytis diseases, Penicillium diseases, Rhizopus diseases, Sclerotinia diseases, Verticilium diseases, Alternaria diseases, Aphanomyces diseases, Ascochyta diseases, Aspergillus diseases, Cladosporium diseases, Cochliobolus diseases, Colletotrichum diseases, Fusarium diseases, Gibberella diseases, Macrophomina diseases, Monographella diseases, Penicillium diseases, Phoma diseases, Phomopsis diseases, Phytophthora diseases, Pyrenophora diseases, Pyricularia diseases, Pythium diseases, Rhizoctonia diseases, Rhizopus diseases, Sclerotium diseases, Septoria diseases, Typhula diseases, Verticillium diseases, Nectria diseases, Monilinia diseases, Exobasidium diseases, Taphrina diseases, Esca diseases, Eutypa dyeback, Ganoderma diseases, Rigidoporus diseases, Botrytis diseases, Rhizoctonia diseases, Helminthosporium diseases, Hymenoscyphus diseases, and Plasmodiophora diseases.

8. The use of the compound according to claim 7, wherein the plant disease caused by oomycetes is Albugo disease caused by Albugo candida or Albugo laibachii, Bremia disease caused by Bremia lactucae, Peronospora disease caused by Peronospora pisi or P. brassicae, Phytophthora disease caused by Phytophthora infestans, P. capsica, P. cinnamomi, P. nicotianae, P. palmivora, P. fragariae or P. sojae, Plasmopara disease caused by Plasmopara viticola, and Pseudoperonospora disease caused by Pseudoperonospora humuli or Pseudoperonospora cubensis.

9. A composition comprising as an active ingredient an effective amount of a compound of formula (I) according to any one of claims 1 - 6 and an agriculturally acceptable support, carrier or filler.

10. The composition according to Claim 9 further comprising at least one fungicide or at least one bactericide.

11. Use of the composition according to claim 9 or 10 for treatment or protection of plant diseases caused fungi, oomycetes or bacteria, wherein the plant disease caused by fungi, oomycetes or bacteria is
Blumeria diseases, Podosphaera diseases, Sphaerotheca diseases, Uncinula diseases, Gymnosporangium diseases, Hemileia diseases, Phakopsora diseases, Puccinia diseases, Uromyces diseases, Albugo diseases, Bremia diseases, Peronospora diseases, Phytophthora diseases, Plasmopara disease, Pseudoperonospora diseases, Pythium diseases, Altemaria disease, Cercospor diseases, Cladiosporum diseases, Cochliobolus diseases, Colletotrichum diseases, Cycloconium disease, Diaporthe disease, Elsinoe diseases, Gloeosporium diseases, Glomerella diseases, Guignardia, Leptosophaeria diseases, Magnaporthe diseases, Mycosphaerella diseases, Phaeosphaeria diseases, Pyrenophora diseases, Ramularia diseases, Rhynchosporium diseases, Septoria diseases, Typhula diseases, Venturia diseases, Corticium diseases, Fusarium diseases, Gaeumannomyces diseases, Rhizoctonia diseases, Sarocladium diseases, Sclerotium diseases, Tapesia diseases, Thielaviopsis diseases, Alternaria diseases, Aspergillus diseases, Cladosporium diseases, Claviceps diseases, Fusarium diseases, Gibberella diseases, Monographella diseases, Sphacelotheca diseases, Tilletia diseases, Urocystis diseases, Ustilago diseases, Aspergillus diseases, Botrytis diseases, Penicillium diseases, Rhizopus diseases, Sclerotinia diseases, Verticilium diseases, Alternaria diseases, Aphanomyces diseases, Ascochyta diseases, Aspergillus diseases, Cladosporium diseases, Cochliobolus diseases, Colletotrichum diseases, Fusarium diseases, Gibberella diseases, Macrophomina diseases, Monographella diseases, Penicillium diseases, Phoma diseases, Phomopsis diseases, Phytophthora diseases, Pyrenophora diseases, Pyricularia diseases, Pythium diseases, Rhizoctonia diseases, Rhizopus diseases, Sclerotium diseases, Septoria diseases, Typhula diseases, Verticillium diseases, Nectria diseases, Monilinia diseases, Exobasidium diseases, Taphrina diseases, Esca diseases, Eutypa dyeback, Ganoderma diseases, Rigidoporus diseases, Botrytis diseases, Rhizoctonia diseases, Helminthosporium diseases, Hymenoscyphus diseases, and Plasmodiophora diseases.

12. The use of the composition according to claim 11, wherein the plant disease caused by oomycetes is Albugo disease caused by Albugo Candida or Albugo laibachii, Bremia disease caused by Bremia lactucae, Peronospora disease caused by Peronospora pisi or P. brassicae, Phytophthora disease caused by Phytophthora infestans, P. capsica, P. cinnamomi, P. nicotianae, P. palmivora, P. fragariae or P. sojae, Plasmopara disease caused by Plasmopara viticola, and Pseudoperonospora disease caused by Pseudoperonospora humuli or Pseudoperonospora cubensis.

13. A method for treatment or protection of a plant disease caused fungi, oomycetes or bacteria, **characterized in that** an agronomically effective and non-phytotoxic quantity of a compound according to any one of the claims 1 - 6 or a composition according to claim 9 or 10 is applied to the soil where plants grow or are capable of growing, to the leaves and/or the fruit of plants or to the seeds of plants.

14. A compound of the formula (I) 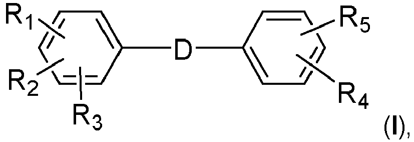 wherein
**D** represents 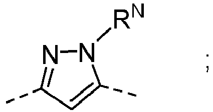
**R₁** and **R₂** are hydrogen and **R₃** represents -Cl, hydroxyl, -OCF₃, -OC₂H₅, -C₂H₅, -NH-CO-CH(NH₂)-CH₂-OH, or
**R₁** is hydrogen and **R₂** and **R₃** form together the residue -O-CH₂-O-;
**R₄** is hydrogen and **R₅** represents -C₂H₅, -N(CH₃)₂, -NH-CO-CF₃, -I, -NH-COO-CH₂CH₂-OCH₃, -NH-C(=O)-NH-**R^{E5}** or
**R₄** and **R₅** represent -CH₃, 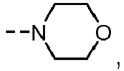
**R^{E5}** represents 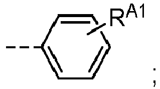
**R^{A1}** represents -F;
**R^{N}** represents hydrogen;
or tautomers, *N*-oxide, hydrates, solvates, metallic complexes, or acid salt forms thereof;
or
**D** represents 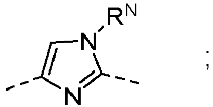
**R₁** and **R₂** are hydrogen and **R₃** represents -NH-CO-CF₃, -OCF₃, 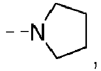 -OH, -NH-CO-Ph, -NH-CO-OC₂H₅, -C₂H₅, or
**R₁** is hydrogen and **R₂** and **R₃** represent independently of each other -Cl, -OCH₃, -OH, -F; or
**R₁** is hydrogen and **R₂** and **R₃** form together the residue -CH₂-CH₂-O-;
**R₄** is hydrogen and **R₅** represents -CH₃, -C₂H₅, -F, -Cl, -Br, -CF₃, -C(=O)NH**R^{E5}**, 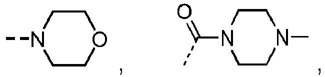 -NH-COO-CH₂CH₂-OCH₃, -NHS(=O)₂**R^{E7}**, -NH-CO-CH(NH₂)-CH₂Ph, -N(CH₃)-CH₂CH₂-OCH₃, -COOH; or
**R₄** and **R₅** represent -OCH₃;
**R^{E5}** represents 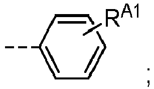
**R^{E7}** represents 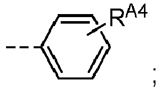
**R^{A1}** represents -OH;
**R^{A4}** represents -Cl;
**R^{N}** represents hydrogen;
or tautomers, *N*-oxide, hydrates, solvates, metallic complexes, or acid salt forms thereof;
or
**D** represents 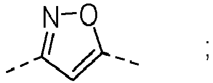
**R₁** and **R₂** are hydrogen and **R₃** represents -Cl, -C₂H₅, -CF₃, -NHC(=O)-NH**R^{E5}**, -F, -OCF₃, -NH-CO-CH(NH₂)-CH(CH₃)₂;
**R₄** is hydrogen and **R₅** represents -NH₂, -SC₂H₅, -F, 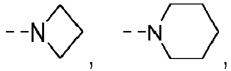 -NHS(=O)₂**R^{E7}**, -COOH, -NH-CO-CH₃; or
**R₄** and **R₅** represent independently of each other -Cl, -OCH₃, -OH, -CH₃;
**R^{E5}** represents 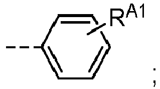
**R^{E7}** represents 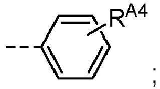
**R^{A1}** represents -OCH₃;
**R^{A4}** represents -CH₃;
or tautomers, *N*-oxide, hydrates, solvates, metallic complexes, or acid salt forms thereof;
or
**D** represents 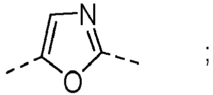
**R₁** and **R₂** are hydrogen and **R₃** represents -CH₃, or
**R₁** is hydrogen and **R₂** and **R₃** represent independently of each other -OH, -CF₃, -Cl, -OCH₃, -CH₃, -F;
**R₄** is hydrogen and **R₅** represents -COOH, -COOC₂H₅, -NH-SO₂-CH₃, -NH-CO-OCH₃, -CO-N(C₂H₅)₂, -NH-CO-CH(NH₂)-CH₂CH₂-CO-NH₂; or
or tautomers, *N*-oxide, hydrates, solvates, metallic complexes, or acid salt forms thereof;
or
**D** represents 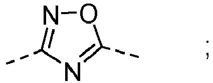
**R₁** and **R₂** are hydrogen and **R₃** represents -OCF₃, -COOC₂H₅, -CF₃, -CH₃, -COOH, 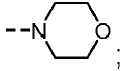
**R₄** is hydrogen and **R₅** represents -SO₂CH₃, -SCH₃, -F, -NH-CO-CH(CH₃)₂, -NH-CO-NH-Ph, -NH-CO-CH(NH₂)-CH₂-COOH; or
**R₄** and **R₅** represent independently of each other -Cl, -SCH₃; or
**R₄** and **R₅** form together the residue -O-CH₂-CH₂-N(CH₃)-;
or tautomers, *N*-oxide, hydrates, solvates, metallic complexes, or acid salt forms thereof;
or
**D** represents 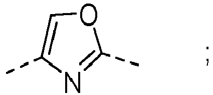
**R₁** and **R₂** are hydrogen and **R₃** represents hydroxyl, -OCF₃, -OC₂H₅, -NHS(=O)₂**R^{E7}**, or
**R₁** is hydrogen and **R₂** and **R₃** form together the residue -O-(CH₂CH₂)ₙ-O-;
**R₄** represents hydrogen and **R₅** represents -OCH(CH₃)₂, -CH(CH₃)₂, 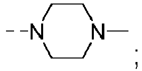 or
**R₄** and **R₅** represent -Cl, -OCH₃, -OH; or
**R₄** and **R₅** form together the residue -N=CH-CH=CH-;
**R^{E7}** represents 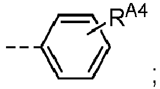
**R^{A4}** represents -OCH₃;
or tautomers, *N*-oxide, hydrates, solvates, metallic complexes, or acid salt forms thereof; or
**D** represents 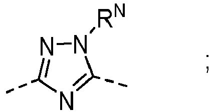
**R₁** and **R₂** are hydrogen and **R₃** represents -F, -Cl, -OCF₃, -CF₃, -N(CH₃)₂, -CH₃, -OCH₃;
**R₄** represents hydrogen and **R₅** represents -COOH, -NH-CO-CH(NH₂)-(CH₂)₄-NH₂, 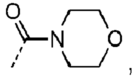 -NH-CO-CF₃, -NH-CO-OCH₃, -NH-CO-NH-CH₂CH₂-OCH₃, -NHS(=O)₂**R^{E7}**, -OC₂H₅, -NH-CO-O-CH₂CH₂-OCH₃; or
**R₄** and **R₅** represent independently of each other -CH₃, -NH₂, -NO₂, -NH-CO-NH-CH₂CH₃, -NHC(=O)**R^{E6},** -NH-CO-CH(NH₂)-CH₂-Ph, -OCH₃, -NH-CO-CH(NH₂)-CH₃;
**R^{E6}** represents 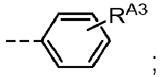
**R^{E7}** represents 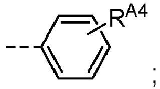
**R^{A3}** represents -Cl;
**R^{A4}** represents -Cl;
or tautomers, *N*-oxide, hydrates, solvates, metallic complexes, or acid salt forms thereof;

15. The compound according to claim 14 selected from the group consisting of:
| | |
|---|---|
| **3** | 4-{3-[3-(4-Chlorophenyl)-1*H*-pyrazol-5-yl]phenyl}morpholine |
| **14** | 3-(1,3-Benzodioxol-5-yl)-5-[3-(dimethylamino)phenyl]-1*H*-pyrazole |
| **15** | 3-(1,3-Benzodioxol-5-yl)-5-(4-iodophenyl)-1*H*-pyrazole |
| **30** | 2,4-Bis(3,4-dimethoxyphenyl)-1*H*-imidazole |
| **45** | 5-(4-Aminophenyl)-3-(2-fluorophenyl)-isoxazole |
| **46** | *N*-{4-[3-(2-Fluorophenyl)-isoxazol-5-yl]phenyl}acetamide |
| **47** | *N*-{4-[3-(2-Fluorophenyl)-isoxazol-5-yl]phenyl}-4-methylbenzenesulfonamide |
| **48** | 5-(4-Chloro-3-methoxyphenyl)-3-[3-(trifluoromethyl)phenyl]-isoxazole |
| **49** | 5-(4-Chloro-3-hydroxyphenyl)-3-[3-(trifluoromethyl)phenyl]-isoxazole |
| **52** | 3-(2-Chlorophenyl)-5-[4-(ethylsulfanyl)phenyl]-isoxazole |
| **53** | 5-(3,4-Dimethylphenyl)-3-[3-(trifluoromethoxy)phenyl]-isoxazole |
| **54** | 2,2,2-Trifluoro-*N*-{3-[2-(4-fluorophenyl)-1*H*-imidazol-5-yl]phenyl}acetamide |
| **55** | *N*-(4-Hydroxyphenyl)-4-{4-[4-(pyrrolidin-1-yl)phenyl]-1*H*-imidazol-2-yl}benzamide |
| **56** | 4-[5-(4-Chlorophenyl)-1*H*-1,2,4-triazol-3-yl]benzoic acid hydrochloride |
| **57** | 1-{4-[5-(3-Fluorophenyl)-1,2-oxazol-3-yl]phenyl}-3-(4-methoxyphenyl)urea |
| **58** | (2*S*)-2,6-Diamino-*N*-(4-{3-[4-(trifluoromethoxy)phenyl]-1*H*-1,2,4-triazol-5-yl}phenyl)hexanamide dihydrochloride |
| **59** | {4-[5-(4-Chlorophenyl)-1*H*-1,2,4-triazol-3-yl]phenyl}(morpholin-4-yl)methanone hydrochloride |
| **60** | 6-[4-(4-Hydroxyphenyl)-1,3-oxazol-2-yl]quinoline |
| **61** | 2,2,2-Trifluoro-*N*-(4-{5-[4-(trifluoromethoxy)phenyl]-1*H*-1,2,4-triazol-3-yl}phenyl)acetamide |
| **63** | Ethyl 3-[5-(2-fluorophenyl)-1,2,4-oxadiazol-3-yl]benzoate |
| **65** | 5-[2-Chloro-5-(methylsulfanyl)phenyl]-3-[3-(trifluoromethoxy)phenyl]-1,2,4-oxadiazole |
| **66** | 2-(4-Ethylphenyl)-4-[4-(trifluoromethoxy)phenyl]-1*H*-imidazole |
| **69** | 3-{5-[2-Hydroxy-4-(trifluoromethyl)phenyl]-1,3-oxazol-2-yl}benzoic acid |
| **75** | Propan-2-yl (4-{3-[3-(trifluoromethyl)phenyl]-1,2,4-oxadiazol-5-yl}phenyl)carbamate |
| **76** | 2-(4-Chlorophenyl)-5-[4-(trifluoromethoxy)phenyl]-1*H*-imidazole |
| **77** | Methyl (4-{5-[4-(trifluoromethoxy)phenyl]-1*H*-1,2,4-triazol-3-yl}phenyl)carbamate |
| **78** | 2-(4-Chlorophenyl)-5-(4-hydroxyphenyl)-1*H*-imidazole |
| **79** | 5-(3,4-Dichlorophenyl)-2-(3-methylphenyl)-1*H*-imidazole |
| **81** | 5-(2-Hydroxy-5-fluorophenyl)-2-[4-(trifluoromethyl)phenyl]-1*H*-imidazole |
| **82** | 1-(2-Methoxyethyl)-3-(4-{5-[4-(trifluoromethyl)phenyl]-1*H*-1,2,4-triazol-3-yl}phenyl)urea |
| **83** | 4-{3-[3-(Trifluoromethyl)phenyl]-1,2-oxazol-5-yl}benzoic acid |
| **84** | 2-(4-Bromophenyl)-5-[4-(trifluoromethoxy)phenyl]-1*H*-imidazole |
| **87** | 2-(3-Bromophenyl)-5-(3-hydroxyphenyl)-1*H*-imidazole |
| **88** | *N*-{3-[2-(4-fluorophenyl)-1*H*-imidazol-5-yl]phenyl}benzamide |
| **89** | 3-(4-Methylphenyl)-5-[4-(methylsulfonyl)phenyl]-1,2,4-oxadiazole |
| **90** | 5-[4-(Azetidin-1-yl)phenyl]-3-(4-ethylphenyl)-1,2-oxazole |
| **91** | 1-{4-[3-(2-Chlorophenyl)-1,2-oxazol-5-yl]phenyl}piperidine |
| **92** | 4-Chloro-*N*-(4-{5-[4-(trifluoromethyl)phenyl]-1*H*-1,2,4-triazol-3-yl}phenyl)benzenesulfonamide |
| **93** | (2*S*)-2-Amino-*N*-{4-[5-(3-fluorophenyl)-1,2-oxazol-3-yl]phenyl}-3-methylbutanamide hydrochloride |
| **94** | 1-Phenyl-3-(4-{3-[3-(trifluoromethyl)phenyl]-1,2,4-oxadiazol-5-yl}phenyl)urea |
| **95** | Ethyl 4-{5-[4-(methylsulfanyl)phenyl]-1,2,4-oxadiazol-3-yl}benzoate |
| **96** | 4-{5-[4-(Methylsulfanyl)phenyl]-1,2,4-oxadiazol-3-yl}benzoic acid |
| **98** | 4-Methyl-7-{3-[3-(morpholin-4-yl)phenyl]-1,2,4-oxadiazol-5-yl}-3,4-dihydro-2*H*-1,4-benzoxazine |
| **99** | 3-(4-Fluorophenyl)-5-(2-methyl-5-nitrophenyl)-1*H*-1,2,4-triazole |
| **100** | 5-(3-Amino-6-methylphenyl)-3-(4-fluorophenyl)-1*H*-1,2,4-triazole |
| **101** | 1-Ethyl-3-{3-[3-(4-fluorophenyl)-1*H*-1,2,4-triazol-5-yl]-4-methylphenyl}urea |
| **102** | 3-Chloro-*N*-{3-[3-(4-fluorophenyl)-1*H*-1,2,4-triazol-5-yl]-4-methylphenyl}benzamide |
| **103** | (2*S*)-2-Amino-*N*-{3-[3-(4-fluorophenyl)-1*H*-1,2,4-triazol-5-yl]-4-methylphenyl)-3-phenylpropanamide hydrochloride |
| **105** | 3-[3-(3,4-Dimethoxyphenyl)-1*H*-1,2,4-triazol-5-yl]-*N*,*N*-dimethylaniline |
| **106** | 5-(3-Ethoxyphenyl)-3-(3-methylphenyl)-1*H*-1,2,4-triazole |
| **108** | Ethyl {3-[2-(4-fluorophenyl)-1*H*-imidazol-5-yl]phenyl}carbamate |
| **109** | 4-{4-[5-(2,3-Dihydro-1-benzofuran-5-yl)-1*H*-imidazol-2-yl]phenyl}morpholine |
| **110** | 4-(4-Ethoxyphenyl)-2-[3-(propan-2-yl)phenyl]-1,3-oxazole |
| **114** | 1-Methyl-4-{3-[4-(2,3-dihydro-1,4-benzodioxin-6-yl)-1,3-oxazol-2-yl]phenyl}piperazine |
| **115** | Ethyl 4-[5-(4-methylphenyl)-1,3-oxazol-2-yl]benzoate |
| **118** | 2-(4-Bromophenyl)-5-(4-hydroxyphenyl)-1*H*-imidazole |
| **119** | *N*-{3-[5-(3,4-Dichlorophenyl)-1,3-oxazol-2-yl]phenyl}methanesulfonamide |
| **120** | Methyl {3-[5-(3,4-dichlorophenyl)-1,3-oxazol-2-yl]phenyl}carbamate |
| **121** | Ethyl 3-{5-[2-methoxy-4-(trifluoromethyl)phenyl]-1,3-oxazol-2-yl}benzoate |
| **122** | 3-{5-[2-Methoxy-4-(thfluoromethyl)phenyl]-1,3-oxazol-2-yl}benzoic acid |
| **123** | *N*,*N*-diethyl-3-{5-[2-methoxy-4-(trifluoromethyl)phenyl]-1,3-oxazol-2-yl}benzamide |
| **124** | 4-Methoxy-*N*-{4-[2-[4-(propan-2-yloxy)phenyl]-1,3-oxazol-4-yl]phenyl}benzenesulfonamide |
| **125** | 3-[4-(4-Ethylphenyl)-1*H*-imidazol-2-yl]benzoic acid |
| **126** | 2-Methoxyethyl {3-[5-(4-ethylphenyl)-1*H*-pyrazol-3-yl]phenyl}carbamate |
| **127** | 1-(4-Fluorophenyl)-3-(4-{5-[3-(trifluoromethoxy)phenyl]-1*H*-pyrazol-3-yl}phenyl)urea |
| **128** | 2,2,2-Trifluoro-*N*-(4-{5-[3-(trifluoromethoxy)phenyl]-1*H*-pyrazol-3-yl}phenyl)acetamide |
| **129** | (2*S*)-2-Amino-*N*-{3-[3-(4-ethylphenyl)-1*H*-pyrazol-5-yl]phenyl}-3-hydroxypropanamide hydrochloride |
| **130** | 2-(3-Chloro-5-methoxyphenyl)-4-[4-(trifluoromethoxy)phenyl]-1,3-oxazole |
| **131** | (2*S*)-2-Amino-*N*¹-{3-[5-(4-fluoro-2-methylphenyl)-1,3-oxazol-2-yl]phenyl}pentanediamide hydrochloride |
| **132** | 2-(3-Chloro-5-hydroxyphenyl)-4-[4-(trifluoromethoxy)phenyl]-1,3-oxazole |
| **133** | (3*S*)-3-Amino-4-oxo-4-[(4-{3-[3-(trifluoromethyl)phenyl]-1,2,4-oxadiazol-5-yl}phenyl)amino]butanoic acid hydrochloride |
| **134** | 3-(3,4-Dimethylphenyl)-5-(4-ethoxyphenyl)-1*H*-pyrazole |
| **135** | 4-{4-[4-(Pyrrolidin-1-yl)phenyl]-1*H*-imidazol-2-yl}benzoic acid |
| **136** | 4-Chloro-*N*-(3-{4-[4-(trifluoromethoxy)phenyl]-1*H*-imidazol-2-yl}phenyl)benzenesulfonamide |
| **137** | 2-Methoxyethyl (3-{4-[4-(trifluoromethoxy)phenyl]-1*H*-imidazol-2-yl}phenyl)carbamate |
| **138** | (4-Methylpiperazin-1-yl){3-[4-(4-methylphenyl)-1*H*-imidazol-2-yl]phenyl}methanone |
| **139** | 2-Methoxyethyl {3-[3-(4-methoxyphenyl)-1H-1,2,4-triazol-5-yl]phenyl}carbamate |
| **140** | (2*S*)-2-Amino-*N*-{3-[3-(4-methoxyphenyl)-1*H*-1,2,4-triazol-5-yl]phenyl}propenamide hydrochloride |
| **141** | (2*S*)-2-Amino-*N*-{3-[4-(4-trifluoromethoxyphenyl)-1*H*-imidazol-2-yl]phenyl}-3-phenylpropanamide hydrochloride |
| **144** | 4-{3-[4-(3,4-Dimethoxyphenyl)-1*H*-imidazol-2-yl]phenyl}morpholine |
or acid salt forms thereof.


SEQUENCE LISTING

<110>  Max-Planck-Gesellschaft zur Förderung der Wissenschaften
       e.V.

<120>  Protein aggregation inhibiting compounds for plant disease
       control

<130>  GAR-P03933EP

<160>  4

<170>  PatentIn version 3.5

<210>  1
<211>  20
<212>  DNA
<213>  Arabidopsis thaliana

<400>  1
aaggaggctg ctgagatgaa                                                20


<210>  2
<211>  20
<212>  DNA
<213>  Arabidopsis thaliana

<400>  2
tggtggtctc gaacttccag                                                20


<210>  3
<211>  21
<212>  DNA
<213>  Arabidopsis thaliana

<400>  3
actttcagca gtggatgtct a                                              21


<210>  4
<211>  21
<212>  DNA
<213>  Arabidopsis thaliana

<400>  4
gatgactcac tgaattctgc a                                              21